# EUROPEAN PATENT SPECIFICATION

(11) **EP 1 687 642 B1**
(45) Date of publication and mention of the grant of the patent: **14.01.2009**
(21) Application number: 04790957.7
(22) Date of filing: 07.10.2004
(51) Int. Cl.: G01N 33/74, G01N 33/50

(54) **Screening methods for combinations of biological compounds**
Screeningverfahren für Kombinationen von biologischen Verbindungen
Procédé de criblage pour des combinaisons de composés biologiques

(30) Priority: 07.10.2003 US 508859 P
(43) Date of publication of application: 09.08.2006
(73) Proprietor: CENTELION, 94400 Vitry Sur Seine (FR); Aventis Pharmaceuticals Inc., Bridgewater, New Jersey 08807 (US)
(72) Inventor: NESBIT, Mark, F-94300 Vincennes (FR); SPADA, Alfred, P., Lansdale, PA 19446 (US); HE, Wei, Collegeville, PA 19426 (US); MYERS, Michael, R., F-78860 Saint Nom La Breteche (US)
(74) Representative: Bouvet, Philippe
(86) International application number: PCT/EP2004/012185
(87) International publication number: WO 2005/038465

(56) References cited:
- EP-A- 0 721 983
- WO-A-00/31051
- WO-A-92/20642
- WO-A-02/072578
- WALTENBERGER JOHANNES ET AL: "A dual inhibitor of platelet-derived growth factor beta-receptor and Src kinase activity potently interferes with motogenic and mitogenic responses to PDGF in vascular smooth muscle cells: A novel candidate for prevention of vascular remodeling" CIRCULATION RESEARCH, vol. 85, no. 1, 9 July 1999 (1999-07-09), pages 12-22, XP002319349 ISSN: 0009-7330
- TOPALY J. ET AL: 'Combination therapy with imatinib mesylate (STI571): synopsis of in vitro studies' BRITISH JOURNAL OF HAEMATOLOGY, OXFORD, GB vol. 119, no. 1, October 2002, pages 3 - 14, XP002346559
- TOPALY J.; ZELLER W.J.; FRUEHAUF S.: 'SYNERGISTIC ACTIVITY OF STI571 WITH CHEMOTHERAPEUTIC DRUGS AND IRRADIATION' BLOOD vol. 96, no. 11, 01 December 2000, page 736A, XP009010656
- O'DWYER M.E. ET AL: 'The role of the tyrosine kinase inhibitor STI571 in the treatment of cancer.' CURRENT CANCER DRUG TARGETS vol. 1, 2001, pages 49 - 57, XP008077857
- HOLCOMBE RANDALL F. ET AL: 'Expression of Kit and platelet-derived growth factor receptors alpha and beta in cholangiocarcinoma, and case report of therapy with imatinib mesylate (STI571).' ANTI-CANCER DRUGS vol. 14, no. 8, September 2003, pages 651 - 657, XP008077873

## Description

### Field of the Invention and Introduction

This invention is directed to the inhibition of cell proliferation and/or cell matrix production and/or cell movement (chemotaxis) and/or T cell activation and proliferation using of quinoline/quinoxaline compounds which are useful protein tyrosine kinase inhibitors (TKIs). Cellular signaling is mediated through a system of interactions which include cell-cell contact or cell-matrix contact or extracellular receptor-substrate contact. The extracellular signal is often communicated to other parts of the cell via a tyrosine kinase mediated phosphorylation event which affects substrate proteins downstream of the cell membrane bound signaling complex. A specific set of receptor- enzymes such as the insulin receptor, epidermal growth factor receptor (EGF-R) or platelet-derived growth factor receptor (PDGF-R) are examples of tyrosine kinase enzymes which are involved in cellular signaling. Autophosphorylation of the enzyme is required for efficient enzyme-mediated phosphorylation of substrate proteins containing tyrosine residues. These substrates are known to be responsible for a variety of cellular events including cellular proliferation, cellular matrix production, cellular migration and apoptosis to name a few.

It is understood that a large number of disease states are caused by either uncontrolled reproduction of cells or overproduction of matrix or poorly regulated programmed cell death (apoptosis). These disease states involve a variety of cell types and include disorders such as leukemia, cancer, glioblastoma, psoriasis, inflammatory diseases, bone diseases, fibrotic diseases, atherosclerosis and restenosis occurring subsequent to angioplasty of the coronary, femoral or kidney arteries or, fibroproliferative disease such as in arthritis, fibrosis of the lung, kidney and liver. In addition, deregulated cellular proliferative conditions follow from coronary bypass surgery. The inhibition of tyrosine kinase activity is believed to have utility in the control of uncontrolled reproduction of cells or overproduction of matrix or poorly regulated programmed cell death (apoptosis).

It is also known that certain tyrosine kinase inhibitors can interact with more than one type of tyrosine kinase enzyme. Several tyrosine kinase enzymes are critical for the normal function of the body. For instance, it would be undesirable to inhibit insulin action in most normal circumstances. Therefore, compounds which inhibit PDGF-R tyrosine kinase activity at concentrations less than the concentrations effective in inhibiting the insulin receptor kinase could provide valuable agents for the selective treatment of diseases characterized by cell proliferation and/or cell matrix production and/or cell movement (chemotaxis).

### Background of and Relevance of the Invention

A number of literature reports describe tyrosine kinase inhibitors which are selective for tyrosine kinase receptor enzymes such as EGF-R or PDGF-R or non-receptor cytosolic tyrosine kinase enzymes such as v-abl, p56^{lck} or c-SRC. Recent reviews by Spada and Myers (Exp. Opin. Ther. Patents 1995, 5(8), 805) and Bridges (Exp. Opin. Ther. Patents 1995, 5(12), 1245) summarize the literature for tyrosine kinase inhibitors and EGF-R selective inhibitors respectively. Additionally Law and Lydon have summarized the anticancer potential of tyrosine kinase inhibitors (Emerging Drugs: The Prospect For Improved Medicines 1996, 241-260).

Known inhibitors of PDGF-R tyrosine kinase activity includes quinoline-based inhibitors reported by Maguire et al. (J. Med. Chem. 1994, 37, 2129), and by Dolle et al. (J. Med. Chem. 1994, 37, 2627). A class of phenylamino-pyrimidine-based inhibitors was recently reported by Traxler et al. in EP 564409 and by Zimmerman, J.; and Traxler, P. et al. (Biorg. & Med. Chem. Lett. 1996, 6(11), 1221-1226) and by Buchdunger, E. et al. (Proc. Nat. Acad. Sci. 1995, 92, 2558). Despite the progress in the field there are no agents from these classes of compounds that have been approved for use in humans for treating proliferative disease.

The correlation between the multifactorial disease of restenosis with PDGF and PDGF-R is well-documented throughout the scientific literature. However, recent developments into the understanding of fibrotic diseases of the lung (Antoniades, H. N.; et al. J. Clin. Invest. 1990, 86, 1055), kidney and liver (Peterson, T. C. Hepatology, 1993, 17, 486) have also implicated PDGF and PDGF-R as playing a role. For instance glomerulonephritis is a major cause of renal failure and PDGF has been identified to be a potent mitogen for mesangial cells in vitro as demonstrated by Shultz et al. (Am. J. Physiol. 1988, 255, F674) and by Floege, et al. (Clin. Exp. Immun. 1991, 86, 334). It has been reported by Thornton, S. C.; et al. (Clin. Exp. Immun. 1991, 86, 79) that TNF-alpha and PDGF (obtained from human rheumatoid arthritis patients) are the major cytokines involved in proliferation of synovial cells. Furthermore, specific tumor cell types have been identified (see Silver, B. J., BioFactors, 1992. 3, 217) such as glioblastoma and Kaposi's sarcoma which overexpress either the PDGF protein or receptor thus leading to the uncontrolled growth of cancer cells via an autocrine or paracrine mechanism. Therefore, it is anticipated that a PDGF tyrosine kinase inhibitor would be useful in treating a variety of seemingly unrelated human disease conditions that can be characterized by the involvement of PDGF and or PDGF-R in their etiology.

The role of various non-receptor tyrosine kinases such as p56^{lck} (hereinafter "LCK") in inflammation-related conditions involving T cell activation and proliferation has been reviewed by Hanke, et al (Inflamm. Res. 1995, 44, 357) and by Bolen and Brugge (Ann. Rev. Immunol., 1997, 15, 371). These inflammatory conditions include allergy, autoimmune disease, rheumatoid arthritis and transplant rejection. Another recent review summarizes various classes of tyrosine kinase inhibitors including compounds having LCK inhibitory activity (Groundwater, et. al Progress in Medicinal Chemistry, 1996, 33, 233). Inhibitors of LCK tyrosine kinase activity include several natural products which are generally non-selective tyrosine kinase inhibitors such as staurosporine, genistein, certain flavones and erbstatin. Damnacanthol was recently reported to be a low nM inhibitor of LCK (Faltynek, et. al, Biochemistry, 1995, 34, 12404). Examples of synthetic LCK inhibitors include: a series of dihydroxy-isoquinoline inhibitors reported as having low micromolar to submicromolar activity (Burke, et. al J. Med. Chem. 1993, 36, 425); and a quinoline derivative found to be much less active having an LCK IC₅₀ of 610 micromolar. Researchers have also disclosed a series of 4-substituted quinazolines that inhibit LCK in the low micromolar to submicromolar range (Myers et al, WO95/15758 and Myers, et. al Bioorg. Med. Chem. Lett. 1997, 7, 417). Hanke, et. al. (J. Biol. Chem. 1996, 271, 695) have disclosed two specific pyrazolopyrimidine inhibitors known as PP1 and PP2 which have low nanomolar potency against LCK and FYN (another SRC-family kinase). No LCK inhibitory has been reported regarding quinoline or quinoxaline based compounds. Therefore, it is anticipated that a quinoline or quinoxaline based inhibitor of LCK tyrosine kinase activity could be useful in treating a variety of seemingly unrelated human disease conditions that can be characterized by the involvement of LCK tyrosine kinase signaling in their etiology.

Platelet-derived growth factor (PDGF) is a potent proliferative agent in cells of mesenchymal origin (Antoniades, H. N. et al. (1979) Proc. Natl. Acad. Sci. USA 76:1809-1813; Bowen-Pope, D. F. and Ross, R. (1982) J. Biol. Chem. 257:5161-5171; Heldin, C.-H. et al. (1983) J. Biol. Chem. 258:10054-10059). PDGF (M.W. 30 kDa) is a dimeric protein having two polypeptides chains joined by disulfide bonds (a disulfide-linked dimer) consisting of 2 homologous polypeptide chains, which are either termed A chain polypeptide or termed B chain polypeptide (Johnsson, A. et al. (1982) Biochem. Biophys. Res. Commun. 104:66-74). The chains may combine with chains of the same or the other type, resulting in 3 isoforms AA, BB or AB (Heldin, C.-H. et al. (1986) Nature 319:511-514). Thus PDGF can have two A chains, two B chains, or an A and an B chain. The mitogen PDGF was first identified (Antoniades, H. N. (1979) Proc. Natl. Acad. Sci. USA 76:1809-1813; Raines, E. W. and Ross, R. (1982) J. Biol. Chem. 257:5154-5160) and purified from human platelets. Subsequent research has shown that several cell types including vascular endothelial cells, vascular smooth muscle cells, macrophages and even fibroblasts synthesize PDGF (Ross, R. et al. (1986) Cell 46:155-169).

PDGF plays an important role in both normal physiological processes such as tissue repair and embryogenesis, and as potent mitogen in pathological proliferation disorders, and in the development of certain carcinomas. Expression of PDGF A chain and PDGF B receptor has been detected in human atherosclerotic plaques by in situ hybridization (Wilcox, J. N. et al. (1988) J. Clin. Invest. 82:1134-1143). Recently, Ferns et al. ((1991) Science 253:1129-1132) have reported that a polyclonal antibody to PDGF significantly reduced the formation of intimal lesions in deendothelialized carotid arteries of athymic nude rats. PDGF has been implicated in the pathology of proliferative diseases in cells of mesenchymal origin (Nister, M. et al. (1984) Proc. Natl. Acad. Sci. USA 81:926-930, and Nister, M. et al. (1987) Cancer Res. 47:4953-4961). Golden et al. have reported that PDGF A chain message was increased in areas of intimal hyperplasia in a baboon model for vascular grafts ((1990) J. Vasc. Surg. 11:580-585). PDGF is also chemotactic for smooth muscle (Westermark, B. et al. (1990) Proc. Natl. Acad. Sci. USA 87:128-132), and platelet PDGF may be the causative agent for the migrations and proliferation of smooth muscle cells in the ballooned rat carotid artery, which results in significant stenosis.

The cellular proliferation induced by all isoforms of PDGF is mediated by ligand binding to the PDGF receptor (Heldin, C.-H. (1983) op. cit., Ek, B. et al. (1982) Nature 295:419-420; Glenn, K. et al. (1982) J. Biol. Chem. 257:5172-5176; Frackelton, A. R. et al. (1984) J. Biol. Chem. 259:7909-7915; Williams, L. T. et al. (1984) J. Biol. Chem. 259:5287-5294). The PDGF receptor (M.W. 180 kDa) belongs to the tyrosine kinase family and consists of two receptor subtypes, termed type A (or type a) (Matsui, T. et al. (1989) Science 243:800-804, and Claesson-Welsh, L. (1989) Proc. Natl. Acad. Sci. USA 86:4917-4921) and type B (or type β) (Yarden, Y. et al. (1986) Nature 323:226-232, and Escobedo, J. A. et al. (1988) Science 240:1532-1534). Both α-and β- containing receptors have been associated with mitogen activity, while only the β-containing receptor has been associated with chemotaxis and actin reorganization (Heldin, C-H, EMBO Journal 11: 4251-4259, 1992).

PDGF ligand binding to the receptor is followed by receptor dimerization (Bishayee, S. et al. (1989) J. Biol. Chem. 264:11699-11705, and Heldin, C.-H. et al. (1989) J. Biol. Chem. 264:8905-8912) and autophosphorylation (Frackelton, et al. on. cit.), and results in a complicated series of intracellular signaling events culminating in DNA synthesis. Mouse and human PDGF β receptor and PDGF α receptor genes have been cloned (Matsui et al. op. cit., Claesson-Welsh et al. op. cit., Yarden et al. op. cit., and Escobedo et al. op. cit.). When referring to PDGF receptors herein, type A and type α or α-PDGFR are used interchangeably, as are type B and type β or β-PDGFR.

The two receptor isoforms may be distinguished by their markedly different ligand binding specificities. PDGF β receptor binds only B-chain (isoforms BB and AB), while PDGF a receptor can bind all forms of PDGF (isoforms containing A and/or B chain (Matsui et al. op. cit., Claesson-Welsh et al. op. cit., and Seifert, R. A. et al. (1989) J. Biol. Chem. 264:8771-8778). The PDGF receptor shows a high degree of structural homology to the macrophage-colony stimulating factor receptor (Coussens, L. et al. (1986) Nature 320:277-280) and the c-kit protooncogene product.

The PDGF receptors are transmembrane receptors characterized by an extracellular domain which may be demarcated into five Ig-like domains based on their β-sheet rich structure. These Ig repeats of approximately 100 amino acids each have regularly spaced cysteine residues (except in the fourth repeat). The receptor has a single transmembrane domain and a cytoplasmic tyrosine kinase domain (Williams, L. T. (1989) Science 243:1564-1570).

Several studies have produced direct and indirect evidence of proof that PDGF and PDGFR are involved in tumor growth and metastasis are angiogenesis-dependent (Brooks et al., Cell, 1994, 79, 1154-1164; Kim KJ et al., Nature, 1993, 362, 841-844). Expansion of the tumor volume requires the induction of new capillary blood vessels. Tumor cells promote angiogenesis by the secretion of angiogenic factors, in particular basic fibroblast growth factor (BFGF) (Kandel J. et al., Cell, 1991, 66, 1095-1104) vascular endothelial growth factor (VEGF) (Ferrara et al., Endocr. Rev., 1997, 18: 4-25) and platelet derived growth factor (PDGF). Tumors may produce one or more of these angiogenic peptides that can synergistically stimulate tumor angiogenesis (Mustonen et al., J Cell Biol., 1995, 129, 865-898). Angiogenesis is the generation of new blood vessels from preexisting vessels in a tissue or organ. Angiogenesis is required and normally observed under normal physiological conditions, such as for example, for wound healing, fetal and embryonic development, for female reproduction, *i.e*., formation of the corpus luteum, endometrium and placenta, organ formation, tissue regeneration and remodeling (Risau W et al., Nature, 1997, 386, 671-674). Angiogenesis begins with local degradation of the basement membrane of capillaries, followed by invasion of stroma by underlying endothelial cells in the direction of an angiogenic stimulus. Subsequent to migration, endothelial cells proliferate at the leading edge of a migrating column and then organize to form new capillary tubes.

Persistent, unregulated angiogenesis occurs in a multiplicity of pathological conditions, tumor metastasis and abnormal growth by endothelial cells and supports the pathological damage seen in these conditions. The diverse pathological disease states in which unregulated angiogenesis are present have been grouped together as angiogenic dependent or angiogenic associated diseases. Outgrowth of new blood vessels under pathological conditions can lead to the development and progression of diseases such as tumor growth, diabetic retinopathy, tissue and organ malformation, obesity, macular degeneration, rheumatoid arthritis, psoriasis, and cardiovascular disorders.

However, current anti-angiogenic therapies or chemotherapies target tumors where there is immature development or growth of the vasculature. It is known that such immature vasculature, which mainly comprises endothelial cells without perivasculature, *i.e*., mural cell support, is influenced by the tumor's production of pro-angiogenic factors and are especially sensitive to anti-angiogenic therapy. Yet, the majority of the vasculature found within tumors of mammals comprises perivasculature, *i.e*., mural cell support to the endothelial cells, including pericytes, smooth muscle cells, and fibroblasts. Such perivasculature can be arterioles, which are endothelial cell tubes surrounded by pericytes/smooth muscle cells. It has been shown by that endothelial cells within this context become resistant or refractory to chemotherapy or anti-angiogenic therapy, and no longer respond to the pro-angiogenic factor produced by the tumor, so the tumor remains nourished by a mature vasculature network (*see, for example,* Minasi et al., Development 129: 2773 (2002)).

Several studies have reported the inhibition of the tyrosine kinase activity of the receptor for the PDGF. For example, Shawver et al. (Clinical Cancer Research, vol. 3, pp 1167-1177, 1997) describe the use of a small organic molecule, *e.g*., N-[4-(trifluoromethyl)-phenyl]5-methylisoxazole-4-carboxamide or the 5-[5-Fluorc-2-oxo-1,2- dihydroindol-(32)-ylidenemethyl]-2,4- dimethyl-1H-pyrrole-3-carboxylic acid, also designated leflunomide or SU101 as inhibitor of the PDGF signaling pathway including receptor tyrosine phosphorylation, DNA synthesis, cell cycle progression, and cell proliferation. Particularly, Shawver et al. demonstrated that leflunomide was capable of inhibiting in vitro growth of cells from glioma, ovarian and prostate origin which express PDGFR, but failed to inhibit tumors cells which do not express PDGFRβ.

Also, Uehara et al. (J Nat Cancer Institute, vol 95, No. 6, 1999) that the tyrosine kinase inhibitor STI 571 is capable of blocking the PDGF signaling pathway by inhibiting PDGFR autophosphorylation. It was shown *inter alia* on tumor growth in a mouse model of experimental prostate cancer bone metastasis that STI 571 alone or in combination with paclitaxel had a statistically significantly lower tumor incidence, smaller tumors, and less bone lysis and lymph node metastasis than mice treated with water or paclitaxel alone, but no statistically significant synergistic interaction associated with treatment using the combination of two drugs except for the delay in the progression of osteolytic lesions. Using the same compound, Dudley et al. (BBRC 310, pp. 135, 2003) showed that inhibition of the PDGF signal transduction pathway by PDGF receptor tyrosine kinase inhibitor blocked aortic smooth muscle cell growth and migration in a rat mode. None of the references address the problem of tumor cells being refractory to chemotherapy. Also, none of the references address the problem of abrogating mature vasculature as found in patients' tumors wherein arterioles which are endothelial cell tubes surrounded by pericytes or smooth muscle cells are already established. In this regard, experimental designs have not adequately represented higher order vessels as present in human tumors.

Thus, there is a need for a treatment capable of not only preventing the development of new vasculature, but also capable of abrogating mature perivasculature in and/or supporting tumors and tumors that are resistant or refractory to standard care therapy.

It is thus one aspect of the present disclosure to provide an anti-cancer therapy capable to efficiently abrogate mature vasculature, which mature vasculature has been shown up to now to be resistant or refractory to current standard anti-cancer therapy.

As described below, it is now been found that the use of a combination of a PDGF-receptor tyrosine kinase inhibitor with an anti-angiogenic or chemotherapeutic agent provides a very beneficial and synergistic effect on abrogating immature and mature vasculature within or near tumors. Such combination is particularly active on chemotherapy refractory tumor. While the disclosure is not limited to any particular theory on the mechanism of this synergistic inhibition on perivasculature, it is believed that the combination is capable, in a unexpected way, of uncoupling the endothelial cell (EC) from the mural cell cross-activation or activation loop (*see, for example,* Ramsuaer and D'Amore, J. Clin. Invest. 110:1615 (2002)), thereby rendering the endothelial cell more responsive to anti-angiogenic and/or chemotherapeutic agents. Also, endothelial cells within chemotherapy refractory tumors become sensitive to chemotherapeutic agents (designated as anti-angiogenic chemotherapeutic agents), even at a low dose regimen. It is further believed that the presence of the PDGF receptor tyrosine kinase inhibitor leads to angiopoietin I release-inhibition by the mural cell type, thereby disrupting the paracrine loop between the EC and mural cell and exposing the endothelial cell to anti-angiogenic or chemotherapeutics agents.

Another aspect of the present disclosure relates to the treatment and/or the prevention of cell proliferative disorders comprising at least one potent inhibitor of at least one or two tyrosine kinases, selected among the class III receptor tyrosine kinase family, the SRC-like tyrosine kinases, and ABL-1 or BCR-ABL tyrosine kinase, or mutants thereof. These tyrosine kinases and mutants thereof have been shown to be involved of several forms of human leukemias.

Human leukemias are malignant diseases of the blood-forming organs which involve proliferation and development of leukocytes and their precursors in bone marrow and blood. The blasts that are normally developing into white blood cells called granulocytes, do not mature and become too numerous. These immature blast cells are then found in the blood and the bone marrow, replace normal blood cells and spread to the liver, spleen, lymph nodes, central nervous system, kidney and gonads.

There are four major types of human leukemias which are classified according to two basic considerations: (1) the duration and character of the disease, *i.e.,* acute vs. chronic and (2) the type of cell involved*, i.e.,* myeloid (myelogenous) vs. lymphoid (lymphocytic). Acute means rapidly growing. Although the cells grow rapidly, they are not able to mature properly. Chronic refers to a condition where the cells look mature but they are not completely normal. The cells live too long, build up, and crowd out normal cells. Lymphocytic and myeloid (or myelogenous) refer to the cell types involved. Lymphocytic leukemias start from lymphocytes in the bone marrow. Myeloid leukemia involves either of 2 types of white blood cells: granulocytes or monocytes.

Lymphocytic leukemia develops from lymphoblasts or lymphocytes in the bone marrow. Myelogenous leukemia (sometimes called *myelocytic leukemia*) develops from myeloid cells.

The lymphoblasts which are the precursors of lymphocytes can transform into lymphoblastic or lymphocytic leukemias, that is, leukemias that involve the lymphocyte white blood cell.

The myeloid stem cells are precursors of cells that develop into white blood cells, red blood cells, or platelet-producing cells (megakaryocytes). Although we think of leukemias as being white blood cell diseases, leukemias in this cell line can give rise to red blood cell leukemias and megakaryocyte leukemias, but are quite rare. Most of these leukemias are the myeloid type (myelogenous) - meaning that they come from nonlymphocytic white blood cells.

Bone marrow is the soft, spongy, inner part of bones. All of the different types of blood cells are made in the bone marrow. Bone marrow is made up of blood-forming cells, fat cells, and tissues that aid the growth of blood cells. Early blood cells are called stem cells. These stem cells grow in an orderly process to produce red blood cells, white blood cells, and platelets.

Red blood cells carry oxygen from the lungs to all other tissues of the body. They also carry away carbon dioxide, a waste product of cell activity. A shortage of red blood cells causes weakness, shortness of breath, and tiredness.

Platelets are actually pieces that break off from certain bone marrow cells. They are called platelets because they look a little bit like plates when seen under the microscope. Platelets help stop bleeding by plugging up areas of blood vessels damaged by cuts or bruises.

White blood cells help defend the body against germs -- viruses and bacteria. There are quite a few types of white blood cells. Each has a special role to play in protecting the body against infection. The 3 main types of white blood cells are granulocytes, monocytes, and lymphocytes. The suffix *-cyte* means cell.

In acute leukemias, the leukemia cells come from early cells - the immature "blasts." These leukemias are fast growing because normal blast cells divide frequently. But the leukemia cells do not divide any more often than do normal blast cells. They just don't stop dividing when normal blast cells would. Although the white blood cell count is often high - around 20,000 to 50,000 or higher, it can be normal or even low. This differs from chronic leukemia, where the white blood cell count is almost always high when the patient is diagnosed.

In chronic leukemias, the leukemia cells arise from more mature cells, but they are not completely normal. The cells live too long and build up. For example, a typical white blood cell count in a chronic leukemia would be 100,000, not the normal 5,000 to 10,000. They tend to be slow growing.

In general, the different types of leukemia are restricted to different age groups. For example, acute lymphoid leukemia (ALL) generally occurs in young children while acute myelogenous leukemia (AML) is found principally in young adults. The chronic forms of leukemia are found principally in adults.

Myeloid leukemias thus involve the myeloid elements of the bone marrow -white cells, red cells and megakaryocytes. Myeloid leukemia accounts for half of all leukemia cases, and is classified as acute myelogenous leukemia (AML) or chronic myelogenous leukemia (CML). Leukemia can be acute (progressing quickly with many immature blasts) or chronic (progressing slowly with more mature looking cancer cells). Acute myeloid leukemia progresses quickly. Chronic myelogenous leukemia (CML) is a human malignancy that affects hematopoietic progenitor cells. The clinical course of CML progresses through three phases, becoming resistant in each successive phase, with chronic and accelerated phases followed by a terminal blast crisis phase or blast cell transformation, which is usually terminal. Blast crisis may occur anywhere between 1-5 years after initial diagnosis but in most patients it takes about 3-4 years. During the acute leukemia terminal phase, myeloid and lymphoid blasts fail to differentiate.

Chronic myelogenous leukemia (CML) affects mostly in middle-aged adults, average age (50s), and is very rare in children. Involved cell is maturing white blood cell called myelocyte. In more than 95% CML patients, the leukemic cells share a chromosome abnormality not found in any nonleukemic white blood cells, nor in any other cells of the patient's body. This abnormality is a reciprocal translocation between one chromosome 9 and one chromosome 22. This translocation is designated t(9;22). It results in one chromosome 9 longer than normal and one chromosome 22 shorter than normal. The latter is called the Philadelphia chromosome and designated Ph. The DNA removed from chromosome 9 contains most of the proto-oncogene designated c-ABL. The break in chromosome 22 occurs in the middle of a gene designated BCR. The resulting Philadelphia chromosome has the 5' section of BCR fused with most of c-ABL. Transcription and translation of the hybrid BCR-ABL fusion protein is a tyrosine kinase that activates constitutively a number of cell activities that normally are turned on only when the cell is stimulated by a growth factor, such as platelet-derived growth factor (PDGF), and leads to an increase of the rate of mitosis and protect the cell from apoptosis. The outcome is an increase in the number of Ph-containing cells. During the chronic phase of the disease, these are still able to exit the cell cycle and to differentiate into mature cells that perform their normal functions. At some point, however, another mutation in a proto-oncogene, such as for example ras or in a tumor-suppressor gene such as for example p53 occur in one of these cells, thereby increase the rate of mitosis. The daughter cells fail to differentiate and the patient enters the crisis phase of the disease.

Acute myeloid leukemia (AML) is seen mostly in adults, and is uncommon in children. AML which is also called acute nonlymphocytic leukemia or ANLL is a form of cancer in which too many immature white blood cells or blasts are found in the blood and bone marrow, have failed to develop into mature infection-fighting cells. AML is a disease in which cancerous cells develop in the blood and bone marrow. There are several forms of AML, such as *inter alia* acute myeloblastic leukemia, acute promyelocytic leukemia, and acute monocytic leukemia. Untreated AML is a fatal disease with median survival time of 3 months. Adult acute myeloid leukemia (AML) is a disease in which cancer (malignant) cells are found in the blood and bone marrow. The patient exhibits abnormal bone marrow with at least 20% blasts and signs and symptoms of the disease, usually accompanied by an abnormal white blood cell count and differential, hematocrit/hemoglobin, and platelet count. There are many different subtypes, based on the microscopic appearance of the cells. Main treatment is chemotherapy, except all-trans retinoic acid (ATRA) is also used.

Acute lymphocytic leukemia (ALL), also called acute lymphoblastic leukemia and acute lymphoid leukemia, is a common leukemia. Most cases of leukemia in children are ALL. It occurs in very young children from 3 to 6 years old, but can also affect adults. ALL is an acute leukemia, which means it is a disease that gets worse quickly. In a healthy person, the bone marrow makes the blood stem cells that turn into the white blood cells, red blood cells and platelets in the blood. However, ALL patients' marrow makes too many lymphoblasts (immature white blood cells, or precursors of lymphocytes). These blast cells should turn into the white blood cells called lymphocytes, but they do not. So many blast cells grow that the marrow does not have room to make the normal red blood cells, white blood cells and platelets. ALL usually spread to brain and spinal cord. A definite diagnosis of ALL is made when blood and marrow samples examined under a microscope show large numbers of blast cells. There are 3 subtypes: L1: typically seen in children, L2: most often in adults, and L3: rare which has poor prognosis. Chemotherapy is usually used for treating ALL.

Chronic lymphocytic leukemia (CLL) affects mostly older adults, with an average age of 60 years old and is almost twice as common as CML. Mature looking lymphocytes are involved. Treatment is chemotherapy; but complete *remissions* are rare and the disease probably cannot be cured, so aggressive therapy is not usually suggested. Extent of disease is measured by the size and number of enlarged lymph nodes, and degree of anemia and thrombocytopenia (low platelet count). CLL patients having primitive cell with no changes in immunoglobulin genes and presence of CD38 protein on cell surface have a poorer prognosis with about half of patients living less than 8 to 10 years and half living more. Other CLL patients with more mature cells that have mutated immunoglobulin genes (they have matured enough to make antibodies) or no CD38 marker, usually live over 20 years.

Leukemias patients receive chemotherapy drugs as soon as possible after diagnosis. The goal of chemotherapy is to achieve remission and to restore normal blood cell production. Chemotherapy can control the chronic phase of the disease and induce remission. Chemotherapy uses strong drugs to kill the leukemia cells by stopping them from reproducing. Unfortunately, chemotherapy also kills normal cells, so patients receiving chemotherapy may have side effects, including nausea, tiredness and a higher risk of infections. With chemotherapy, 60-70% of adult patients and close to 80% of pediatric patients are expected to achieve remission. Even though chemotherapy regimens are effective for the treatment of leukemias, such as AML, 75-80% of patients who achieve remission relapse within 15 months and less than 5% of these individuals will survive long term.

Common chemotherapy drugs include hydroxyurea, busulfan, alpha-interferon (IFN-alpha), doxorubicin, fludarabine, cyclophosphamide, 2-drug regimen of cytarabine with daunorubicin or idarubicin, or a 3-drug regimen of cytarabine with daunorubicin or idarubicin, in conjunction with thioguanine.

Cytosine arabinoside-cytarabine commercialized under Cytosar-U is an antimetabolite specific for cells in the S-phase of the cell cycle, which acts through inhibition of DNA polymerase and cytosine incorporation into DNA and RNA. Daunorubicin also named Cerubidine and Idarubicin or Idamycin are topoisomerase-II inhibitors, inhibiting DNA and RNA polymerase. Mitoxantrone commercialized under Novantrone, inhibits cell proliferation by intercalating DNA and inhibiting topoisomerase II. IFN-alpha induces hematologic and even cytogenetic remission. Dose is 3-10 MU/day subcutaneously. However the cost of therapy is very high. Response to IFN-alpha therapy is longer than with conventional chemotherapy, but it has not proved efficacious in accelerated or blast phase. Hydroxyurea at a dose of 1-1.5 gm/day is capable of improving all the hematological abnormalities in CML. Busulfan is an alkylating agent given in dose of 4 to 8 mg/day. Busulfan may however cause severe myelosuppression.

Standard care of AML induction treatment consists however in cytarabine with an anthracycline, which is usually daunorubicin, idarubicin, or doxorubicin. Cytarabine based therapy induces remission in approximately 65% of adult AML patients and 70% of younger patients. Consolidation treatment also consists in a 2-drug regimens comprising cytarabine in combination with either daunorubicin, idarubicin, doxorubicin, or mitoxantrone. In remission, drug therapy is continued as untreated patients normally get recurrence of AML within four months. Cytarabine as monotherapy or cytarabine combinations are administered at extended intervals to keep blood cell counts in the normal range. The mean duration of the remission is approximately one and a half years, with 95% of those achieving primary remission relapsing. Response rates are much lower for second line cytarabine therapy as leukemias cells often develop resistance to these regimens.

For most patients, chemotherapy restores normal blood cell production within a few weeks, and microscopic examinations of their blood and marrow samples show no signs of leukemia cells, indicating that the disease is in remission. Although chemotherapy often brings long-lasting remissions in children, in adults, leukemia frequently returns, and thus necessitates more chemotherapy or a blood stem cell transplant.

Two types of blood stem cell transplants that can be used to treat leukemia patients, *i.e.,* autologous blood stem cell transplants use the patient's own blood stem cells. Allogeneic blood stem cell transplants use the blood stem cells of a donor, either someone from the patient's family or an unrelated donor.

For an autologous stem cell transplant, the patient's own blood stem cells are collected from his or her marrow or blood and frozen. After the patient has received high-dose chemotherapy and/or radiation therapy, the stem cells are put back into the patient.

For an allogeneic transplant, a donor is needed. The donor can be either related or unrelated to the patient. Related donors are usually siblings, provided that tissue type matches that of the patient.

Myelotarg^{™} an antibody specific to the CD33 receptor found on 80% of AML patients coupled to a cytotoxic agent, such as calicheamicin, has been approved as a second line of treatment for patients over 60s.

A bone marrow transplant preceded by high-dose chemotherapy and radiation therapy remains the standard treatment.

As described above, human leukemias such as mostly CML and ALL result from the translocation of the c-ABL gene on chromosome 9 and the bcr gene on chromosome 22 and generation of the Philadelphia chromosome which has been shown to be present in essentially all cases of chronic myelogenous leukemia and some of acute lymphocytic leukemia. The leukemogenic fusion proteins BCR-ABL present a constitutive tyrosine kinase activity and transform hematopoietic cells in vitro and cause CML- or ALL-like syndromes in mice.

Deregulation of tyrosine kinase activity through mutation or overexpression is a well-established mechanism underlying cell transformation (Hunter et al., 1985, supra; Ullrich et al., supra).

Protein tyrosine kinases comprise a large family of proteins, including many growth factor receptors and potential oncogenes, which differ from serine/threonine-specific protein kinases, and may further be defined as being receptors or non-receptors.

Receptor-type protein tyrosine kinases, which have a transmembrane topology have been studied extensively. The binding of a specific ligand to the extracellular domain of a receptor protein tyrosine kinase is thought to induce receptor dimerization and phosphorylation of tyrosine residues. Individual phosphotyrosine residues of the cytoplasmic domains of receptors may serve as specific binding sites that interact with a host of cytoplasmic signalling molecules, thereby activating various signal transduction pathways (Ullrich et al., 1990, Cell 61:203-212). Receptor-type tyrosine kinases include Class III receptor tyrosine kinase family, i.e., PDGFR, c-KIT, and Flt-3 (FMS-like receptor tyrosine kinase -3).

The intracellular, cytoplasmic, non-receptor protein tyrosine kinases may be broadly defined as those protein tyrosine kinases which do not contain a hydrophobic, transmembrane domain. Cytoplasmic protein tyrosine kinases can be divided into four distinct morphotypes: the SRC family (Martinez et al., 1987, Science 237:411-414; Sukegawa et al., 1987, Mol. Cell. Biol. 7:41-47; Yamanishi et al., 1987, 7:237-243; Marth et al., 1985, Cell 43:393-404; Dymecki et al., 1990, Science 247:332-336), the FMS family (Ruebroek et al., 1985, EMBO J. 4:2897-2903; Hao et al., 1989, Mol. Cell. Biol. 9:1587-1593), the ABL family (Shtivelman et al., 1986, Cell 47:277-284; Kruh et al., 1986, Science 234:1545-1548), and the JAK family.

More particularly, chronic melogenous leukemia (CML) results from the 210kDa form of BCR-ABL (p210) while the acute lymphocytic leukemia (ALL) is associated with an 185kDa form (p 185). It is known that BCR-ABL can activate multiple signal transduction pathways normally associated with the growth, survival, and differentiation of hematopoietic cells, such as for example the Ras pathway, MAPK (mitogen-activated protein kinase), STAT (signal transducer and activator of transcription), JNK/SAPK, NF-KB, c-myc, and phosphatidylinositol 3 kinase (PI-3K)/AKT. Constitutive activation of Stat transcription factors, such as Stat5, which in turns upregulates transcription of several genes, including Bclx and cyclin D1, necessary for the growth and anti-apoptotic effects observed in CML.

BCR-ABL further activates nonreceptor tyrosine kinases, particularly members of SRC family. To this regard, Lionberger et al. (J. Biol. Chem, Vol. 275, No. 24, pp. 18585) have shown that BCR-ABL interacts with two kinases of the SRC family, *e.g*., LYN and HCK tyrosine kinase, thereby facilitating the coupling of BCR-ABL to Ras. Particularly, it was demonstrated that HCK interacts with Brc-Abl via its domains SH2 and SH3 and phosphorylate p210 BCR-ABL on Tyr 177, and that interaction of of BCR-ABL with HCK or other SRC family members is essential for the transformation signaling by BCR-ABL. In effect, a kinase inactive mutant of HCK strongly suppressed BCR-ABL proliferative signals in myeloid leukemia cells, suggesting that HCK or other SRC-like kinases is required for BCR-ABL mediated transformation of myeloid leukemia cells to cytokine independence.

Also, it was demonstrated by Chai et al. (1997) that Stat5 displayed a pronounced phosphorylation on tyrosine residues in BCR-ABL-positive cells suggesting interaction with an activated tyrosine kinase. Stat5 plays an important role in BCR-ABL mediated leukemogenesis. Phosphorylation of the C-terminal portion of Stat5 is an essential step for its dimerization and activation of the transcriptional activity. However, it has been shown that inhibition of BCR-ABL does not significantly affect Stat5 phosphorylation, suggesting that other kinases would also be involved in the Stat5 phosphorylation. Klejman A. et al. (EMBO, 2002) suggested the existence of a BCR-ABL-HCK-Stat5 pathway as a major signaling pathway implicating HCK as an intermediate in BCR-ABL dependent activation of Stat5.

Activation of these pathways can lead to growth factor independence, increased proliferation, altered differentiation, and resistance to apoptosis. BCR-ABL which is found to be necessary for the initiation and maintenance of the CML phenotype together with SRC-like tyrosine kinase, and particularly HCK thus represents appropriate target for drug therapy.

The FDA recently approved a new drug for CML called imatinib mesylate or STI-571, an inhibitor of the BCR-ABL constitutive kinase activity, commercialized under the trade name Gleevec®, for treating patients with myeloid blast crisis and in patients with lymphoid blast crisis or Philadelphia chromosome-positive acute lymphoblastic leukemia. STI-571 is a 2-phenylaminopyrimidine that acts as a competitive inhibitor for the ATP binding pocket within the kinase region of BCR-ABL. In a phase I trial, 4 of 38 patients with myeloid blast crisis had a complete hematologic remission and 17 had a decrease in blasts in the marrow to 15% or less. Of the 20 patients in the lymphoid cohort, 4 had a complete hematologic response and 10 had a decrease in blasts in the marrow to 15% or less. Unfortunately, these responses have not been durable. Of 21 responding patients with myeloid blast crisis, 9 relapsed between 42 and 194 days; of the 14 responding patients with lymphoid disease, 12 relapsed with a median duration of time to relapse of 58 days. Seven of the 21 responding patients with myeloid blast crisis continue in remission with longest follow-up of 349 days. Two larger trials involving a total of 304 patients in blastic phase chronic myelogenous leukemia (CML) confirm a hematologic response rate of 52% to 55% and a major cytogenetic response rate of 16%, but the estimated 1-year survival is under 35%.

However, some CML patients develop resistance to the STI-571 drug and relapse, Gorre et al. (2001) showed that the resistance can be due to one single amino acid substitution in the kinase domain of BCR-ABL which rendered it unable to bind to the drug, or developed resistance through BCR-ABL gene amplification. However, STI-571 does not inhibit SRC-like kinases (Warmuth M et al. Blood, 2003).

It is thus necessary to develop additional drug that would inhibit BCR-ABL tyrosine kinase and mutants associated with resistance as well as additional targets such as the SRC-like kinases which have been shown to play a role in the BCR-ABL mediated transformation, for efficiently blocking the progression CML and ALL, particularly those in the accelerated phase or blast crisis, and to prevent or overcome BCR-ABL leukemia resistance.

The SRC family of non-receptor tyrosine kinases consists of eight members, *e.g*., SRC, LCK, FYN, YES, BLK, LYN, and FGR, and has been implicated in a wide variety of cellular signaling pathways in hematopoietic cells. Each hematopoietic cell lineage may express more than one member of the SRC family, for example, myeloid cells express HCK and LYN, T lymphocytes express LCK and FYN, and B lymphocytes express BLK and LYN (Corey et al., 1999). BCR-ABL expressed in myeloid cells activitate both HCK and LYN suggesting that these kinases might play a role in CML and AML. However, in ALL cells, BCR-ABL may stimulate different SRC-like family kinases, such as BLK, LCK and/or FYN.

Also, in addition to leukemias and lymphomas, a number of studies have linked SRC expression to cancers such as colon, breast, hepatic and pancreatic cancers (Lutz et al., BBRC 1998). SRC-like tyrosine kinase proteins as well as FMS-like tyrosine kinase proteins have been showed to be involved in growth factor signal transduction, cell cycle progression and neoplastic transformation is now well-established. Subversion of normal growth control pathways leading to proliferation and survival of myeloid progenitors and oncogenesis has been shown to be caused by activation and overexpression of protein tyrosine kinase which constitute a large group of dominant oncogenic proteins.

Studies with dominant-negative and SRC inhibitors suggest that SRC kinases may contribute to the proliferation and survival of myeloid cells expressing BCR-ABL. Hu et al. (Nature Genetics 2004) have showed that SRC-like kinases are required for the induction of B-ALL by BCR-ABL but are dispensable for induction of CML-like myeloproliferative disease.

SRC kinases expressed in myeloid cells, including HCK, LYN, FYN, and FGR which are essential intermediates coupling BCR-ABL to Stat5 and Ras signaling pathway seem to be rationale alternative targets for CML drug therapy, particularly in patients refractory to treatment with STI-571 (Wilson et al, Oncogene 2002). Also, SRC-like family kinases would constitute beneficial therapeutic targets in Philadelpia chromosome-positive and B-cell acute lymphoblastic leukemia (B-ALL).

Combinatorial therapy of a compound having a dual specificity, i.e., as SRC and BCR-ABL inhibitors would provide a significant therapeutic benefit. For example, combination of an inhibitor of ABL kinase, and an inhibitor of BCR-ABL downstream effectors, such as SRC-like kinase, and particularly HCK would provide synergistic anti-leukemia effects. Simultaneous inhibition of SRC-like family kinases and BCR-ABL would benefit acute leukemia patients..

The present disclosure relates to a potent anti-proliferative compound for treating CML or ALL patients that are Philadelphia chromosome positive and refractory to standard of care for CML or ALL treatment, that is capable of inhibiting ABL or BCR-ABL tyrosine kinase, or an activating or treatment resistant mutant thereof. The compound and composition of the present disclosure advantageously inhibit SRC-like kinase, such as in particular HCK.

In addition to the above described mutations in the ABL and BCR genes, additional mutations of receptor tyrosine kinases, including cKIT, PDGFR and FLT-3, have been found in human leukemia, and in particular AML. FLT-3 (fms like tyrosine kinase 3), KIT and PDGFR are members of the class III receptor tyrosine kinases.

FLT-3 gene encodes a tyrosine kinase receptor that is expressed in normal human bone marrow, selectively in CD34⁺ immature hematopoietic stem or progenitor cells and in CD34-dendritic cell progenitors. FLT-3 tyrosine kinase regulates proliferation and differentiation of hematopoietic stem cells. It is also expressed in placenta, gonads and brain, wherein it may regulates proliferation and apoptosis events. FLT-3 signalling pathway is important in the development of hematopoietic stem cells, B-cell progenitors, dendritic cells and natural killer (NK) cells.

Mutations of FLT-3 include any changes to any FLT-3 gene sequence including point mutations, deletions, insertions, internal tandem duplications, polymorphisms. Examples of known mutations in FLT-3 are mutations in the activation loop of the kinase domain, *i.e*., D835, 1836, N841, Y842, and insertion between G840 and N841. These mutations and insertion maintain the activation loop of the FLT-3 tyrosine kinase in an open configuration which allows ATP to access to its binding site. Mutations in the activating loop of FLT-3 are known to result in constitutive activation based on homology to other tyrosine kinase receptors such as c-KIT. Point mutation at amino acid residue 835 in human FLT-3 (D835), identified in approximately 7% of patients (Abu-Duhier et al (Br J Haematol June 2001; 113(4):983-8; Abu-Duhier et al., British J. of Heamotology, Vol. 11, pages 190-195 (2000). FLT- 3 antiapoptotic pathway involves PK3K/AKT activation, as well as RAS and MAPK which are usually transient, but becomes constitutive in FLT-3-ITD.

Internal tandem duplication (ITD) of the juxtamembrane (JM) domain-coding sequence of the FLT-3 gene is one of the most frequent mutations. The ITD consists of the duplication of 3 to 400 nucleotides in the juxtamembrane domain and often an insertion of one or two novel amino acids prior to the tandem repeat Such ITD modifies the JM conformation which regulates the autoinhibitory mechanism of the tyrosine kinase activity, and results in constitutive activation of the FLT-3 kinase domain and downstream pathways.

The internal tandem duplication (ITD) mutations of the receptor tyrosine kinase FLT-3 have been found in 20-30% of patients in with AML (Levis et al., Blood, vol. 98, pages 885-887, 2001). ITD are internal tandem duplications, mutations found in the juxtamembrane domain, repeats range in size but the duplicated sequence appears always to be in frame. Such mutations may be diagnosed in FLT-3-ITD positive patients using PCR and gel electrophoresis testing of genomic DNA from AML patients. The FLT-3 mutant is found in some patients with AML (in 20-30% of AML adult patients and in around 14% of AML children) and 6% of myelodysplastic syndrome cases (MDS), whereas it appears rare in CML and lymphoid malignancies (ALL or CLL). The presence of the FLT-3 gene mutation is related to high peripheral white blood cell counts. The ITD of the FLT-3 gene sometimes emerged during progression of MDS or at relapse of AML which had no ITD at first diagnosis. This suggests that FLT-3 mutation promotes leukemia progression. (Zhao et al., Leukemia, vol. 14, pages 374-378 (2000)).

Patients with AML have FLT-3-1TD (internal tandem duplication) positive typically exhibit poor response to traditional chemotherapy. In effect, FLT-3 mutations constitutively activate the receptor and appear to be associated with a poor response to chemotherapy. Constitutive active forms of FLT-3 are able to transform hematopoietic -cell lines, but not primary cell lines (thus not sufficient for full transformation). Evidence suggests that this constitutive activation is leukemogenic, rendering this receptor a potential target for specific therapy.

Patients bearing ITD mutant FLT-3 are known to have poor prognosis, high relapse rate and decreased overall survival after conventional treatment, relative to non ITD mutant patients. Current therapies for AML have poor patient response rates and poor toxicity profiles. Therapies are generally nonspecific and not targeted exclusively to the diseased cells or to the mechanism which drives the malignancy. Inhibition of FLT-3 which mediates cell survival and proliferation signals would directly target the leukemic cells, inhibit signaling resulting in elimination of leukemic cell population.

A compound called CEP-701 from the company Cephalon is currently in phase I/II as monotherapy for patients with refractory, relapsed, or poor-risk AML expressing FLT-3 activating mutation (Blood, 2004; 103:3669-3676). Also, a phase II for testing CEP-701 in combination with chemotherapy is ongoing in patients with relapsed acute myeloid leukemia with mutant FLT-3. PKC-412 is also used in a phase II clinical trial as monotherapy for patients with refractory, relapsed or poor-risk AML expressing FLT-3 activating mutation (Blood 2003; 102:2270a).

Man-518 is currently tested in a phase I clinical trial in patients with AML and MDS (Blood 2002; 100:558a).

Sugen is developing and testing two compounds, SU11248 in a phase I single dose escalating pharmacokinetic and toxicity clinical study in AML patients (Clin Can Res 2003 19:5465) and SU5416 in phase II trials as monotherapy in patients with refractory AML and MDS (Blood 2003; 102:795) not restricted to FLT-3 mutant. However, resistance and cell escape have been shown when using SU11248.

Accordingly, there is a great need to develop potent therapy against standard care refractory human leukemias that would increase the survival rate and that would be capable of inhibiting more than one protein kinase, including BCR-ABL fusion tyrosine kinase, SRC-like tyrosine kinases, and Class III receptor tyrosine kinase, chosen among KIT, FLT-3 and PDGR tyrosine kinase, and also useful in treating various conditions associated with protein kinase activation. Particularly the potent TK inhibitors according to the present disclosure are useful for treating cell proliferative disorders, such as cancer and human leukemias. The compounds of the present disclosure are particularly useful for treating AML or MDS patients positive for FLT-3-ITD but not restricted to FLT-3-ITD, or an activating mutant of FLT-3.

The present disclosure thus relates to a potent antiproliferative and proapoptotic compound family that is capable to inhibit more than one tyrosine kinase, and compositions and methods for the prevention and treatment of cell proliferative disorders, such as cancers and leukemias. Preferably, compounds and compositions of the present invention are used in combination with standard care for ALL, CML or AML treatment. In particular, they are useful in the treatment of patients with AML who are FLT-3-ITD positive, and CML or ALL that are Philadelphia chromosome positive, and refractory to standard care.

In addition, patients diagnosed with cell proliferative disorders, such sarcomas, melanomas, and solid tumors where the pathophysiology indicates that FLT-3-ITD or FLT-3, PDGFR, KIT tyrosine kinases, SRC-like and BCR-ABL tyrosine kinases are associated with the malignancy may be treated by administering the compounds of the present disclosure either alone, but preferably in combination with the standard of care.

### Summary of the Invention (i)

The present invention relates to a method for screening for a combination of biological compounds capable of abrogating mature vasculature in a patient's tumor comprising: (i) introducing tumor cells to a collection of microvascular cells including mural cells and pericytes; (ii) regularly administering to the tumor cells a PDGF-receptor beta inhibitor; (iii) administering to the tumor cells one or more anti-angiogenic or anti-cancer agents; and (iv) measuring the one or more tumor volume, mean vessel density, EC division, or EC apoptosis in the cells compared to a control, whereby a difference between the control and the cells administered the PDGF-receptor beta inhibitor and the one or more anti-cancer agents can be detected.

In another aspect, the present invention relates to a method for screening for a combination of biological compounds capable of abrogating mature vasculature in a patient's tumor comprising (i) introducing tumor cells to a collection of microvascular cells including mural cells and pericytes, with the exception that the collection of microvascular cells does not consist of bone cells or bone tissue; (ii) regularly administering to the tumor cells a PDGF-receptor beta inhibitor; (iii) administering to the tumor cells one or more anti-cancer agents, abrogen polypeptide, and/or kringle polypeptide; and (iv) measuring the one or more of tumor volume, mean vessel density, EC division, or EC apoptosis in the cells compared to a control, whereby a difference between the control and the cells administered the PDGF-receptor beta inhibitor and the one or more anti-angiogenic agents or chemotherapeutic agents can be detected. Abrogating mature vasculature is meant to refer to the ability to reduce or prevent the development or proliferation of blood vessels at or near the site of a tumor or the ability to destroy mature blood vessels at or near the site of a tumor. In another aspect, abrogating mature vasculature means the ability to prevent cell division in a mature blood vessel so that new vessel formation is prevented. For example, abrogating mature vasculature can mean preventing cell division in endothelial cells and smooth muscle cells within arterioles of perivasculature of a tumor.

In another aspect, the present invention provides for a method for screening for a combination of biological compounds capable of inhibiting the activation loop between the endothelial cell and smooth muscle cells within arterioles of perivasculature of a patient's tumor comprising (i) introducing tumor cells to a collection of microvascular cells including mural cells and pericytes, with the exception that the collection of microvascular cells does not consist of bone cells or bone tissue; (ii) regularly administering to the tumor cells a PDGF-recepfor beta inhibitor; (iii) administering to the tumor cells one or more anti-cancer agents, abrogen polypeptide, and/or kringle polypeptide; (iv) and measuring the one or more of tumor volume, mean vessel density, EC division, or EC apoptosis in the cells compared to a control, whereby a difference between the control and the cells administered the PDGF-receptor beta inhibitor and the one or more anti-angiogenic and/or anti-chemotherapeutic agents can be detected.

(ii) According to an embodiment, the PDGF-receptor beta inhibitor is the extracellular binding domain of the PDGF-Rβ fused to the constant region of an lg (PDGF-Rβ-Fc). The one or more anti-cancer agents is selected from the group consisting of taxotere, paclitaxel, docetaxel, gemcitabine, fluorouracil, mitomycin, and epirubicin.

Alternatively, it is possible to use one chemotherapeutic agent selected from the group of anti-microtubule agents, platinum coordination complexes, alkylating agents, antibiotic agents, topoisomerase II inhibitors, antimetabolites, topoisomerase I inhibitors, hormones and hormone analogues, signal transduction pathway inhibitors, immunotherapeutic agents, proapoptotic agents, and cell cycle signaling inhibitors.

According to the invention, the method of measuring EC division or EC apoptosis comprises TUNEL. (in situ terminal dUTP nick-end labeling).

In one aspect of the invention, the collection of microvascular cells is one of the group consisting of brain tissue, lung tissue, pancreas tissue, ovarian tissue, liver tissue, lymph tissue, or skin tissue.

The treatment regimen is of the method of the invention is more than 10 days, and preferably more than 20 days.

Methods for studying drug effects and interactions are discussed in Topaly et al. (2002, British Journal of Haematology, 119; 3-14).

### Detailed Description of Exemplary Embodiments

### Definitions

### "Patient" includes both human and other mammals.

"Effective amount" means an amount of compound of the present invention effective in inhibiting PDGF-R tyrosine kinase activity and/or LCK tyrosine kinase activity, and thus producing the desired therapeutic effect.

"Alkyl" means aliphatic hydrocarbon group which may be branched-or straight-chained having about 1 to about 10 carbon atoms. Preferred alkyl is "loweralkyl" having about 1 to about 6 carbon atoms. Branched means that one or more lower alkyl groups such as methyl, ethyl or propyl are attached to a linear alkyl chain. The alkyl group is also optionally substituted by alkoxy, halo, carboxy, hydroxy or R⁵R⁶N-. Examples of alkyl include methyl, fluoromethyl, difluoromethyl, trifluoromethyl, ethyl, n-propyl, isopropyl, butyl, sec-butyl, t-butyl, amyl and hexyl.

"Alkenyl" means an aliphatic hydrocarbon group containing a carbon-carbon double bond and which may be straight or branched having about 2 to about 10 carbon atoms in the chain. Preferred alkenyl groups have 2 to about 6 carbon atoms in the chain; and more preferably about 2 to about 4 carbon atoms in the chain. Branched means that one or more lower alkyl groups such as methyl, ethyl or propyl are attached to a linear alkenyl chain. "Lower alkenyl" means about 2 to about 4 carbon atoms in the chain which may be straight or branched. The alkenyl group may be substituted by carbalkoxy. Exemplary alkenyl groups include ethenyl, propenyl, n-butenyl, i-butenyl, 3-methylbut-2-enyl, n-pentenyl, heptenyl, octenyl, cyclohexylbutenyl and decenyl.

### "Ethylenyl" means a -CH=CH- group.

"Cycloalkyl" means a non-aromatic mono- or multicyclic ring system of about 3 to about 10 carbon atoms. The cycloalkyl group may be substituted by one or more, preferably one to three, more preferably one to two, of the following "cycloalkyl substituents", alkyl, hydroxy, acyloxy, alkoxy, halo, R₅R₆N-, acylR₅N-, carboxy or R₅R₆NCO-- substituents, more preferred substituents are alkyl, hydroxy, acyloxy, alkoxy, and R₅R₆NCO-. Furthermore, when the cycloalkyl group is substituted with at least two hydroxy substituents, then at least two of the hydroxy substituents may be ketalated or acetalated with an aldehyde or ketone of one to six carbon atoms to form the corresponding ketal or acetal. "Hydroxycycloalkyl" means HO-cycloalkyl wherein the cycloalkyl may be substituted as noted. When the hydroxycycloalkyl group is derived from a cycloalkyl group which is also substituted with hydroxy, two of the hydroxy substituents may be ketalated or acetalated with an aldehyde or ketone of one to six carbon atoms to form the corresponding ketal or acetal. Ketalization of a gem-diol results in formation of a spiro fused ring system. A preferred spiro cycloalkyl ring is 1,4-dioxaspiro[4,5]dec-8-yl. Preferred unsubstituted or substituted monocyclic cycloalkyl rings include cyclopentyl, hydroxycyclopentyl, fluorocyclopentyl, cyclohexyl, hydroxycyclohexyl, hydroxymethylcyclohexyl and cycloheptyl; more preferred are hydroxycyclohexyl and hydroxycyclopentyl. Exemplary multicyclic cycloalkyl rings include 1-decalin, adamant-(1- or 2-)yl, [2.2.1]bicycloheptanyl (norbornyl), hydroxy[2.2.1]bicycloheptanyl (hydroxynorbornyl), [2.2.2]bicyclooctanyl and hydroxy[2.2.2]bicyclooctanyl; more preferred are hydroxy[2.2.1]bicycloheptanyl (hydroxynorbornyl), and hydroxy[2.2.2]bicyclooctanyl.

"Cycloalkenyl" means a non-aromatic monocyclic or multicyclic ring system containing a carbon- carbon double bond and having about 3 to about 10 carbon atoms. The cycloalkenyl group may be substituted by one or more, preferably one to three, more preferably one to two cycloalkyl substituents as described above. "Hydroxycycloalkenyl" means HO-cycloalkenyl wherein the cycloalkyl may be substituted as noted. Preferred unsubstituted or substituted monocyclic cycloalkenyl rings include cyclopentenyl, cyclohexenyl, hydroxycyclopentenyl, hydroxycyclohexenyl and cycloheptenyl; more preferred is hydroxycyclopentenyl and hydroxycyclohexenyl. Preferred multicyclic cycloalkenyl rings include [2.2.1]bicycloheptenyl (norbomenyl) and [2.2.2]bicyclooctenyl.

"Aryl" means aromatic carbocyclic radical containing about 6 to about 10 carbon atoms. Exemplary aryl include phenyl or naphthyl, or phenyl or naphthyl substituted with one or more aryl group substituents which may be the same or different, where "aryl group substituent" includes hydrogen, hydroxy, halo, alkyl, alkoxy, carboxy, alkoxycarbonyl or Y¹ Y²NCO-, wherein Y¹ and Y² are independently hydrogen or alkyl. Preferred aryl group substituents include hydrogen, halo and alkoxy.

"Heteroaryl" means about a 5- to about a 10-membered aromatic monocyclic or multicyclic hydrocarbon ring system in which one or more of the carbon atoms in the ring system is/are element(s) other than carbon, for example nitrogen, oxygen or sulfur. The "heteroaryl" may also be substituted by one or more of the above-mentioned "aryl group substituents". Exemplary heteroaryl groups include substituted pyrazinyl, furanyl, thienyl, pyridyl, pyrimidinyl, isoxazolyl, isothiazolyl, oxazolyl, thiazolyl, pyrazolyl, furazanyl, pyrrolyl, imidazo[2,1-b]thiazolyl, benzofurazanyl, indolyl, azaindolyl, benzimidazolyl, benzothienyl, quinolinyl, imidazolyl and isoquinolinyl.

"Heterocyclyl" means an about 4 to about 10 member monocyclic or multicyclic ring system wherein one or more of the atoms in the ring system is an element other than carbon chosen amongst nitrogen, oxygen or sulfur. The heterocyclyl group may be substituted by one or more, preferably one to three, more preferably one to two cycloalkyl substituents as described above. "Hydroxyheterocyclyl" means HO-heterocyclyl wherein the heterocyclyl may be substituted as noted. "Azaheterocyclyl" means a heterocyclyl as noted herein wherein at least one of the ring atoms is nitrogen. Exemplary heterocyclyl moieties include quinuclidyl, pentamethylenesulfide, tetrahydropyranyl, tetrahydrothiophenyl, pyrrolidinyl, tetrahydrofuranyl or 7-oxabicyclo[2.2.1]heptanyl.

"Heterocyclylcarbonyloxy" means a heterocyclyl group as defined herein which is attached to the parent molecular moiety through a carbonyloxy (-C(O)O-) group. The heterocyclyl moiety is optionally substituted by one or more, preferably one to three, more preferably one cycloalkyl substituents as defined above. A representative heterocyclylcarbonyloxy is [1,4']-bipiperidin-1'-ylcarbonyloxy.

"Heterocyclenyl" means a heterocyclyl ring system as defined herein which contains at least one carbon-carbon or carbon-nitrogen double bond. The heterocyclenyl group may be substituted by one or more, preferably one to three, more preferably one to two cycloalkyl substituents as described above.

"Hydroxyheterocyclenyl" means HO-heterocyclenyl wherein the heterocyclenyl may be substituted as noted. "Azaheterocyclenyl" means a heterocyclenyl as noted herein wherein at least one of the ring atoms is nitrogen. Representative monocyclic heterocyclenyl groups include 1,2,3,4-tetrahydrohydropyridine, 1,2-dihydropyridyl, 1,4-dihydropyridyl, 1,2,3,6-tetrahydropyridine, 1,4,5,6-tetrahydropyrimidine, 3,4-dihydro-2H-pyran, 2-pyrrolinyl, 3-pyrrolinyl, 2-imidazolinyl, 2-pyrazolinyl, tetrahydrothiophenyl, tetrahydrothiopyranyl, and the like.

"Acyl" means an H--CO-- or alkyl-CO-- group in which the alkyl group is as previously described. Preferred acyls contain a lower alkyl. Exemplary acyl groups include formyl, acetyl, propanoyl, 2-methylpropanoyl, butanoyl and palmitoyl.

"Aroyl" means an aryl-CO-- group in which the alkyl group is as previously described. Exemplary groups include benzoyl and 1- and 2-naphthoyl.

"Alkoxy" means an alkyl-O-- group in which the alkyl group is as previously described. Preferred alkoxy is "lower alkoxy" having about 1 to about 6 carbon atoms. The alkoxy may be optionally substituted by one or more amino, alkoxy, carboxy, alkoxycarbonyl, carboxyaryl, carbamoyl or heterocyclyl groups. Exemplary alkoxy groups include methoxy, ethoxy, n-propoxy, i-propoxy, n-butoxy, heptoxy, 2-(morpbolin-4-yl)ethoxy, 2-(ethoxy)ethoxy, 2-(4-methylpiperazin-1-yl)ethoxy, carbamoyl, N-methylcarbamoyl, N,N-dimethylcarbamoyl, carboxymethoxy and methoxycarbonylmethoxy.

"Cycloalkyloxy" means a cycloalkyl-O-- group in which the cycloalkyl group is as previously described. Exemplary cycloalkyloxy groups include cyclopentyloxy, cyclohexyloxy, hydrocyclopentyloxy and hydroxycyclohexyloxy.

"Heterocyclyloxy" means a heterocyclyl-O-- group in which the heterocyclyl group is as previously described. Exemplary heterocyclyloxy groups include quinuclidyloxy, pentamethylenesulfideoxy, tetrahydropyranyloxy, tetrahydrothiophenyloxy, pyrrolidinyloxy, tetrahydrofuranyloxy or 7-oxabicyclo[2.2.1]heptanyloxy, hydroxytetrahydropyranyloxy and hydroxy-7-oxabicyclo[2.2.1]heptanyloxy.

"Aryloxy" means aryl-O-- group in which the aryl group is as previously described.

"Heteroaryloxy" means heteroaryl-O- group in which the heteroaryl group is as previously described.

"Acyloxy" means an acyl-O- group in which the acyl group is as previously described.

"Carboxy" means a HO(O)C- (carboxylic acid) group.

"R₅R₆N-" means a substituted or unsubstituted amino group, wherein R₅ and R₆ are as previously described. Exemplary groups include amino (H₂N-), methylamino, ethylmethylamino, dimethylamino and diethylamino.

"R₅R₆NCO-" means a substituted or unsubstituted carbamoyl group, wherein R₅ and R₆ are as previously described. Exemplary groups are carbamoyl (H₂NCO-), N-methylcarbamoyl (MeNHCO-) and N,N-dimethyylaminocarbamoyl (Me₂ NCO-).

"AcylR₅N-" means an acylamino group wherein R₅ and acyl are as defined herein.

"Halo" means fluoro, chloro, bromo, or iodo. Preferred are fluoro, chloro or bromo, and more preferred are fluoro or chloro.

"Prodrug" means a form of the compound of formula I suitable for administration to a patient without undue toxicity, irritation, allergic response, and the like, and effective for their intended use, including ketal, ester and zwitterionic forms. A prodrug is transformed in vivo to yield the parent compound of the above formula, for example by hydrolysis in blood. A thorough discussion is provided in T. Higuchi and V. Stella, Pro-drugs as Novel Delivery Systems Vol. 14 of the A. C. S. Symposium Series, and in Edward B. Roche, ed., Bioreversible Carriers in Drug Design, American Pharmaceutical Association and Pergamon Press, 1987, both of which are incorporated herein by reference.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolable solvates. Representative solvates include ethanolates, methanolates, and the like. "Hydrate" is a solvate wherein the solvent molecule(s) is/are H₂O.

### Non-limiting example

A preferred compound aspect of the disclosure is a compound of formula I 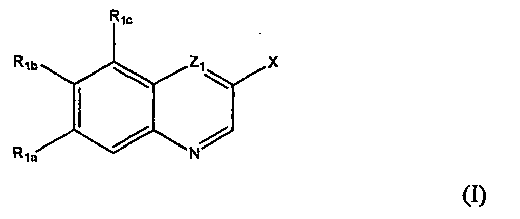 wherein
L₁ is (CR₃ₐR_{3b})ᵣ or (CR₃ₐR_{3b})ₘ -Z₃ -(CR_{3'a} R_{3'b})ₙ ;
L₂ is (CR₃ₐR_{3b})ₚ -Z₄ -(CR_{3'a} R_{3'b})_{q} or ethenyl;
Z₂ is optionally substituted hydroxycycloalkyl or optionally substituted hydroxyheterocyclyl;
Z₄ is O and NR₄;
m is 0;
n is 2 or 3;
p+q=0 or 1;
R₁ₐ and R_{1b} are independently optionally substituted alkyl, optionally substituted alkoxy, optionally substituted cycloalkyloxy, optionally substituted heterocyclyloxy, or R₅R₆N-, or one of R₁ₐ and R_{1b} is hydrogen or halo;
R_{1c} is hydrogen, optionally substituted alkyl or optionally substituted alkoxy;
R₃ₐ, R_{3b}, R_{3'a} and R_{3'b} are independently hydrogen or lower alkyl;
R₄ is hydrogen; and
R₅ and R₆ taken together with the nitrogen atom to which R₅ and R₆ are attached form azaheterocyclyl, or an N-oxide thereof, hydrate thereof, solvate thereof, prodrug thereof, or pharmaceutically acceptable salt thereof.
Another preferred compound aspect of the disclosure is a compound of formula (I) 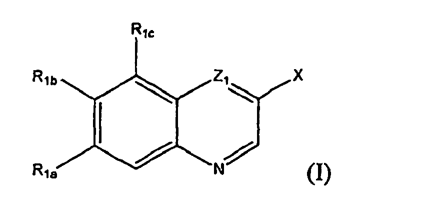 wherein X is L₂Z₂;
L₂ is (CR₃ₐ R_{3b})ₚ -Z₄ -(CR_{3'a} R_{3'b})_{q};
Z₂ is optionally substituted hydroxycycloalkyl;
Z₄ is O and NR₄ ;
p is 0;
q is 0 or 1;
R₁ₐ and R_{1b} are independently optionally substituted alkyl, optionally substituted alkoxy, optionally substituted cycloalkyloxy or optionally substituted heterocyclyloxy, or one of R₁ₐ and R_{1b} is hydrogen or halo and the other of R₁ₐ and R_{1b} is optionally substituted alkyl, optionally substituted alkoxy, optionally substituted cycloalkyloxy or optionally substituted heterocyclyloxy;
R_{1c} is hydrogen;
R_{3'a} and R_{3'b} are independently hydrogen; and
R₄ is hydrogen, or an N-oxide thereof, hydrate thereof, solvate thereof, prodrug thereof, or pharmaceutically acceptable salt thereof.

Another preferred compound aspect of the disclosure is a compound of formula I wherein R₁ₐ and R_{1b} are independently optionally hydroxy substituted lower alkyl, hydroxy, lower alkoxy, cycloalkyloxy, heterocyclyloxy, or one of R₁ₐ and R_{1b} is hydrogen or halo and the other of R₁ₐ and R_{1b} is optionally hydroxy substituted lower alkyl, hydroxy, lower alkoxy, cycloalkyloxy, heterocyclyloxy.

Another preferred compound aspect of the disclosure is a compound of formula I wherein R₁ₐ and R_{1b} are independently heterocyclylcarbonyloxy or optionally substituted lower alkoxy; more preferably, the lower alkoxy is methoxy or ethoxy.

Another preferred compound aspect of the disclosure is a compound of formula I wherein R₁ₐ and R_{1b} are lower alkyl; more preferably the lower alkyl is methyl or ethyl.

Another preferred compound aspect of the disclosure is a compound of formula I wherein one of R₁ₐ and R_{1b} is lower alkoxy, and the other of R.sub.la and R.sub.1b is halo; more preferably the lower alkoxy is methoxy or ethoxy, and the alo is chloro or bromo.

Another preferred compound aspect of the disclosure is a compound of formula I wherein one of R₁ₐ and R_{1b} is lower alkyl, and the other of R.sub.la and R.sub.lb is lower alkoxy; more preferably the lower alkoxy is methoxy or ethoxy, and the lower alkyl is methyl or ethyl.

Another preferred compound aspect of the disclosure is a compound of formula I wherein one of R₁ₐ and R_{1b} is lower alkoxy, and the other of R₁ₐ and R_{1b} is cycloalkyloxy; more preferably the lower alkoxy is methoxy or ethoxy, and the cycloalkyloxy is cyclopentyloxy or cyclohexyloxy.

Another preferred compound aspect of the disclosure is a compound of formula I wherein one of R₁ₐ and R_{1b} is hydrogen, and the other of R₁ₐ and R_{1b} is lower alkoxy, cycloalkyloxy or heterocyclyloxy; more preferably the lower alkoxy is methoxy or ethoxy, and the cycloalkyloxy is cyclopentyloxy or cyclohexyloxy, and the heterocyclyloxy is furanyloxy.

Another preferred compound aspect of the disclosure is a compound of formula I wherein R₁ₐ and R_{1b} are lower alkoxy wherein the lower alkoxy is optionally substituted with alkoxy, heterocyclyl, carboxy, alkoxycarbonyl or carbamoyl.

Another preferred compound aspect of the disclosure is a compound of formula I wherein one of R₁ₐ and R_{1b} is unsubstituted lower alkoxy and the other of R₁ₐ and R_{1b} optionally substituted heterocyclylcarbonyloxy or is lower alkoxy substituted with alkoxy, heterocyclyl, carboxy, alkoxycarbonyl or carbamoyl.

Another preferred compound aspect of the disclosure is a compound of formula I wherein one of R₁ₐ and R_{1b} is methoxy and the other of R₁ₐ and R_{1b} is [1,4']-bipiperadin-1'-ylcarbonyloxy, 2-(ethoxy)ethoxy, 2-(4-morpholinyl)ethoxy, 2-(4-methylpiperazin-1-yl)ethoxy, carboxymethoxy, methoxycarbonylmethoxy, aminocarbonylmethoxy, N-methylaminocarbonylmethoxy or N,N-dimethylaminocarbonylmethoxy.

Another preferred compound aspect of the disclosure is a compound of formula I wherein R_{1c} is hydrogen, lower alkyl or lower alkoxy; more preferably the lower alkoxy is methoxy or ethoxy.

Another preferred compound aspect of the disclosure is a compound of formula I wherein Z₁ is CH.

Another preferred compound aspect of the disclosure is a compound of formula I wherein Z₁ is N.

Another preferred compound aspect of the disclosure is a compound of formula I wherein Z₂ is optionally substituted hydroxycycloalkyl.

Another preferred compound aspect of the disclosure is a compound of formula I wherein p and q are 0.

Another preferred compound aspect of the disclosure is a compound of formula I wherein p+q=1.

Another preferred compound aspect of the disclosure is a compound of formula I wherein Z₄ is O.

Another preferred compound aspect of the disclosure is a compound of formula I wherein Z₄ is O, and p and q are 0.

Another preferred compound aspect of the disclosure is a compound of formula I wherein Z₄ is O, and p+q=1.

Another preferred compound aspect of the disclosure is a compound of formula I wherein Z₄ is NR₄.

Another preferred compound aspect of the disclosure is a compound of formula I wherein Z₄ is NR₄, and p and q are 0.

Another preferred compound aspect of the disclosure is a compound of formula I wherein Z₄ is NR₄, and m+n=1.

Another preferred compound aspect of the disclosure is a compound of formula I wherein Z₄ is S.

Another preferred compound aspect of the disclosure is a compound of formula I wherein Z₄ is S, and p and q are 0.

Another preferred compound aspect of the disclosure is a compound of formula I wherein Z₄ is S, and p +q=1.

Another preferred compound aspect of the disclosure is a compound of formula I wherein Z₂ is (hydroxy or alkyl) substituted hydroxycycloalkyl, more preferred is (lower alkyl)hydroxycycloalkyl.

Preferred compounds according to the disclosure are selected from the following species:
trans-4-(7-Chloro-6-methoxyquinoxalin-2-ylamino)-cyclohexanol;
trans-4-(6-Chloro-7-methoxyquinoxalin-2-ylamino)-cyclohexanol;
trans-4-(6,7-Dimethoxyquinoxalin-2-ylamino)-cyclohexanol;
cis-4-(6,7-Dimethoxyquinoxalin-2-ylamino)-cyclohexanol;
(2endo,5exo)-5-(6,7-Dimethoxyquinoxalin-2-ylamino)-bicyclo[2.2.1]heptan-2-ol;
(2exo,5exo)-5-(6,7-Dimethoxyquinoxalin-2-ylamino)-bicyclo[2.2.1]heptan-2-ol;
(2endo,3exo,5exo)-5-(6,7-Dimethoxyquinoxalin-2-ylamino)-bicyclo[2.2.]heptane-2,3-diol;
cis-2-(6-Methoxyquinoxalin-2-ylamino)-cyclopentanol;
trans-2-(6-Methoxyquinoxalin-2-ylamino)-cyclopentanol;
trans-4-(6-Methoxyquinoxalin-2-ylamino)-cyclohexanol;
[3aR,4S,6R,6aS]-6-(6,7-Dimethoxyquinoxalin-2-ylamino)-2,2-dimethyl-tetrahydro-cyclopenta[1,3]dioxole-4-carboxylic ethylamide;
2-(1,4-Dioxa-spiro[4,5]dec-8-yloxy)-6,7-dimethoxyquinoxaline;
4-(6,7-Dimethoxyquinoxatin-2-yloxymethyl)-cyclohexanol;
3-(6,7-Dimethoxyquinoxalin-2-yloxy)cyclohexanol;
4-(6,7-Dimethoxyquinoxalin-2-yloxy)-cyclohexanol;
5-(6,7-Dimethoxyquinoxalin-2-yloxy)-bicyclo[2.2.1]heptane-2,3-diol;
Acetic acid cis-4-(6,7-dimethoxyquinoxalin-2-yloxy)-cyclohexyl ester;
cis-4-(6,7-Dimethoxyquinoxalin-2-yloxy)-cyclohexanol;
Dimethyl-carbamic acid 4-(6,7-dimethoxyquinoxalin-2-yloxy)-cyclohexyl ester;
trans-4-(6,7-Dimethoxy-4-oxyquinoxalin-2-ylamino)-cyclohexanol;
Acetic acid trans-4-(6,7-dimethoxyquinoxalin-2-ylamino)-cyclohexyl ester;
(2exo,5exo)5-(6,7-Dimethoxyquinoxalin-2-ylamino)-bicyclo[2.2.1]-heptan-2-ol;
(2endo,5exo)-5-(6,7-Dimethoxyquinoline-2-ylamino)-bicyclo[2.2.1]heptan-2-ol;
(2exo,6exo)-6-(6,7-Dimethoxyquinolin-2-ylamino)-bicyclo[2.2.1]heptan-2-ol;
4-(6,7-Dimethoxyquinoxalin-2-ylamino)-2-methyl-cyclohexanol;
(2trans,4trans)-4-(6,7-Dimethoxyquinoxalin-2-ylamino)-2-methyl-cyclohexanol;
(+)-(2trans,4trans)-4-(6,7-Dimethoxyquinoxalin-2-ylamino)-2-methyl-cyclohex anol;
(-)-(2trans,4trans)-4-(6,7-Dimethoxyquinoxalin-2-ylamino)-2-methyl-cyclohex anol;
(2trans,4trans)-4-(6,7-Dimethoxyquinoxalin-2-ylamino)-2-methyl-cyclohexanol,
(2cis,4cis)-4-(6,7-Dimethoxyquinoxalin-2-ylamino)-2-methyl-cyclohexanol;
(2cis,4trans)-4-(6,7-Dimethoxyquinoxalin-2-ylamino)-2-methyl-cyclohexanol;
4-(6,7-Dimethylquinoxalin-2-ylamino)cyclohexanol; and
(1S,2R,4S,SR)-5-(6,7-Dimethoxyquinoxalin-2-ylamino)-Bicyclo[3.2.1]-heptan-2-ol.

### More preferred compounds are the following:

trans-4-(6,7-Dimethoxyquinoxalin-2-ylamino)-cyclohexanol (herein after GN963);
cis-4-(6,7-Dimethoxyquinoxalin-2-ylamino)-cyclohexanol;
4-(6,7-Dimethoxyquinoxalin-2-ylamino)-2-methyl-cyclohexanol;
(-)-(2trans,4trans)-4-(6,7-Dimethoxyquinoxalin-2-ylamino)-2-methyl-cyclohexanol (herein after GN271);
(2exo,5exo)-5-(6,7-Dimethoxyquinoxalin-2-ylamino)-bicyclo[2.2.1]heptan-2-ol;
trans-4-(4-Chloro-6-methoxyquinoxalin-2-ylamino)-cyclohexanol; and
4-(6,7-Dimethoxyquinolin-3-ylamino)-cyclohexanol; and
5-(6,7-Dimethoxyquinoxalin-2-ylamino)-Bicyclo[2.2.1]-heptan-2ol (GN804).

It is to be understood that this disclosure covers all appropriate combinations of the particular and preferred groupings referred to herein.

The compounds of this disclosure may be prepared by employing procedures known in the literature starting from known compounds or readily prepared intermediates. Exemplary general procedures follow.

In addition, compounds of formula I are prepared according to the following Schemes I-X herein the variables are as described above, excepting those variables which one skilled in the art would appreciate would be incongruent with the method described.

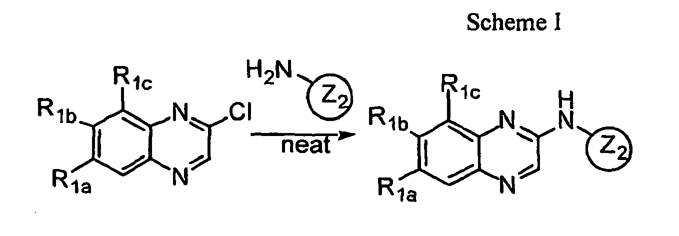

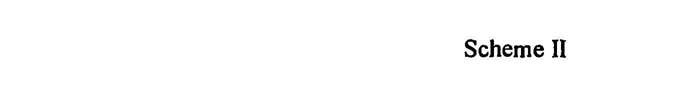

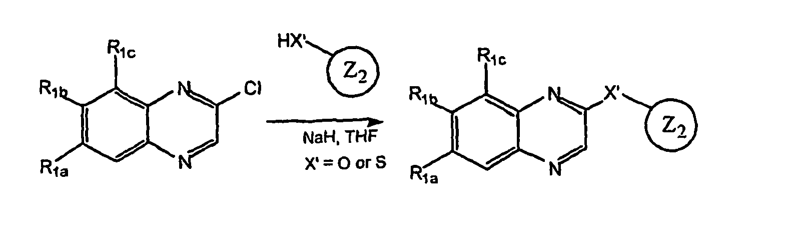

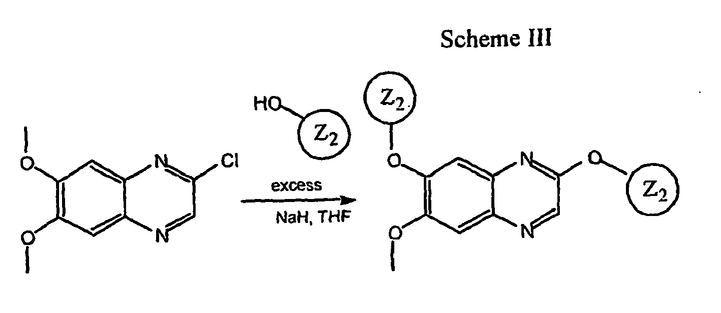

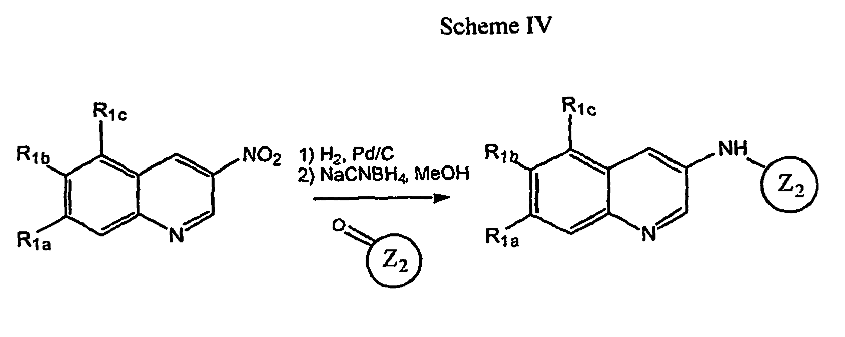

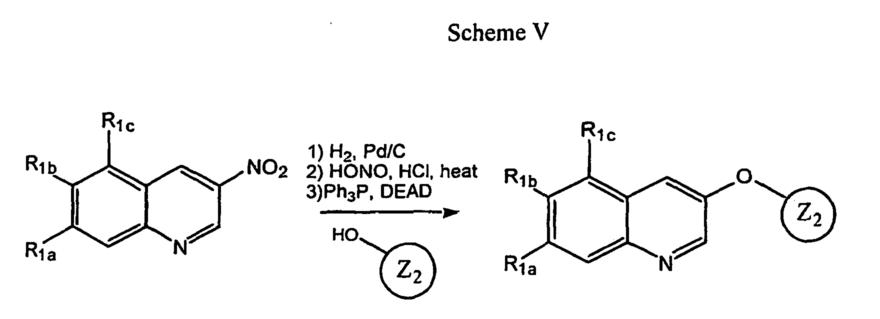

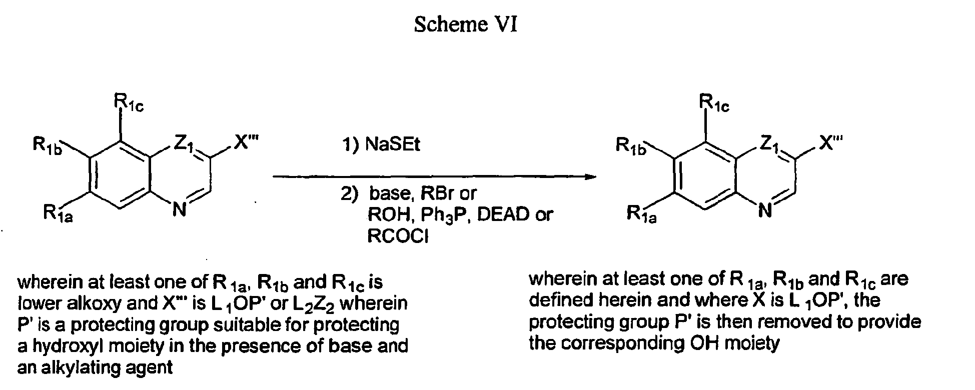

In Schemes VI, VII and VIII, R represents a precursor group to R₁ₐ, R_{1b} or R_{1c} as defined herein, such that reaction of RBr, ROH, or RCOCI with the aromatic hydroxy group under the conditions described in Schemes VI, VII and VIII results in formation of R₁ₐ, R_{1b} or R_{1c}.
Representative RBr include bromoacetic acid and methyl and ethyl bromoacetate.
Representative ROH include 2-ethoxyethanol, 2-(4-morpholinyl)ethanol and
3-(4-methylpiperazinyl)propanol.
A representative RCOCI is [1,4']-bipiperidin-1'-ylcarbonyl chloride.

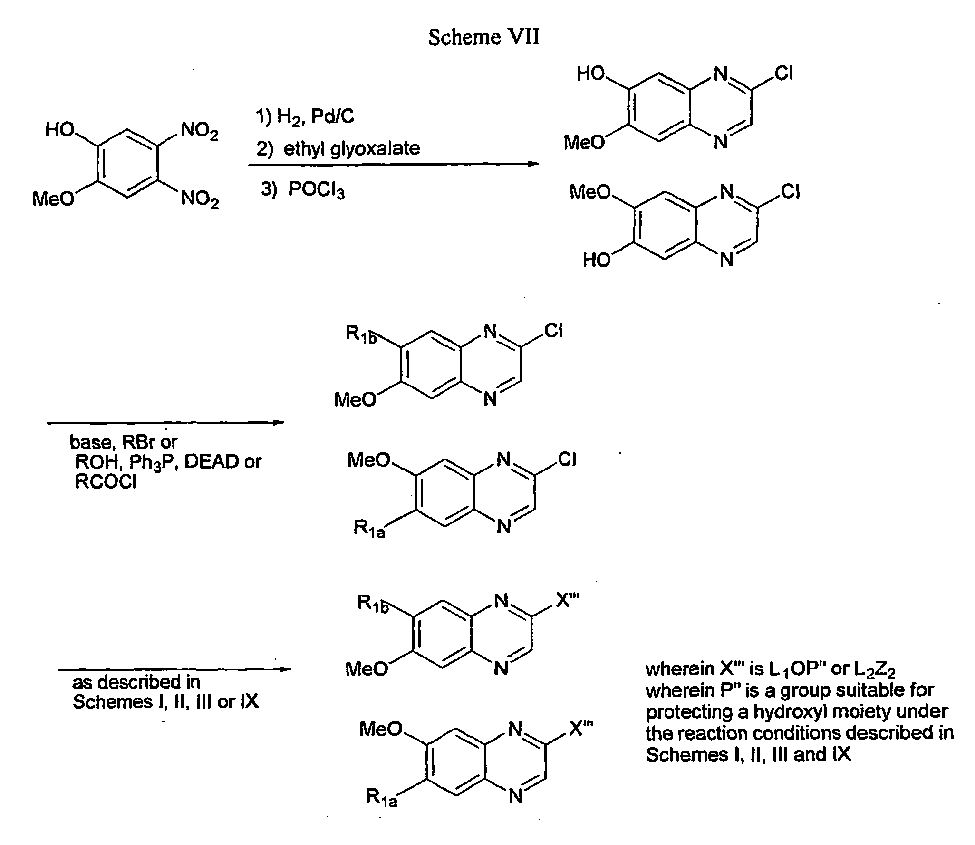

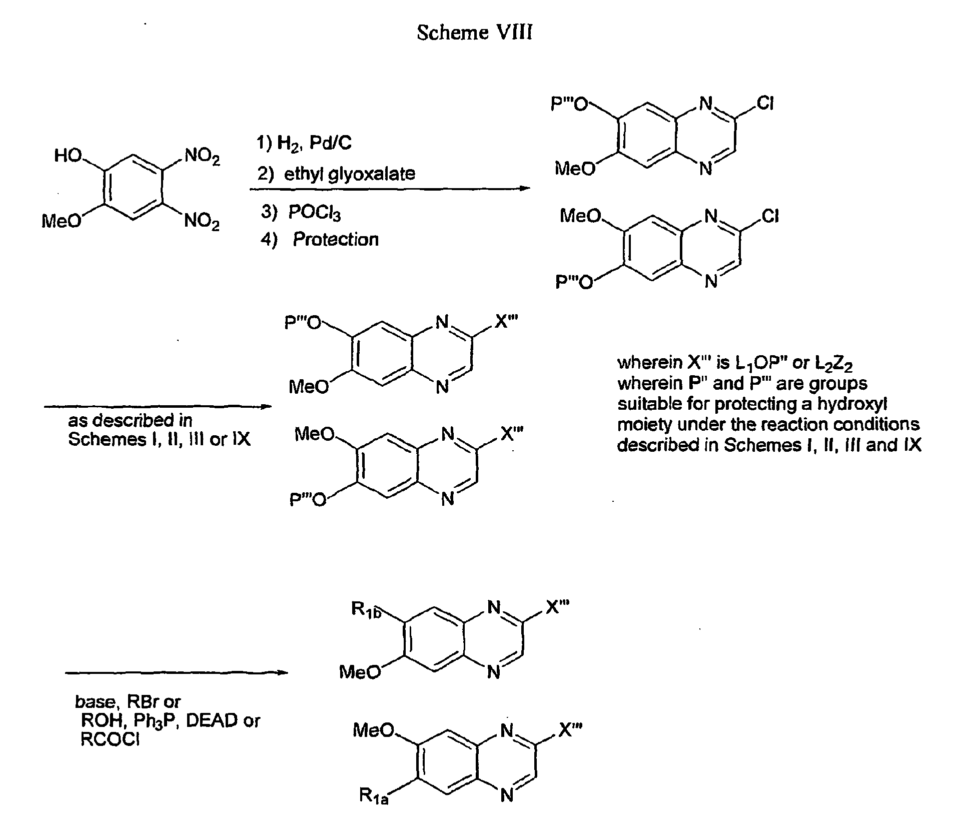

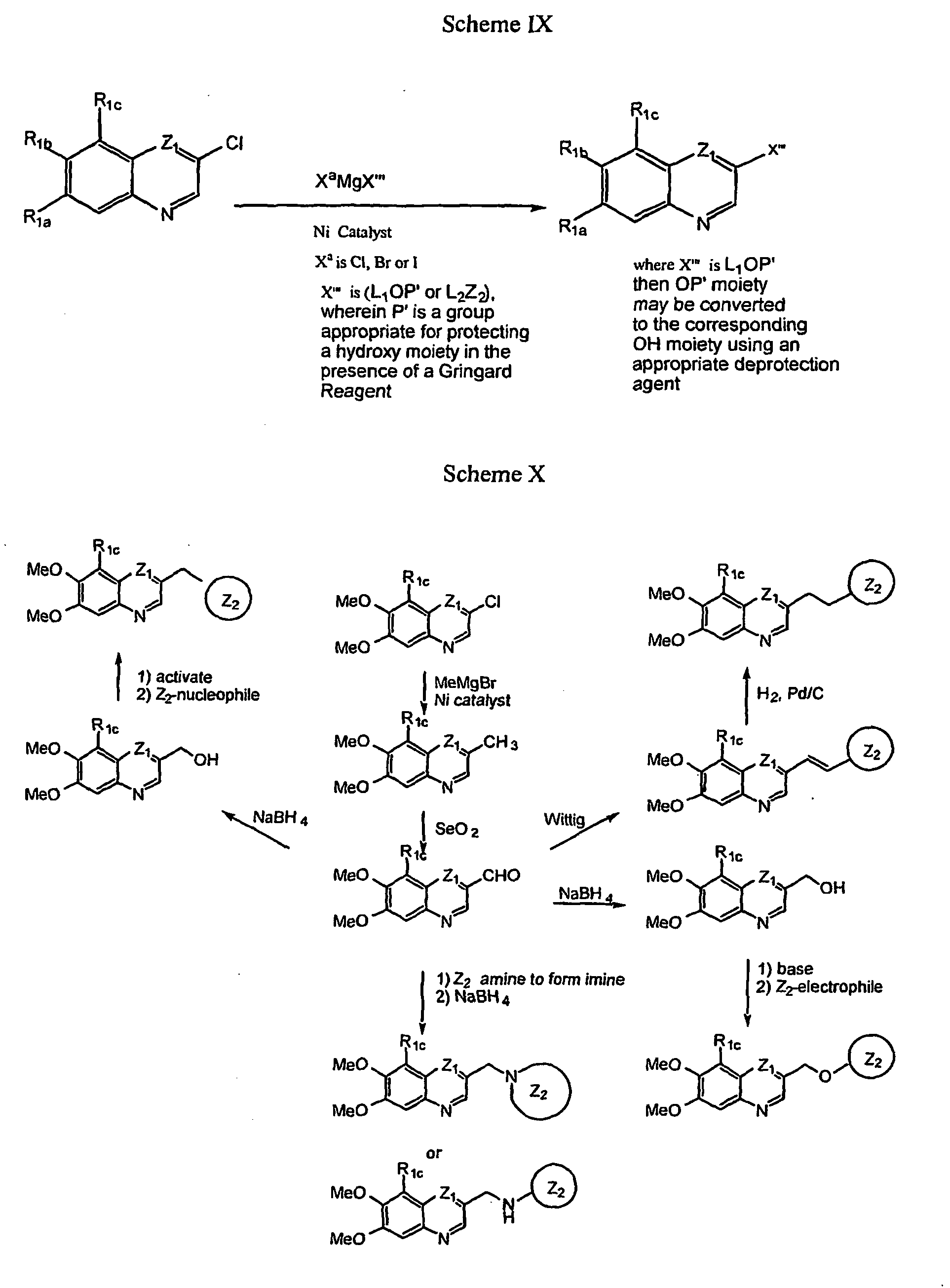

### I. General Procedures:

### 1. Coupling of 2-chloro substituted quinoxaline and amines or anilines

A mixture of 2-chloro-6,7-dimethoxyquinoxaline (1 eq.) and an amine (about 1 to about 5 eq.) is heated at about 160 to about 180 °C from about three hours to overnight. The dark-brown residue is dissolved in methanol/ methylene chloride (0%-10%) and chromatographed on silica gel eluted with hexane/ethyl acetate or methanol/methylene chloride (0%-100%) to yield the desired product. The desired product may be purified further through recrystallization in methanol, methylene chloride or methanol/water.

### 2. Coupling of 2-chloro substituted quinoxaline and alcohols or phenols

A suspension of an alcohol or mercaptan (1 eq.) and sodium hydride (about 1 to about 3 eq.) in anhydrous DMF/THF (0%-50%) is refluxed for 1 hour before addition of 2-chloro-6,7-dimethoxyquinoxaline (1 eq.). The resulting mixture is refluxed for about one to about four hours. The suspension is neutralized to about pH 5-8 and partitioned between methylene chloride and brine. The residue after concentration of methylene chloride is chromatographed on silica gel eluted with hexane/ethyl acetate or methanol/methylene chloride (0%-100%) to give the desired product.

### 3. Reductive amination reaction with amino-quinolines and aldehydes or ketones.

An appropriately substituted 3-amino quinoline (1 eq.) is stirred with 1 eq. of the appropriate aldehyde or ketone in methanol (or another suitable solvent mixture) until TLC indicates imine formation is complete. Excess NaCNBH₄ or NaBH₄, or another suitable reducing agent is added and the mixture is stirred until TLC shows consumption of the intermediate imine. The mixture is concentrated and the residue is chromatographed on silica gel with hexane/ethyl acetate (0-100 %) or chloroform/methanol (0-20%) to give the desired product.

### 4. coupling reaction of 3-amino substituted quinolines and bromophenyl compounds.

An appropriately substituted 3-amino quinoline (1 eq.) is stirred with ~1.4 eq. of a strong base such as sodium t-butoxide, 1 eq. of the appropriate bromophenyl compound, and catalytic amounts of 2,2'-bis(diphenylphosphino)-1-1'-binaphthyl (S-BINAP) and bis(dibenzylideneacetone)-Palladium (Pd(dba)₂) are mixed in an inert organic solvent such as toluene under an inert atmosphere such as argon and heated to about 80°C overnight. The mixture is cooled, diluted with a solvent such as ether, filtered, concentrated and chromatographed with 50% EtOAc/hexane to give the desired product.

### 5. Ether formation from 3-hydroxy substituted quinolines via Mitsunobu conditions.

A THF solution of an appropriately substituted hydroxyquinoxaline (at about 0 to about 25 °C) is treated with 1 eq. each of the desired alcohol, triphenylphosphine and finally diethylazodicarboxylate (DEAD) or a suitable equivalent. The reaction progress is monitored via TLC and upon completion of the reaction (about I to about 24 hours) the mixture is concentrated and the residue is chromatographed on silica gel to yield the desired product.

### 6. Dealkylation of a lower alkoxy substituted quinoline or quinoxaline, and subsequent alkylation.

An appropriate lower alkoxy substituted quinoline or quinoxaline (1 eq.) in DMF is treated with excess sodium ethanthiolate (usually about 2 or more eq.) and the reaction mixture is stirred with heating from about 1 to about 24 hours. The mixture is partitioned between water and ethyl acetate. Extractive workup followed by chromatography, if necessary, provides the corresponding desired hydroxy substituted quinoline or quinoxaline product.

The hydroxy substituted quinoline or quinoxaline product can be alkylated using the conditions for the Mitsunobu reaction as detailed above. Alternatively, simple alkylation using methods well-known in the art with a reactive alkyl- or benzyl- halide using NaH or another appropriate base in a suitable solvent provides the desired alkylated product.

### 7. Oxidation of nitrogen in a quinoline or quinoxaline to the corresponding N-oxide.

An imine (=N-) moiety in a quinoline or quinoxaline compound of formula (I), may be converted to the corresponding compound wherein the imine moiety is oxidized to an N-oxide, preferably by reacting with a peracid, for example peracetic acid in acetic acid or m-chloroperoxybenzoic acid in an inert solvent such as dichloromethane, at a temperature from about room temperature to reflux, preferably at elevated temperature.
The compounds of the present disclosure are useful in the form of the free base or acid or in the form of a pharmaceutically acceptable salt thereof
Where the compound of the present disclosure is substituted with a basic moiety, acid addition salts are formed and are simply a more convenient form for use; and in practice, use of the salt form inherently amounts to use of the free base form. The acids which can be used to prepare the acid addition salts include preferably those which produce, when combined with the free base, pharmaceutically acceptable salts, that is, salts whose anions are non-toxic to the patient in pharmaceutical doses of the salts, so that the beneficial inhibitory effects on PDGF inherent in the free base are not vitiated by side effects ascribable to the anions. Although pharmaceutically acceptable salts of said basic compounds are preferred, all acid addition salts are useful as sources of the free base form even if the particular salt, per se, is desired only as an intermediate product as, for example, when the salt is formed only for purposes of purification, and identification, or when it is used as intermediate in preparing a pharmaceutically acceptable salt by ion exchange procedures. Pharmaceutically acceptable salts within the scope of the invention are those derived from the following acids: mineral acids such as hydrochloric acid, sulfuric acid, phosphoric acid and sulfamic acid; and organic acids such as acetic acid, citric acid, lactic acid, tartaric acid, malonic acid, methanesufonic acid, ethanesulfonic acid, benzenesulfonic acid, p-toluenesulfonic acid, cyclohexylsulfamic acid, quinic acid, and the like. The corresponding acid addition salts comprise the following: hydrohalides, e.g. hydrochloride and hydrobromide, sulfate, phosphate, nitrate, sulfamate, acetate, citrate, lactate, tartarate, malonate, oxalate, salicylate, propionate, succinate, fumarate, maleate, methylene-bis-β-hydroxynaphthoates, gentisates, mesylates, isethionates and di-p-toluoyltartratesmethanesulfonate, ethanesulfonate, benzenesulfonate, p-toluenesulfonate, cyclohexylsulfamate and quinate, respectively.

According to a further feature of the disclosure acid addition salts of the compounds of this invention are prepared by reaction of the free base with the appropriate acid, by the application or adaptation of known methods. For example, the acid addition salts of the compounds of this invention are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

The compounds of this disclosure can be regenerated from the acid addition salts by the application or adaptation of known methods. For example, parent compounds of the invention can be regenerated from their acid addition salts by treatment with an alkali, e.g. aqueous sodium bicarbonate solution or aqueous ammonia solution.

Where the compound of the disclosure is substituted with an acidic moiety, base addition salts may be formed and are simply a more convenient form for use; and in practice, use of the salt form inherently amounts to use of the free acid form. The bases which can be used to prepare the base addition salts include preferably those which produce, when combined with the free acid, pharmaceutically acceptable salts, that is, salts whose cations are non-toxic to the animal organism in pharmaceutical doses of the salts, so that the beneficial inhibitory effects on PDGF inherent in the free acid are not vitiated by side effects ascribable to the cations. Pharmaceutically acceptable salts, including for example alkali and alkaline earth metal salts, within the scope of the invention are those derived from the following bases: sodium hydride, sodium hydroxide, potassium hydroxide, calcium hydroxide, aluminum hydroxide, lithium hydroxide, magnesium hydroxide, zinc hydroxide, ammonia, trimethylammonia, triethylammonia, ethylenediamine, n-methyl-glucamine, lysine, arginine, ornithine, choline, N,N'-dibenzylethylenediamine, chloroprocaine, diethanolamine, procaine, n-benzylphenethylamine, diethylamine, piperazine, tris(hydroxymethyl)-aminomethane, tetramethylammonium hydroxide, and the like.

Metal salts of compounds of the present disclosure may be obtained by contacting a hydride, hydroxide, carbonate or similar reactive compound of the chosen metal in an aqueous or organic solvent with the free acid form of the compound. The aqueous solvent employed may be water or it may be a mixture of water with an organic solvent, preferably an alcohol such as methanol or ethanol, a ketone such as acetone, aliphatic ether such as tetrahydrofuran, or an ester such as ethyl acetate. Such reactions are normally conducted at ambient temperature but they may, if desired, be conducted with heating.

Amine salts of compounds of the present disclosure may be obtained by contacting an amine in an aqueous or organic solvent with the free acid form of the compound. Suitable aqueous solvents include water and mixtures of water with alcohols such as methanol or ethanol, ethers such as tetrahydrofuran, nitriles such as acetonitrile, or ketones such as acetone. Amino acid salts may be similarly prepared.

The compounds of this disclosure can be regenerated from the base addition salts by the application or adaptation of known methods. For example, parent compounds of the disclosure can be regenerated from their base addition salts by treatment with an acid, eg., hydrochloric acid.

As well as being useful in themselves as active compounds, salts of compounds of the disclosure are useful for the purposes of purification of the compounds, for example by exploitation of the solubility differences between the salts and the parent compounds, side products and/or starting materials by technique well known to those skilled in the art.

Compounds of the present disclosure may contain asymmetric centers. These asymmetric centers may independently be in either the R or S configuration. It will also be apparent to those skilled in the art that certain compounds of formula I may exhibit geometrical isomerism. Geometrical isomers include the *cis* and *trans* forms of compounds of the invention, i.e., compounds having alkenyl moieties or substituents on the ring systems. In addiction, bicyclo ring systems include *endo* and *exo* isomers. The disclosure comprises the individual geometrical isomers, stereoisomers, enantiomers and mixtures thereof.

Such isomers can be separated from their mixtures, by the application or adaptation of known methods, for example chromatographic techniques and recrystallization techniques, or they are separately prepared from the appropriate isomers of their intermediates, for example by the application or adaptation of methods described herein.

The starting materials and intermediates are prepared by the application or adaptation of known methods, for example methods as described in the Reference.
The compounds of formula I as described herein inhibit inhibition of cell proliferation and/or cell matrix production and/or cell movement (chemotaxis) via inhibition of PDGF-R tyrosine kinase activity. A large number of disease states are caused by either uncontrolled reproduction of cells or overproduction of matrix or poorly regulated programmed cell death (apoptosis). These disease states involve a variety of cell types and include disorders such as leukemia, cancer, glioblastoma, psoriasis, inflammatory diseases, bone diseases, fibrotic diseases, atherosclerosis and occurring subsequent to angioplasty of the coronary, femoral or kidney arteries or, fibroproliferative disease such as in arthritis, fibrosis of the lung, kidney and liver. In particular, PDGF and PDGF-R have been reported to be implicated in specific types of cancers and tumors such as brain cancer, ovarian cancer, colon cancer, prostate cancer lung cancer, Kaposi's sarcoma and malignant melanoma. In addition, deregulated cellular proliferative conditions follow from coronary bypass surgery. The inhibition of tyrosine kinase activity is believed to have utility in the control of uncontrolled reproduction of cells or overproduction of matrix or poorly regulated programmed cell death (apoptosis).

The present invention provides for a method of screening for a combination of biological compounds capable of abrogating mature vasculature in a tumor. The present invention also provides for a method for screening for a combination of biological compounds capable of inhibiting the activation loop between the endothelial cell and smooth muscle cells within arterioles of perivasculature of a tumor.

The methods of screening according to the present invention comprise the step consisting of (i) introducing tumor cells to a collection of microvascular cells including mural cells and pericytes; (ii) regularly administering to the tumor cells a PDGF-receptor beta inhibitor as described above; (iii) administering to the tumor cells one or more anti-angiogenic chemotherapeutic; and (iv) measuring the one or more of tumor volume, mean vessel density, EC division, or EC apoptosis in the cells compared to a control, whereby a difference between the control and the cells administered the PDGF-receptor beta inhibitor and the one or more anti-cancer agents can be detected.
Most preferably, methods of screening of the invention comprises introducing tumor cells to a collection of microvascular cells including mural cells and pericytes; (ii) regularly administering to the tumor cells a PDGF-receptor beta inhibitor as described above; (iii) administering to the tumor cells one or more anti-angiogenic agent or chemotherapeutic agent, abrogen polypeptide, and/or kringle polypeptide; (iv) and measuring the one or more of tumor volume, mean vessel density, EC division, or EC apoptosis in the cells compared to a control, whereby a difference between the control and the cells administered the PDGF-receptor beta inhibitor and the one or more anti-cancer agents can be detected.

According to a most preferred aspect of the present invention, the chemotherapeutic agent used are docetaxel which is commercially available as an injectable solution as taxotere.

Until now, it has not been demonstrated that systemic treatment with trans-4-(6,7-dimethoxy-quinoxalin-2-ylamino)-cyclohexanol sulfate, i.e., GN963, or with GN804, or GN271 and a chemotherapeutic agent used as standard care for cancer treatment such as taxotere, docetaxel, or gemcitabine can induce a synergistic abrogation of the mature vasculature in tumors, especially tumors known to be resistant to chemotherapy. Examples of tumors at can be efficiently abrogated with the pharmaceutical combination of the present disclosure are those of prostate, ovarian, and pancreatic cancers.

Docetaxel, (2R, 3S)- N-carboxy-3-phenylisoserine, N-tert-butyl ester, 13-ester with 5(3-20-epoxy-1, 2a, 4, 7, 10, 130C-hexahydroxytax-H-en-9-one 4-acetate 2benzoate, trihydrate, indicated for the treatment of breast cancer. Docetaxel is a semisynthetic derivative of paclitaxel q. v., prepared using a natural precursor, 10-deacetyl-baccatin III, extracted from the needle of the European Yew tree. Uses and process of preparation of docetaxel are described in US 4,814,470; US 5,438,072; US 5,698,582; US 6,714,512.

Gemcitabine, 2'-deoxy-2', 2'-difluorocytidine monohydrochloride (3-isomer), is commercially available as GEMZAR. Gemcitabine which is a cytidine analog, exhibits cell phase specificity at S phase and by blocking progression of cells through the G1/S boundary. Method of use and preparation are described inter alia in US 4,808,614 and US 5,464,826. Gemcitabine has been used in combination with cisplatin in the treatment of locally advanced non-small cell lung cancer and alone in the treatment of locally advanced pancreatic cancer. However, use of Gemcitabine in combination with trans-4-(6,7-dimethoxy-quinoxalin-2-ylamino)-cyclohexanol sulfate (GN963) or with GN804 or GN271 was never demonstrated as capable synergistically abrogation of the mature vasculature in tumors known to be resistant to chemotherapy, including prostate, ovarian, and pancreatic cancers.

According to the present invention, other anti-angiogenic agents or chemotherapeutic agents may be screened for use in a synergistic combination with the above described PDGF-R inhibitor. Useful anti-angiogenic chemotherapeutic include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and antifolate compounds; topoisomerase I inhibitors such as camptothecins; hormones and hormonal analogues; signal transduction pathway inhibitors; non-receptor tyrosine kinase angiogenesis inhibitors; immunotherapeutic agents; proapoptotic agents; and cell cycle signaling inhibitors. Anti-microtubule or anti-mitotic agents are phase specific agents active against the microtubules of tumor cells during M or the mitosis phase of the cell cycle.

Examples of anti-microtubule agents include, but are not limited to, diterpenoids and vinca alkaloid.

Diterpenoids, which are derived from natural sources, are phase specific anticancer agents that operate at the G2/M phases of the cell cycle. It is believed that the diterpenoids stabilize the -tubulin subunit of the microtubules, by binding with this protein. Disassembly of the protein appears then to be inhibited with mitosis being arrested and cell death following. Examples of diterpenoids include, but are not limited to paclitaxel.

Paclitaxel, 5, 20-epoxy-1, 20, 4,7 (3, 10a, 13a-hexa-hydroxytax-11-en-9-one 4,10diacetate 2-benzoate 13-ester with (2R, 3S)-N-benzoyl-3-phenylisoserine ; is a natural diterpene product isolated from the Pacific yew tree *Taxus brevifolia* and is commercially available as an injectable solution TAXOL. It is a member of the taxane family of terpenes. It was first isolated in 1971 by Wani et al. J. Am. Chem, Soc., 93: 2325.1971), who characterized its structure by chemical and X-ray crystallographic methods. One mechanism for its activity relates to paclitaxel's capacity to bind tubulin, thereby inhibiting cancer cell growth. Schiff et al., Proc. Natl, Acad, Sci. USA, 77: 1561-1565 (1980); Schiff et al., Nature, 277: 665-667 (1979); Kumar, J. Biol, Chem, 256: 10435-10441 (1981). For a review of synthesis and anticancer activity of some paclitaxel derivatives see: D. G. I. Kingston et al., Studies in Organic Chemistry vol. 26, entitled "New trends in Natural Products Chemistry 1986", Attaur-Rahman, P. W. Le Quesne, Eds.(Elsevier, Amsterdam, 1986) pp 219-235.

Paclitaxel has been approved for clinical use in the treatment of refractory ovarian cancer in the United States (Markman et al., Yale Journal of Biology and Medicine, 64: 583,1991; McGuire et al., Ann. Intem, Med., 111: 273, 1989) and for the treatment of breast cancer (Holmes et al., J. Nat. Cancer Inst., 83 1797, 1991.) It is a potential candidate for treatment of neoplasms in the skin (Einzig et. al., Proc. Am. Soc. Clin. Oncol., 20: 46) and head and neck carcinomas (Forastire et. al., Sem. Oncol., 20: 56, 1990). The compound also shows potential for the treatment of polycystic kidney disease (Woo et. al., Nature, 368:750, 1994), lung cancer and malaria. Treatment of patients with paclitaxel results in bone marrow suppression (multiple cell lineages, Ignoff, R. J. et. al, Cancer Chemotherapy Pocket Guide, 1998) related to the duration of dosing above a threshold concentration (50nM) (Kearns, C. M. et. al., Seminars in Oncology, 3 (6) p. 16-23, 1995).

Vinca alkaloid are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as Vebano as an injectable solution. It has possible indications for a second line therapy of various solid tumors.

Vincristine, vincaleukoblastine, 22-oxo-, sulfate, is commercially available as Oncovino as an injectable solution. Vincristine is indicated for the treatment of acute leukemias and has also found use in treatment regimens for Hodgkin's and non Hodgkin's malignant lymphomas.

Vinorelbine, 3', 4'-didehydro-4'-deoxy-C'-norvincaleukoblastine [R-(R*, R*)-2, 3dihydroxybutanedioate (1:2) (salt)], commercially available as an injectable solution of vinorelbine tartrate (Navelbineo), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

Platinum coordination complexes are non-phase specific anti-cancer agents, which are interactive with DNA. The platinum complexes enter tumor cells, undergo, aquation and form intra-and interstrand crosslinks with DNA causing adverse biological effects to the tumor. Examples of platinum coordination complexes include, but are not limited to, cisplatin and carboplatin.

Cisplatin, cis-diamminedichloroplatinum, is commercially available as Platinol; as an injectable solution. Cisplatin is primarily indicated in the treatment of metastatic testicular and ovarian cancer and advanced bladder cancer. Carboplatin, platinum, diammine [1,1-cyclobutane-dicarboxylate (2-)-O, O'], is commercially available as Paraplatino as an injectable solution. Carboplatin is primarily indicated in the first and second line treatment of advanced ovarian carcinoma.

Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfhydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl suffonates such as busulfan; nitrosoureas such as carmustine; and triazenes such as dacarbazine.

Cyclophosphamide, 2-[bis (2-chloroethyl) amino] tetrahydro-2H-1, 3, 2oxazaphosphorine 2-oxide monohydrate, is commercially available as an injectable solution or tablets as Cytoxan. Cyclophosphamide is indicated as a single agent or in combination with other chemotherapeutic agents, in the treatment of malignant lymphomas, multiple myeloma, and leukemias.

Melphalan, 4- [bis (2-chloroethyl) amino]-L-phenylalanine, is commercially available as an injectable solution or tablets as Alkerano. Melphalan is indicated for the palliative treatment of multiple myeloma and non-resectable epithelial carcinoma of the ovary.

Chlorambucil, 4-[bis (2-chloroethyl) amino] benzenebutanoic acid, is commercially available as Leukeran tablets. Chlorambucil is indicated for the palliative treatment of chronic lymphatic leukemia, and malignant lymphomas such as lymphosarcoma, giant follicular lymphom, and Hodgkin's disease. Bone marrow suppression is the most common dose limiting side effect of chlorambucil.

Busulfan, 1, 4-butanediol dimethanesulfonate, is commercially available as Myleran. Busulfan is indicated for the palliative treatment of chronic myelogenous leukemia. Bone marrow suppression is the most common dose limiting side effects of busulfan.

Carmustine, 1, 3- [bis (2-chloroethyl)-1-nitrosourea, is commercially available as single vials of lyophilized material as BiCNU;. Carmustine is indicated for the palliative treatment as a single agent or in combination with other agents for brain tumors, multiple myeloma, Hodgkin's disease, and non-Hodgkin's lymphomas. Delayed myelosuppression is the most common dose limiting side effects of carmustine.

Dacarbazine, 5-(3, 3-dimethyl-1-triazeno)-imidazole < 4-carboxamide, is commercially available as single vials of material as DTIC. Dacarbazine is indicated for the treatment of metastatic malignant melanoma and in combination with other agents for the second line treatment of Hodgkin's Disease.

Antibiotic anti-neoplastics are non-phase specific agents, which bind or intercalate with DNA. Typically, such action results in stable DNA complexes or strand breakage, which disrupts ordinary function of the nucleic acids leading to cell death.

Examples of antibiotic anti-neoplastic agents include, but are not limited to, actinomycins such as dactinomycin, anthrocyclins such as daunorubicin and doxorubicin; and bleomycins.

Dactinomycin, also know as Actinomycin D, is commercially available in injectable form as Cosmegeno. Dactinomycin is indicated for the treatment of Wilm's tumor and rhabdomyosarcoma..

Daunorubicin, (8S-cis-)-8-acetyl-10- [(3-amino-2, 3,6-trideoxy-a-L-lyxohexopyranosyl) oxy]-7, 8,9,10-tetra hyd ro-6,8,11-trihyd roxy-l-methoxy-5,12 naphthacenedione hydrochloride, is commercially available as a liposomal injectable form or as an injectable form. Daunorubicin is indicated for remission induction in the treatment of acute nonlymphocytic leukemia and advanced HIV associated Kaposi's sarcoma.

Doxorubicin, (8S, 10S)-10- [(3-amino-2, 3,6-trideoxy-a-L-lyxohexopyranosyl) oxy]-8-glycoloyl, 7,8,9,10-tetrahydro-6,8,11-trihydroxy-1-methoxy-5,12 naphthacenedione hydrochloride; is commercially available as an injectable form as Rumex; or Adriamycin. Doxorubicin is primarily indicated for the treatment of acute lymphoblastic leukemia and acute myeloblastic leukemia, but is also a useful component in the treatment of some solid tumors and lymphomas.

Bleomycin, a mixture of cytotoxic glycopeptide antibiotics isolated from a strain of Streptomyces verticillus, is commercially available as Blenoxane. Bleomycin is indicated as a palliative treatment, as a single agent or in combination with other agents, of squamous cell carcinoma, lymphomas, and testicular carcinomas.

Topoisomerase II inhibitors include, but are not limited to, epipodophyllotoxins. Epipodophyllotoxins are phase specific anti-neoplastic agents derived from the mandrake plant. Epipodophyllotoxins typically affect cells in the S and G2 phases of the cell cycle by forming a ternary complex with topoisomerase 11 and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9 [4,6-0- (R)-ethylidene-p-D glucopyranoside], is commercially available as an injectable solution or capsules and is commonly known as VP-16. Etoposide is indicated as a single agent or in combination with other chemotherapy agents in the treatment of testicular and nonsmall cell lung cancers.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to 5-fluorouracil, methotrexate, cytarabine, mecaptopurine, and thioguanine.

5-fluorouracil, 5-fluoro-2, 4- (1H, 3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Other fluoropyrimidine analogs include 5-fluoro deoxyuridine (floxuridine) and 5-fluorodeoxyuridine monophosphate.

Cytarabine, 4-amino-1-p-D-arabinofuranosyl-2 (H)-pyrimidinone, is commercially available as Cytosar-U, and is commonly known as Ara-C. It is believed that cytarabine exhibits cell phase specificity at S-phase by inhibiting DNA chain elongation by terminal incorporation of cytarabine into the growing DNA chain.

Cytarabine is indicated as a single agent or in combination with other chemotherapy agents in the treatment of acute leukemia. Another cytidine analog includes 5-azacytidine. Cytarabine induces leucopenia, thrombocytopenia, and mucositis.

Mercaptopurine, 1,7-dihydro-6H-purine-6-thione monohydrate, is commercially available as Purinetholo. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. A useful mercaptopurine analog is azathioprine.

Thioguanine, 2-amino-1, 7-dihydro-6H-purine-6-thione, is commercially available as Tabloid. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Other purine analogs include pentostatin, erythrohydroxynonyladenine, fludarabine phosphate, and cladribine.

Methotrexate, N- [4 [[ (2, 4-diamino-6-pteridinyl) methyl] methylamino] benzoyl]-L-glutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphom, and carcinomas of the breast, head, neck, ovary and bladder.

Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7- (4-methylpiperazino-methylene)-10, 11-ethylenedioxy-20- camptothecin described below.

Irinotecan HCI, (4S)-4,11-diethyl-4-hydroxy-9- [ (4-piperidinopiperidino) carbonyloxy]-l H-pyrano [3', 4', 6,7] indolizino [1, 2-b] quinoline-3, 14 (4H, 12H)-dione hydrochloride, is commercially available as the injectable solution Camptosaro.

Irinotecan is a derivative of camptothecin which binds, along with its active metabolite SN-38, to the topoisomerase I-DNA complex. It is believed that cytotoxicity occurs as a result of irreparable double strand breaks caused by interaction of the topoisomerase I : DNA: irintecan or SN-38 ternary complex with replication enzymes irinotecan is indicated for treatment of metastatic cancer of the colon or rectum.

Topotecan HCI, (S)-10- [(dimethylamino) methyl]-4-ethyl-4, 9-dihydroxy-l H-pyrano [3', 4', 6,7] indolizino [1, 2-b] quinoline-3, 14- (4H, 12H)-dione monohydrochloride, is commercially available as the injectable solution Hycamtino. Topotecan is a derivative of camptothecin which binds to the topoisomerase I-DNA complex and prevents religation of singles strand breaks caused by Topoisomerase I in response to torsional strain of the DNA molecule. Topotecan is indicated for second line treatment of metastatic carcinoma of the ovary and small cell lung cancer.

A variety of different anti-angiogenic polypeptides, agents, and/or chemotherapeutic agents or anti-cancer polypeptides can also be selected. Information sources such as www.clinicaltrials.gov, www.ncbi.nlm.nih, and www.drugs.com, include references to polypeptides and agents that can be selected.

As recited above, a method of treating cancer is provided which includes administering therapeutically effective amounts of a compound of formula I, II, III, IV, or V or physiologically functional derivatives thereof and at least one anti-angiogenic or chemotherapeutic agent.

Non-limiting examples

### EXAMPLE 1 3-Cyclohexyloxy-6,7-dimethoxyquinoline

To a THF solution (30 mL) at 0°C is added 3-hydroxy-6,7-dimethoxyquinoline (0.237 g, 1.15 mmole), cyclohexanol (0.347 g, 3.46 mmole), Ph₃P (0.908 g, 3.46 mmole). Diethylazodicarboxylate is added portionwise until the solution retained a deep red color (0.663 g, 3.81 mmole). After 4 hours the solution is concentrated and the residue chromatographed (50% EtOAc in hexanes). The product is recrystallized from isopropanol/hexanes as the HCl salt as a white solid (m.p. 229-232°C, dec.).

### EXAMPLE 2 2-Anilino-6-isopropoxy-quinoxaline hydrochloride

To NaH (0.033 g, 0.84 mmol) under argon is added 1 mL DMF. 2-Anilino-6-quinoxalinol (0.1 g, 0.42 mmol) in 1.5 mL DMF is added portionwise. After 30 minutes, 2-bromopropane is added dropwise and the solution is heated to 50°C for 1.5 hours. The cooled reaction mixture is quenched with water and partitioned between EtOAc and H₂O, washed with H₂O (3X), brine, dried (MgSO₄), and concentrated. The resulting residue is chromatographed (30% EtOAc/hexanes) to provide 0.05 g dialkylated product and 0.1 g of the title compound. An analytical sample of the HCl salt is obtained by addition of IPA (isopropanol)/HCl to an Et₂O/IPA solution of the free base to provide HCl salt (m.p. 205-210°C dec). Anal. Calcd. for C₁₇H₁₇N₃O •HCl: C, 64.65; H, 5.74; N, 13.31; Found: C, 64.51; H, 5.90; N, 13.09.

### EXAMPLE 3 2-Anilino-6-methoxy-quinoxaline hydrochloride

To 2-chloro-6-methoxy-quinoxaline (0.93 g, 4.8 mmol) under argon is added aniline (1.3 mL, 14.3 mmol). The reaction mixture is heated at 120°C for 2 hours, then at 150°C for 1.5 hours. The mixture is cooled and CH₂Cl₂ is added. The resulting suspension is stirred and the orange solid is filtered off, washed with CH₂Cl₂/Et₂O, then stirred vigorously in H₂O for 40 minutes, filtered, and washed with Et₂O to provide a bright-yellow solid.

### EXAMPLE 4 2-Anilino-6-quinoxalinol

By the method of Feutrill, G. I.; Mirrington, R. N. Tet. Lett. 1970, 1327; the aryl methyl ether is converted to the phenol derivative. To 2-anilino-6-methoxy-quinoxaline (0.27 g, 1.07 mmol) under argon in DMF is added the sodium salt of ethanethiol (0.19 g, 2 mmol). The reaction mixture is heated to 110°C overnight. The mixture is concentrated and partitioned between EtOAc and H₂O/5% tartaric acid such that the pH of the aqueous layer is approximately 4. The organic layer is washed with H₂O (4X), then with 2.5% NaOH (4X). The basic layers combined, washed with EtOAc (2X), re-acidified with 5% tartaric acid, and washed with multiple portions of EtOAc. The organic layers are combined, washed with brine, dried (Na₂SO₄) and concentrated. The resulting solid is chromatographed (50% EtOAc/ hexanes). An analytical sample is obtained by triturating the product with Et₂O to provide a yellow powder (m.p. 211-213°C). Anal. Calcd. for C₁₄H₁₁N₃O: C, 70.88; H, 4.67; N, 17.71; Found: C, 70.64; H, 4.85; N, 17.58.

### EXAMPLE 5 Phenyl-[6-(tetrahydrofuran-3-(R)-yl-oxy)quinoxalin-2-yl]amine

To a THF solution at 0°C under argon is added 2-anilino-6-quinoxalinol (0.23 g, 0.97 mmol), (S)-(+)-3-hydroxytetrahydrofuran (0.086 mL, 1.3 mmol), and triphenylphosphine (0.31 g, 1.2 mmol). DEAD (0.18 mL, 1.2 mmol) is added portionwise. The reaction is allowed to warm to room temperature and stirred for 1.5 hours. The mixture is concentrated and partitioned between EtOAc and H₂O. The organic layer is washed with H₂O, brine, dried (MgSO₄), and concentrated. The resulting yellow oil is chromatographed (50% EtOAc/hexanes) and taken up in Et₂O/IPA. HCl/ Et₂O solution is added dropwise and the resulting red-orange powder is dried in vacuo. The powder is free-based by stirring in MeOH with washed (3X H₂O, 5X MeOH) basic ion exchange resin. The mixture is stirred 30 minutes, filtered, concentrated, and recrystallized from EtOAc/hexanes to provide, in two crops, the product (m.p. 173-175°C). Anal. Calcd. for C₁₈H₁₇N₃O₂: C, 70.35; H, 5.57; N, 13.67; Found: C, 70.19; H, 5.60; N, 13.66.

### EXAMPLE 6 2,7-Bis-cyclohexyloxy-6-methoxy-quinoxaline

To a DMF solution (5 mL) of NaH (0.32 g, 8 mmol) under argon, cyclohexanol (0.7 mL, 6.7 mmol) is added dropwise. The mixture is stirred at room temperature for 25 minutes, then 2-chloro-6,7-dimethoxyquinoxaline is added portionwise. The reaction is stirred for 15 minutes at room temperature, at 90°C for 2 hours, and at 110°C for 1 hour. The mixture is cooled, quenched with H₂O, and partitioned between EtOAc/ H₂O. The organic layer is washed with H₂O and brine, dried (MgSO₄), and chromatographed (10% EtOAc/hexanes) to provide a waxy white solid (m.p. 75-78°C). Anal. Calcd. for C₂₁H₂₈N₂O₃: C, 70.76; H, 7.92; N, 7.86; Found: C, 70.81; H, 7.79; N, 7.70.

### EXAMPLE 7 Cyclohexyl-(6,7-dimethoxyquinoxalin-2-ylmethyl)-amine

To a 0.067 M solution of 6,7-dimethoxy-2-quinoxaline carboxaldehyde in 2:1 MeOH/1,2-dichloroethane (7.5 mL, 0.5 mmol) is added cyclohexylamine (0.11 mL, 0.9 mmol). The reaction is allowed to stir at room temperature overnight, then NaBH₄ (0.038 g, 1 mmol) is added and the reaction mixture is stirred overnight. The mixture is then concentrated and chromatographed (50% EtOAc/hexanes-approximately 5% MeOH in 50% EtOAc/hexanes). The oil is dissolved in EtOAc/ hexanes and treated with HCl in EtOH. The resulting solution is concentrated and the solids are triturated with isopropanol to provide a white solid after drying in vacuo at 60 °C (m.p. 185-190°C, dec.). Anal. Calcd. for C₁₇H₂₃N₃O₂ •HCl: C, 60.44; H, 7.16; N, 12.44; Found: C, 60.48; H, 6.88; N, 12.07.

### EXAMPLE 8 (6,7-Dimethoxyquinolin-3-yl)-trans-(3-(R)-methyl-cyclohexyl)-amine and (6,7-Dimethoxyquinolin-3-yl)-cis-(3-(R)-methyl-cyclohexyl)-amine

The reaction is performed similarly to the above preparation using the free base of 3-amino-6,7-dimethoxyquinoline (0.32 g, 1.6 mmol) and (R)-(+)-3-methylcyclohexanone (0.23 mL, 1.9 mmol). The product mixture obtained is chromatographed (70% EtOAc/hexanes), and recrystallized from EtOAc/hexanes to obtain a white solid (1:1 mixture of *cis* and *trans* isomers) (m.p. 153-160°C). Anal. Calcd. for C₁₈H₂₄N₂O₂: C, 71.97; H, 8.05; N, 9.33; Found: C, 72.12; H, 7.85; N, 9.29.

### EXAMPLE 9 3-(6,7-Dimethoxyquinolin-3-yl-amino)-2,2-dimethyl-propan-1-ol

The reaction is run similar to the preparation in Example 7. To a MeOH solution of 4Å powdered molecular sieves (0.35 g) under argon is added 3-amino-6,7-dimethoxyquinoline (0.32 g, 1.6 mmol) and 2,2-dimethyl-3-hydroxypropionaidehyde (0.19 g, 1.9 mmol). The product mixture is chromatographed (3% MeOH/CHCl₃) to afford 0.10 g of material which is partitioned between CH₂Cl₂/10% NaOH. The organic layer is washed with 10% NaOH, H₂O, and brine, then dried (MgSO₄), and recrystallized from EtOAc/hexanes to provide a light-orange solid (m.p. 170-173.5°C). Anal. Calcd. for C₁₆H₂₂N₂O₃: C, 66.18; H, 7.64; N, 9.65; Found: C, 66.11; H, 7.49; N, 9.33.

### EXAMPLE 10 Cyclohexyl-(6-methoxy-7-morpholin-4-yl-quinoxalin-2-yl)-amine

This preparation is based on an adaptation of the method described by Buchwald, et al, J. Am. Chem. Soc., 1996, 118, 7215. To a toluene solution of 2-cyclohexylamino-6-methoxy-7-bromo-quinoxaline (0.1 g, 0.3 mmol) under argon is added morpholine (0.1 g, 0.3 mmol), sodium *tert*-butoxide (0.04 g, 0.42 mmol), S-(-)-BINAP (cat., 0.001 g), and bis(dibenzylideneacetone)-palladium (cat., 0.001 g). The reaction mixture is heated to 80°C overnight. The mixture is cooled, diluted with Et₂O, filtered, concentrated, and chromatographed (50% EtOAc/hexanes). The product is recrystallized from EtOAc/hexanes to provide, in two crops, to provide a yellow solid (m.p. 194-196°C). Anal. Calcd. for C₁₉H₂₆N₄O₂: C, 66.64; H, 7.65; N, 16.36; Found: C, 66.60; H, 7.60; N, 16.51.

### EXAMPLE 11 trans -4-(7-Chloro-6-methoxy-quinoxalin-2-amino)-cyclohexanol and trans -4-(6-Chloro-7-methoxy-quinoxalin-2-yl-amino)-cyclohexanol

To a reaction flask under argon fitted with a Dean-Stark trap and a condenser is added 6:1 2,7-dichloro-6-methoxy-quinoxaline : 2,6-dichloro-7-methoxy-quinoxaline (0.30 g, 1.3 mmol) and *trans*-4-amino-cyclohexanol (0.35 g, 3 mmol). The reaction mixture is heated to 170°C for approximately 10 hours, then concentrated and chromatographed twice, (7% MeOH/CHCl₃, then 5% MeOH/CHCl₃). The product is recrystallized from EtOAc/hexanes to provide a light-yellow solid (m.p. 144-147°C). Anal. Calcd. for C₁₉H₂₆N₄O₂ •0.4 H₂O: C, 57.20; H, 6.02; N, 13.34; Found: C, 57.21; H, 5.97; N, 13.08.1H NMR analysis revealed that the product is a 2:1 mixture of *trans* -4 -(7-chloro-6-methoxy-quinoxalin-2-amino)-cyclohexanol : *trans* -4 -(6-chloro-7-methoxy-quinoxalin-2-yl-amino)-cyclohexanol.

### EXAMPLE 12 trans-4-(6,7-Dimethoxyquinoxalin-2-ylamino)-cyclohexanol (GN963)

*trans*- 4-aminocyclohexanol (0.11 g, 2 eq.) and 2-chloro-6,7-dimethoxyquinoxaline (0.1 g, 1 eq.) are combined and heated to 160-180°C for a period of 4-8 hours. The dark-brown suspension is filtered and concentrated. The residue is purified on a flash column eluted with 3% methanol/methylene chloride to provide the product as a yellow powder with m.p. of 119-123°C. Anal. Calcd. for C₁₆H₂₁N₃O₃: C, 62.33; H, 7.05; N, 13.63; Found: C, 62.35; H, 7.09; N, 13.18.

The compound could be recrystallized by the following method. Starting with 0.2 g of yellow powder in a mixture of 2.5 mL of water and 1.25 mL of methanol a clear orange-colored solution is obtained upon reflux. The hot solution is left standing and cooled gradually. Orange-colored needle-like crystals are collected by filtration and dried under high vacuum to give a yellow solid (m.p. 119-120 °C).

Alternatively, the HCl salt of the title compound is prepared as follows: To a solution of *trans*-4-(6,7-dimethoxyquinoxalin-2-ylamino)-cyclohexanol in isopropanol is added a solution of HCl at 0°C. The mixture is stirred for 15 minutes before filtration. The solid collected is dried under a high vacuum to provide the *trans*-4-(6,7-dimethoxyquinoxalin-2-ylamino)-cyclohexanol hydrochloric acid salt. Anal. Calcd. for C₁₆H₂₂ClN₃O₃ •1.2 H₂O: C, 53.19; H, 6.80; N, 11.63; Cl, 9.81; Found: C, 53.14; H, 6.85; N, 11.24; Cl, 10.28.

Alternatively, the sulfate salt of the title compound is prepared as follows: In a typical procedure, *trans*-4-(6,7-dimethoxyquinoxalin-2-ylamino)-cyclohexanol is dissolved in acetone or another suitable organic solvent with warming up to 45 °C as necessary. To the resultant solution is carefully added aqueous H₂SO₄ (1 equiv., 1 M soln) with rapid stirring. The salt thus formed is collected and dried to provide the sulfate in >80% yield.

The following compounds are prepared similarly beginning with the appropriate starting material.

3-(6,7-Dimethoxyquinoxalin-2-ylamino)-propan-1-ol (m.p. 154.5-156°C). Anal. Calcd. for C₁₃H₁₇N₃O₃: C, 59.30; H, 6.51; N, 15.96; Found: C, 59.30; H, 6.46; N, 15.87.

3-(6,7-Dimethoxyquinoxalin-2-ylamino)-2,2-dimethyl-propan-1-ol (m.p. 174-176.5°C). Anal. Calcd. for C₁₅H₂₁N₃O₃: C, 61.84; H, 7.27; N, 14.42; Found: C, 61.67; H, 7.22; N, 14.22.

4-(6,7-Dimethylquinoxalin-2-ylamino)-cyclohexanol (m.p. 168-171°C). Anal. Calcd. for C₁₆H₂₁N₃O: C, 70.82; H, 7.80; N, 15.48; Found: C, 70.76; H, 7.90; N, 15.20.

### EXAMPLE 13 cis-4-(6,7-Dimethoxyquinoxalin-2-ytamino)-cyclohexanol

A mixture of *cis*-4-aminocyclohexanol (400 mg, 3.48 mmole) and 2-chloro-6,7-dimethoxyquinoxaline (450 mg, 2 mmole) in 5 mL of ethanol is placed in sealed tube and then heated at 180°C for 3 hours. The dark-brown mixture is chromatographed on silica gel and eluted with ethyl acetate to provide the desired product (m.p. 65-67°C). Anal. Calcd. for C₁₆H₂₁N₃O₃ •0.6 H₂O: C, 61.17; H, 7.12; N, 13.37; Found: C, 61.22; H, 7.19; N, 12.19.

### EXAMPLE 14 (±)-Bicyclo [2.2.1] hept-2-yl-(6,7-dimethoxyquinoxalin-2-yl)-amine

Procedure A: A mixture of 2-chloro-6,7-dimethoxyquinoxaline (5 g, 22.3 mmole) and (±)-*exo*-norbomyl-2-amine (10 g, 90 mmole) is heated at 160-180°C overnight. The dark-brown residue is dissolved in 200 mL of methylene chloride and washed with 1N NaOH (50 mL). The organic layer is dried over magnesium sulfate and then filtered. The residue after concentration is chromatographed on silica gel eluted with hexane/ethyl acetate (80%) to provide the desired product as a yellow solid which can be recrystalized in methanol.

Procedure B: A mixture of 2-chloro-6,7-dimethoxyquinoxaline (9 g, 40.1 mmole) and (±)-ejeo-norbomyl-2-amine (5.77 g, 52 mmole), Sodium t-butoxide (4.22 g, 44 mmole), 2,2'-bis(diphenylphosphino)-1-1'-binaphthyl (BINAP, 120 mg) and bis(dibenzylideneacetone)-palladium Pd(dba)₂, 40 mg in 80 mL of toluene is heated at 80°C for eight hours. Another portion of BINAP (60 mg) and Pd(dba)₂ (20 mg) is added and the mixture is heated at 100°C overnight. After being diluted with 200 mL of methylene chloride, the reaction mixture is washed with 1N NaOH (100 mL). The organic layer is dried over magnesium sulfate and filtered. The residue after concentration is chromatographed on silica gel eluted with hexane/ethyl acetate (80%) to provide the desired product as a light-yellow solid (m.p. 188-189°C). Anal. Calcd. for C₁₇H₂₁N₃O₃: C, 68.20; H, 7.07; N, 14.04; Found: C, 68.18; H, 7.03; N, 14.03.

The following compounds are prepared similarly beginning with the appropriate starting material (procedure A).
*exo*-bicyclo[2.2.1]hept-5-en-2-yl-(6,7-dimethoxyquinoxalin-2-yl)-amine (m.p. 175-177°C).
Anal. Calcd. for C₁₇H₁₉N₃O₂ •0.4 H₂O: C, 60.94; H, 6.56; N, 13.78; Found: C, 66.98; H, 6.62; N, 12.73.
(2*endo*,5*exo*)-5-(6,7-Dimethoxyquinoxalin-2-ylamino)-bicyclo[2.2.1] heptan-2-ol (m.p. 90-93°C).
(2*exo*, 5*exo*)-5-(6,7-Dimethoxyquinoxalin-2-ylamino)-bicyclo[2.2.1]heptan-2-ol (m.p. 97-100°C).
(2*endo, 3exo,* 5*exo*)-5-(6,7-Dimethoxyquinoxalin-2-ylamino)-bicyclo[2.2.1]heptane-2,3-diol (m.p. 220-222°C). Anal. Calcd. for C₁₇H₂₁N₃O₄ 0.2 H₂O: C, 60.96; H, 6.44; N, 12.54; Found: C, 60.93; H, 6.06; N, 11.60.

Cyclohexyl-(6,8-dimethyl-quinoxalin-2-yl)-amine [MS m/z: 255 (M+)]. Anal. Calcd. for C₁₆H₂₁N₃: C, 75.26; H; 8.29; N, 16.46; Found: C, 75.08; H, 8.28; N, 15.86.
*cis*/*trans*-2-(6-Methoxy-quinoxalin-2-ylamino)-cyclopentanol (m.p. 137-139°C). Anal. Calcd. for C₁₄H₁₇N₃O₂: C, 64.85; H, 6.61; N, 16.20; Found: C, 64.87; H, 6.45; N, 16.22. *trans*-4-(6-Methoxy-quinoxalin-2-ylamino)-cyclohexanol (m.p. 70-75°C). Anal. Calcd. for C₁₅H₁₉N₃O₂ •0.3 H₂O: C, 64.64; H, 7.09; N, 15.08; Found: C, 64.68; H, 7.06; N, 14.77.
[3aR,4S,6R,6aS]-6-(6,7-Dimethoxyquinoxalin-2-ylamino)-2,2-dimethyl-tetrahydrocyclopenta[1,3]dioxole-4-carboxylic ethylamide (m.p. 94-97°C).
Anal. Calcd. for C₂₁H₂₈N₄O₅ •0.3 H₂O: C, 59.79; H, 6.83; N, 13.28; Found: C, 59.80; H, 6.89; N, 12.03.
(6,7-Dimethoxyquinoxalin-2-yl)-(4-methoxy-cyclohexyl)-amine (m.p. 58-68°C). Anal. Calcd. for C₁₇H₂₃N₃O₃ •0.5 H₂O: C, 62.56; H, 7.41; N, 12.87; Found: C, 62.53; H, 7.22; N, 12.22.

### EXAMPLE 15 exo-2-(Bicyclo[2.2.1]hept-2-yloxy)-6,7-dimethoxy quinoxaline

A mixture of *exo*-2-norborneol (223 mg, 2 mmole) and NaH (60%, 100 mg, 2.5 mmole) in 10 mL of anhydrous THF is refluxed for 0.5 hour before addition of 2-chloro-6,7-dimethoxyquinoxaline (336 mg, 1.5 mmole). The resulting mixture is continued to refluxed for two hours. The residue after filtration and concentration is chromatographed on silica gel (50% ether/hexane) to provide the desired product as a white solid (m.p. 135-137 °C). Anal. Calcd. for C₁₇H₂₀N₂O₃: C, 67.98; H, 6.71; N, 9.33; Found: C, 67.96; H, 6.762; N, 9.19.

The following compounds are prepared similarly beginning with the appropriate starting material.
*exo*-2-(Bicyclo[2.2.1]hept-5-en-2-yloxy)-6,7-dimethoxyquinoxaline (m.p. 108-110°C). Anal. Calcd. for C₁₇H₁₈N₂O₃: C, 68.44; H, 6.08; N, 9.39; Found: C, 68.54; H, 6.23; N, 9.27.
2-(Bicyclo[2.2.1]hept-5-en-2-yloxy)-6,7-dimethoxyquinoxaline (m.p. 93-95°C). Anal. Calcd. for C₁₇H₁₈N₂O₃: C, 68.44; H, 6.08; N, 9.39; Found: C, 68.32; H, 5.98; N, 9.25.
2-(1,4-Dioxa-spiro[4,5]dec-8-yloxy)-6,7-dimethoxyquinoxaline (m.p. 124-125°C). Anal. Calcd. for C₁₈H₂₂N₂O₅: C, 62.42; H, 6.40; N, 8.09; Found: C, 62.63; H, 6.46; N, 7.79.

### EXAMPLE 16 cis/trans-4-(6,7-Dimethoxyquinoxalin-2-yloxy)-cyclohexane carboxylic acid.

A mixture of *cis*/*trans*-4-hydroxy-cyclohexanecarboxylic acid (144 mg, I mmole) and NaH (60%, 160 mg, 4 mmole) in anhydrous THF/DMF(10 mL/2 mL) is refluxed for one hour before addition of 2-chloro-6,7-dimethoxyquinoxaline (225 mg, 1 mmole). The resulting mixture is continued to refluxed for four hours. The reaction mixture is neutralized to pH 5 and extracted with ethyl acetate (2x50 mL). The combined organic solutions are dried over magnesium sulfate and filtered. The residue after concentration is chromatographed on silica gel (ethyl acetate, followed by methanol) to provide the desired product as a white solid (m.p. 90-93 °C). Anal. Calcd. for C₁₇H₂₀N₂O₅ 0.5 H₂O: C, 59.89; H, 6.19; N, 8.22; Found: C, 59.91; H, 6.62; N, 7.90.
The following compounds are prepared similarly beginning with the appropriate starting material
4-(6,7-Dimethoxyquinoxalin-2-yloxymethyl)-cyclohexanol (m.p. 118-121 °C). Anal. Calcd. for C₁₇H₂₂N₂O₄ •0.3 H₂O: C, 63.15; H, 7.03; N, 8.66; Found: C, 63.13; H, 6.65; N, 9.01.
3-(6,7-Dimethoxyquinoxalin-2-yloxy)-cyclohexanol (m.p. 151-153°C). Anal. Calcd. for C₁₆H₂₀N₂O₄: C, 63.14; H, 6.62; N, 9.20; Found: C, 62.56; H, 6.58; N, 8.67.
4-(6,7-Dimethoxyquinoxalin-2-yloxy)-cyclohexanol (m.p. 162-164°C). Anal. Calcd. for C₁₆H₂₀N₂O₄: C, 63.14; H, 6.62; N, 9.20; Found: C, 62.52; H, 6.80; N, 8.88.

### EXAMPLE 17 5-(6,7-Dimethoxyquinoxalin-2-yloxy)-bicyclo [2.2,1]heptane-2,3-diol

To a solution of 2-(bicyclo[2.2.1]hept-5-en-2-yloxy)-6,7-dimethoxy-quinoxaline (149 mg, 0.5 mmole) and 4-methylmorpholine N-oxide (234 mg, 2 mmole) at room temperature in 5 mL of THF is added a solution of OsO₄ in t-butanol (2.5% by wt., 0.2 mL). The brown solution is stirred vigorously for two hours before being quenched with saturated NaHS₂O₃ (2 mL). Ether (3x100 mL) is used to extract and then dried over magnesium sulfate. The residue after filtration and concentration is chromatographed on silica gel (50% ethyl acetate/hexane) to provide the desired product (m.p. 85-88°C). Anal. Calcd. for C₁₇H₂₀N₂O₅ •0.9 H₂O: C, 58.73; H, 6.29; N, 8.06; Found: C, 58.74; H, 5.91; N, 7.53.

Prepared similarly is (2*exo,* 3*exo,* 5*exo*)-5-(6,7-dimethoxyquinoxalin-2-ylamino)-bicyclo[2.2.1]heptane-2,3-diol (m.p. 150-153 °C).

### EXAMPLE 18 Acetic acid cis-4-(6,7-dimethoxyquinoxalin-2-yloxy)-cydohexyl ester and cis-4-(6,7-dimethoayquinoxalin-2-yloxy)-cyclohexanol

A mixture of *cis* 4-acetoxy-cyclohexanol (632 mg, 4 mmole) and NaH (60%, 220 mg, 5.5 mmole) in 15 mL of anhydrous THF is refluxed for 0.5 hour before addition of 2-chloro-6,7-dimethoxyquinoxaline (674 mg, 3 mmole). The resulting mixture is continued to be refluxed for two hours. The residue after filtration and concentration is chromatographed on silica gel (ether) to provide acetic acid *cis*-4-(6,7-dimethoxyquinoxalin-2-yloxy)-cyclohexyl ester (m.p. 150-152°C). Anal. Calcd. for C₁₈H₂₂N₂O₅: C, 62.42; H, 6.40; N, 8.09; Found: C, 62.39; H, 6.55; N, 7.82 and *cis*-4-(6,7-dimethoxyquinoxalin-2-yloxy)-cyclohexanol (m.p. 148-150°C). Anal. Calcd. for C₁₆H₂₀N₂O₄: C, 63.14; H, 6.62; N, 9.20; Found: C, 62.80; H, 6.76; N, 8.67.
*trans*-4-(6,7-Dimethoxyquinoxalin-2-yloxy)-cyclohexanol [MS *m*/*z:* 304 (M⁺)] is prepared similarly.

### EXAMPLE 19 Dimethyl-carbamic acid 4-(6,7-dimethoxyquinoxalin-2-yloxy)-cyclohexyl ester

A mixture of 4-(6,7-dimethoxyquinoxalin-2-yloxy)-cyclohexanol (100 mg, 0.33 mmole), dimethylcarbamyl chloride (90 µL, 1.2 mmole) and NaH (60%, 19.6 mg, 0.49 mmole) in 5 mL of THF is stirred at room temperature for three days to provide a white solid (m.p. 152-155 °C) isolated by chromatography (50% ethyl acetate/hexane). Anal. Calcd. for C₁₉H₂₅N₃O₅: C, 60.79; H, 6.71; N, 11.19; Found: C, 60.38; H, 6.54; N, 10.43.

### EXAMPLE 20 3-Cyclohexyloxy-6,7-dimethoxyquinoxaline 1-oxide.

A mixture of 2-cyclohexyloxy-6,7-dimethoxyquinoxaline (110 mg, 0.38 mmole) and meta-chlorobenzoic peracid (70%, 113 mg, 0.46 mmole) in 10 mL of methylene chloride is stirred at room temperature for one day. The solution after filtration is concentrated and the residue is chromatographed on silica gel (20% ethyl acetate/hexane) to provide the desired product (m.p. 167-169 °C).
*trans*-4-(6,7-Dimethoxy-4-oxy-quinoxalin-2-ylamino)-cyclohexanol (m.p. 220-222°C) is prepared similarly. Anal. Calcd. for C₁₆H₂₁N₃O₄ •0.2 H₂O: C, 59.42; H, 6.69; N, 12.99; Found: C, 59.43; H, 6.64; N, 12.95.

### EXAMPLE 21 Acetic acid trans-4-(6,7-dimethoxyquinoxalin-2-ylamino)-cyclohexyl ester

A mixture of *trans*-4-(6,7-dimethoxyquinoxalin-2-ylamino)-cyclohexanol (303 mg, 1 mmol), acetic anhydride (2 mL) and pyridine (2 mL) in 10 mL of dichloromethane is stirred at room temperature overnight. The mixture is quenched with water (5 mL) and extracted with dichloromethane (2x 30 mL). After drying over magnesium sulfate and filtration, the solution is concentrated on a rotovap. The residue is chromatographed on silica gel (ethyl acetate) to provide the desired acetate as a light yellow solid (m.p. 176-177°C). Anal. Calcd. for C₁₈H₂₃N₃O₄: C, 62.59; H, 6.71; N, 12.17; Found: C, 62.89; H, 6.67; N, 11.95.

### EXAMPLE 22 (2exo,5exo)-5-(6,7-Dimethoxyquinoxaline-2-ylamino)-bicyclo[2.2.1]heptan-2-ol

A mixture of (2*exo*,5*exo*)-5-aminobicyclo[2.2.1]heptan-2-acetate (127 mg, 0.75 mmol ) and 2-chloro-6,7-dimethoxyquinoxaline (224 mg, 1 mmol) is heated to 180°C for six hours. After which time, the mixture is cooled to room temperature, dissolved in methylene chloride and purified via flash column. The recovered product (20 mg, 7.5 % yield) is dissolved in methanol (2 mL), and a fresh solution of 1 N sodium methoxide (0.063 mL, 0.063 mmol ) is added. The reaction mixture is refluxed for ninety minutes. The crude mixture is purified by preparative thin layer chromatography to provide the product as a yellow solid with a m.p. of 97-100°C. C₁₇H₂₁N₃O₃ (m/z): 315.

The following compounds are prepared similarly beginning with the appropriate starting material
(2*endo*,5*exo*)-5-(6,7-Dimethoxyquinoline-2-ylamino)-bicyclo[2.2.1]heptan-2-ol, as a yellow solid. C₁₇H₂₁N₃O₃ (m/z ): 315. (2*exo*, 6*exo*)-6-(6,7-Dimethoxy-quinolin-2-ylamino)-bicyclo[2.2.1]heptan-2-ol, as a yellow solid (30 mg, overall 21 %). C₁₇H₂₁N₃O₃ (m/z ): 315. Anal. Calcd. for C₁₇H₂₁N₃O₃: C 64.74; H, 6.71; N, 13.32; Found C 58.42; H, 6.26; N, 11.56.

### EXAMPLE 23 (2trans,4cis)-4-(6,7-Dimethoxyquinoxaline-2-ylamino)-2-methyl-cyclohexanol and (2trans,4trans)-4-(6,7-dimethoxyquinoxalin-2-ylamino)-2-methyl-cyclohexanol (herein after GN

A mixture of 2-chloro-6,7-dimethoxy quinoxaline (1.08 g, 4.81 mmol ) and (2*trans*)-4-amino-2-methylcyclohexanol (620 mg, 4.81 mmol) is heated to 180°C for six hours. The reaction yielded two diastereomers.

The major isomer is isolated as a yellow solid, assigned as (2*trans*,4 *trans)-*4-(6,7-dimethoxyquinoxalin-2-ylamino)-2-methyl-cyclohexanol (240 mg, 0.76 mmol. C₁₇H₂₃N₃O₃ (m/z): 317. Anal. Calcd. for C₁₇H₂₃N₃O₃ •2H₂O: C 58.00; H, 7.69; N, 11.94; Found C 58.0; H, 6.58; N, 11.24.

The minor isomer is also a yellow solid, assigned as (2*trans,*4*cis*)-4-(6,7-dimethoxyquinoXalin-2-ylamino)-2-methyl-cyclohexanol, C₁₇H₂₃N₃O₃ (m/z): 317. Anal. Calcd. for C₁₇H₂₃N₃O₃ •H₂O: C 60.08; H, 6.94; N, 12.53; Found C 61.21; H, 6.94; N, 11.56.

The (2*trans*,4*trans*)-4-(6,7-dimethoxyquinoxalin-2-ylamino)-2-methyl-cyclohexanol is separated further by chiral HPLC into its individual enantiomers. The first enantiomer has a (+)-rotation (elution order on Chiracel OJ). The second enantiomer has a (-)-rotation (elution order on Chiracel OJ). Analytical conditions using a Chiracel OD column resulted in the (+) enantiomer eluting second. The (-)-enantiomer exhibits a preferred activity in a PDGF-R ELISA assay.

### EXAMPLE 24 (2cis,4cis)-4-(6,7-Dimethoxyquinoxalin-2-ylamino)-2-methyl-cyclohexanol and (2cis,4trans)-4-(6,7-dimethoxyquinoxalin-2-ylamino)-2-methyl-cyclohexanol

To a solution of a 2:1 mixture of (2*trans*,4*trans*)-4-(6,7-dimethoxy-quinoxalin-2-ylamino)-2-methyl-cyclohexanol and (2*trans*,4*cis*)-4-(6,7-dimethoxyquinoxalin-2-ylamino)-2-methyl-cyclohexanol (120 mg, 0.38 mmol) in THF (7 mL) is added triphenylphosphine (110 mg, 0.42 mmol) and diethyl azodicarboxylate (0.066 mL, 0.42 mmol) and benzoic acid (46.4 mg, 0.38 mmol). The mixture is stirred at room temperature overnight and the residue after work-up is separated on silica gel (30% ethyl acetate/hexane) to provide a mixture of benzoates.

To a solution of the major benzoate (50 mg, 0.12 mmol) in methanol (2 mL) is added 1N sodium hydroxide (0.12 mL, 0.12 mmol). The pure product (13 mg, 32 % yield) is isolated from preparative thin layer chromatography as a yellow solid (m.p. 85-88°C), assigned as (2*cis*,4*cis*)-4-(6,7-dimethoxy-quinoxalin-2-ylamino)-2-methyl-cyclohexanol. C₁₇H₂₃N₃O₃ (m/z ): 317.

Similarly the minor benzoate (4.4 mg) is hydrolyzed and the desired product (3.3 mg, 100%) is also isolated from preparative thin layer chromatography as a yellow solid, assigned as (2*cis*,4*trans*)-4-(6,7-dimethoxyquinoxalin-2-ylamino)-2-methyl-cyclohexanol. C₁₇H₂₃N₃O₃ (m/z): 317.

### EXAMPLE 25 (1R,2R,4S)-(+)-Bicyclo[2.2.1]hept-2-yl-(6,7-dimethoxy quinoxalin-2-yl)-amine.

The (±)-bicyclo[2.2.1]hept-2-yl-(6,7-dimethoxyquinoxalin-2-yl)-amine of Example 14 is resolved on a chiral HPLC column (Chiralpac AD, 25x2 cm, 60% heptane/40% ethanol with 10 mM (1S)-(+)-camphorsulfonic acid, 12 mL/minute) and the above titled product is obtained as the first eluent. The fractions collected are combined and washed with 50 mL of 1 N NaOH before drying (MgSO₄) The solution after filtration is concentrated on a rotovap and then dried under a high vacuum. A yellow solid is obtained. [α]_{d}²⁰ +19.5° (c=0.20, CH₂Cl₂) m.p. 184-186 °C. Anal. calcd for C₁₇H₂₁N₃O₂ x 0.3 H₂O: C, 66.90; H, 7.15; N, 13.77. Found: C, 66.86; H, 7.01; N, 13.86.

### EXAMPLE 26 Biotransformative Preparation of (1S,2R,4S,5R)-5-(6,7-Dimethoxyquinoxalin-2-ylamino)-Bicyclo[2.2.1]heptan-2-ol (or GN804)

Fungi Strain F 2052 (Mortierella isabellina) is purchased from the Northern Utilization Research and Development Division (NRRL).

The fungi is stored at -25°C. 250 mL conical flasks each containing 50 mL seed culture medium (medium 216) are inoculated with 2 mL of fungi suspension and incubated on a rotary shaker (200 rpm ) at 23°C for 3 days. 250 mL conical flasks each containing 50 mL of the same medium were inoculated with 2 mL of the seed culture and incubated on a rotary shaker (200 rpm ) at 23°C. After 24 hours, (1R,2R,4S)-(+)-Bicyclo[2.2.1]hept-2-yl-(6,7-dimethoxyquinoxalin-2-yl)-amine of Example 25 is dissolved in MeOH and added to the flasks to a final concentration of 300 mg/L. The cultures are harvested after 24 hours of incubation. (Medium 216: Glucose 0.4%, Yeast extract 0.05%, Soya flour 0.05%, NaCl 0.05%, KH₂PO₄ 0.05.) The extraction is performed using 2 volumes of acetonitrile, 1 volume de tert-butylmethyl ether and 1 volume of n-heptane were added to I volume of broth. After magnetic stirring at 22°C, the extract separates to 3 layers. The intermediate layer is collected and evaporated to dryness, and redissolved in ethyl acetate. The ethyl acetate extract is separated on silica gel (0.04-0.063 mm ) using ethyl acetate as eluent. Fractions containing the biotransformation product are separated on C18 silica using a H₂O/MeOH gradient as eluent. This chromatography yields the pure titled compound as an amorphous yellow powder, m.p. 190-192°C.

### EXAMPLE 27 trans-4-[7-methoxy-6-(2-morpholin-4-yl-ethoxy)-quinoxalin-2-ylamino]- cyclohexanol and trans-4-[6-methoxy-7-(2-morpholin-4-yl-ethoxy)-quinoxalin-2-ylamino]-cyclohexanol

The title compound is prepared by Mitsunobu coupling of 6-hydroxy-7-methoxy-2-chloroquinoxaline:7-(2-morpholin-4-ylethoxy)-6-methoxy-2 chloroquinoxaline and 2-(morpholin-4-yl)ethanol using the procedure of Example 1 and reaction of the resulting 6-(2-morpholin-4-ylethoxy)-7-methoxy-2-chloroquinoxaline: 7-(2-morpholin-4-ylethoxy)-6-methoxy-2-chloroquinoxaline and trans-4-amino-cyclohexanol using the procedure of Example 11.

### EXAMPLE 28 2-[2-(trans-4-Hydroxy-cyclohexylamino)-7-methoxy-quinoxalin-6 yloxyl]-1-acetic acid and 2-[2-(trans-4-Hydroxy-cyclohexylamino)-6-methoxy-quinoxalin-7-yloxyl]-1-acetic acid

The title compound is prepared by dealkylation of 4-(6,7-dimethoxyquinoxaline-2-ylamino)cyclohexanol using the sodium salt of ethanethiol in DMF as described in Example 4, followed by alkylation with bromoacetic acid in the presence of base as described in general procedure 6.

### EXAMPLE 29 2-[2-(trans-4-Hydroxy-cyclohexylamino)-7-methoxy-quinoxalin-6-yloxyl]-N,N-dimethyl-acetamide and 2-[2-(trans-4-Hydroxy-cyclohexylamino)-6-methoxy- quinoxalin-7-yloxyl]-N,N-dimethyl-acetamide

This compound is prepared by aminolysis of the compound of Example 28 using dimethylamine.

### INTERMEDIATE EXAMPLE 1 4-Bromo-5-methoxy-benzene-1,2-diamine dihydrochloride

To a solution of EtOAc (50 mL) and 5-bromo-4-methoxy-2-nitro-phenylamine (2.5 g, 10 mmol) under argon is added 5% Pd/C (0.5 g). The reaction mixture is hydrogenated at 50 psi for 1 hour. The mixture is filtered through Celite into a solution of HCl/IPA/EtOAc, and the pad is washed with additional EtOAc. The resulting precipitate is filtered off to provide white solid.

### INTERMEDIATE EXAMPLE 2 7-Bromo-6-methoxy-quinoxalin-2-ol and 6-Bromo-7-methoxy-quinoxalin-2-ol

To a solution of MeOH (15 mL) under argon is added pulverized NaOH pellets (0.86 g, 21 mmol) and 4-bromo-5-methoxy-benzene-1,2-diamine dihydrochloride (2.7 g, 9.3 mmol). The mixture is stirred for 10 minutes, then a solution of 45% ethyl glyoxylate in toluene (2.7 g, 12 mmol) is added portionwise. The reaction mixture is refluxed for 1 hour, then cooled. Water is added, then the suspension is filtered. The resulting solid is washed successively with H₂O, MeOH, IPA, and Et₂O to provide a yellow powder.

### INTERMEDIATE EXAMPLE 3 7-Bromo-2-chloro-6-methoxy-quinoxaline and 6-Bromo-2-chloro-7-methoxly-quinoxaline

To a mixture of 7-bromo-6-methoxy-quinoxalin-2-ol and 6-bromo-7-methoxy-quinoxalin-2-ol (1 g, 3.9 mmol is added POCl₃ (5 mL). The reaction mixture is refluxed 1 hour, poured into ice water, filtered, then washed with water to provide a light-tan solid. Ratio of 7-bromo-2-chloro-6-methoxy-quinoxaline : 6-bromo-2-chtoro-7-methoxy-quinoxaline is approximately 7:1 by ¹H NMR.

### INTERMEDIATE EXAMPLE 4 5-Chloro-4-methoxy-2-nitroaniline

To a solution of N-(5-chloro-4-methoxy-2-nitrophenyl)-acetamide (2 g, 8.2 mmol) in 5N HCl (20 mL) is added 1,4-dioxane (10 mL), and the mixture is stirred at 60°C for 1.5 hours. The reaction mixture is concentrated and partitioned between EtOAc/2 N NaOH. The aqueous layers are washed with EtOAc (3X), brine, dried (MgSO₄), adsorbed onto silica gel, and chromatographed (70% EtOAc/hexanes) to provide an orange powder.

### INTERMEDIATE EXAMPLE 5 4-Chloro-5-methoxy-benzene-1,2-diamine dihydrochloride

To a solution of EtOAc (25 mL) and 5-chloro-4-methoxy-2-nitro-phenylamine (1.6 g, 7.9 mmol) under argon is added 5% Pd/C (0.5 g). The reaction mixture is hydrogenated at 50 psi for 1 hour. The mixture is filtered under N₂ through Celite into a solution of 1 N HCl/Et₂O in EtOAc, and the pad is washed with additional EtOAc. The resulting precipitate is filtered off to provide a white solid.

### INTERMEDIATE EXAMPLE 6 7-Chloro-6-metboxy-quinoxalin-2-ol and 6-Chloro-7-methoxy-quinoxalin-2-ol

To a solution of 4-chloro-5-methoxy-benzene-1,2-diamine dihydrochloride (1.8 g, 7.2 mmol) in EtOH (15 mL) under argon is added TEA (2.5 mL, 18 mmol) at 0°C. The mixture is stirred for 20 minutes, then a solution of 45% ethyl glyoxylate in toluene (2.1 g, 9.3 mmol) is added portionwise. The reaction mixture is warmed to room temperature, refluxed for 1.5 hour, and then cooled. Water is added, the suspension is then filtered and washed successively with H₂O, IPA, and Et₂O to provide a light-yellow powder. The product is azeotroped several times with toluene and dried *in vacuo* before use.

### INTERMEDIATE EXAMPLE 7 2,7-Dichloro-6-methoxy-quinoxaline and 2,6-Dichloro-7-methoxy-quinoxaline

To a mixture of 7-chloro-6-methoxy-quinoxalin-2-ol and 6-chloro-7-methoxy-quinoxalin-2-ol (1 g, 4.7 mmol) under a CaCl₂ drying tube is added POCl₃ (5 mL). The reaction mixture is refluxed 30 minutes, poured into cold saturated NaHCO₃ solution, filtered, then washed with water to provide a solid. The ratio of 2,7-dichloro-6-methoxy-quinoxaline : 2,6-dichloro-7-methoxy-quinoxaline is approximately 6:1 by ¹H NMR.

### INTERMEDIATE EXAMPLE 8 cis-4-Aminocyclohexanol

*cis*-4-aminocyclohexanol is made according to the literature procedure with minor modification [J. Med Chem. 18(6) 634 1975].

### INTERMEDIATE EXAMPLE 9 exo-Bicyclo[2.2.1]hept-5-en-2-amine

*exo*-bicyclo[2.2.1]hept-5-en-2-amine is prepared with the same procedures as in INTERMEDIATE EXAMPLE 15 from 5-norbornen-2-ol via a versatile intermediate *exo*- 2-bicyclo[2.2.1]hept-5-en-2-yl isoindole-1,3-dione

### INTERMEDIATE EXAMPLE 10 (2exo, 6exo)-2-(6-Hydroxy-bicyclo[2.2.1] hept-2-yl isoindole-1,3-dione and (2exo, 5exo)-2-(5-hydroxy-bicyclo[2.2.1]hept-2-yl isoindole-1,3-dione

To a mixture of *exo*-2-bicyclo[2.2.1]hept-5-en-2-yl isoindole-1,3-dione (320 mg, 1.34 mmole) in 5 mL of THF at 0 °C is added a BH₃/THF solution (1 M, 2 mL, 2 mmole). The mixture is stirred at room temperature for two hours before addition of water (2 mL) and NaBO₃ ●4H₂O (900 mg). The resulting suspension is stirred overnight. Ether (3x50 mL) is used to extract and dried over magnesium sulfate. The residue after filtration and concentration is chromatographed on silica gel (ether) to provide the desired products which can be further separated.

### INTERMEDIATE EXAMPLE 11 (2exo, 5endo)-2-(5-Hydroxy-bicyclo[2.2.1] hept-2-yl isoindole-1,3-dione

(a): A mixture of (2*exo*, 6*exo*)-2-(6-hydrory-bicyclo[2.2.1]hept-2-yl isoindole-1,3-dione and (2*exo*, 5*exo*)-2-(5-hydroxy-bicyclo[2.2.1]hept-2-yl isoindole-1,3-dione (800 mg, 3.3 mmole), and pyridinium chlorochromate (2 g) in 10 mL of methylene chloride is stirred at room temperature over the weekend. After being diluted with ether (100 mL), the suspension is filtered and the solution is concentrated. The residue is chromatographed on silica gel (ether) to give 750 mg (95%) of the corresponding ketones. The ketones are further separated by reverse phase HPLC (CH₃CN/H₂O, 10-70%) to provide *exo*-2-(5-oxy-bicyclo[2.2.1]hept-2-yl isoindole-1,3-dione.
(b): To a solution of *exo*-2-(5-oxy-bicyclo[2.2.1]hept-2-yl isoindole-1,3-dione (250 mg, 0.98 mmole) in 10 mL of methanol at 0°C is added NaBH₄ (38 mg, 1 mmole). The mixture is stirred for additional half-hour and quenched with 1N HCl (1mL). After being concentrated, the residue is extracted with methylene chloride (2x50 mL). Evaporation of methylene chloride gave the desired product used directly without further purification.

### INTERMEDIATE EXAMPLE 12 (2endo, 5exo)-5-Amino-bicyclo[2.2.1]heptan -2-ol, (2exo, 5exo)-5-amino-bicyclo[2.2.1]heptan-2-ol, (2endo, 6exo)-6-amino-bicyclo[2.2.1]heptan-2-ol, and (2exo, 6exo)-6-amino-bicyclo[2.2.1]heptan-2-ol

The titled compounds are prepared from proper starting material by application of above procedure of INTERMEDIATE EXAMPLE 11.

### INTERMEDIATE EXAMPLE 13 2-Methyl-6,7-dimethoxyquinoxaline

The title compound is prepared using an adaptation of the published method of Tamao, et al. Tetrahedron, 1982, 38, 3347-3354. To a THF solution under argon is added 2-Chloro-6,7-dimethoxyquinoxaline (5 g, 26 mmol) and NiCl₂(dppp) (0.14 g, 0.26 mmol). The reaction mixture is cooled to 0°C, and a 3 M solution of MeMgBr in Et₂O (13 mL, 39 mmol) is added portionwise. The reaction mixture is allowed to warm to room temperature, stirred for 1 hour, then refluxed for 1.5 hours. The mixture is cooled, quenched with 10% HCl, stirred 10 minutes, then made basic with 5% NaOH. CH₂Cl₂ and H₂O are added to the reaction, and the mixture stirred overnight. Additional CH₂Cl₂,H₂O, and NaCl are then added and the mixture is filtered. The resulting solution is poured into a separatory funnel, and the aqueous layers are washed 3X with CH₂Cl₂. The organic layers are combined, washed with brine, dried (MgSO₄) concentrated onto silica gel, and chromatographed (50%-80% EtOAc/hexanes) to provide a orange solid (49% yield).

### INTERMEDIATE EXAMPLE 14 6,7-Dimethoxy-2-quinoxaline carboxaldehyde

To a reaction flask under argon is added 1,4-dioxane (20 mL), 2-methyl-6,7-dimethoxyquinoxaline (1.09 g, 5.3 mmol) and SeO₂ (1.8g, 16 mmol). The mixture is heated to 100°C for 2 hours 45 minutes, cooled, and filtered through Celite. The pad is washed with portions of EtOAc and CH₂Cl₂. The resulting solution is concentrated, taken up in MeOH/ CH₂Cl₂, loaded onto a silica gel column, and chromatographed (30% EtOAc/CH₂Cl₂) to provide an off-white solid (73% yield).

### INTERMEDIATE EXAMPLE 15 (2exo, 5exo)-5-Aminobicyclo[2.2.1]heptan-2-acetate

*exo*-5-Acetoxybicyclo[2.2.1]heptan-2-one and *exo*-6-acetoxybicyclo[2.2.1] heptan-2-one are obtained from the bicyclo[2.2.1]hepta-2,5-diene according to the procedure of R. Gagnon (J. Chem. Soc., Perkin trans. 1, 1505 1995) with minor modification.

To a solution of *exo*-5-acetoxybicyclo[2.2.1]heptan-2-one (350 mg, 2.08 mmol) in 10 mL of THF at room temperature is added a 1M borane/THF solution (1.2 mL, 1.2 mmol). The mixture is stirred for 0.5 hour before quenched at 0°C with methanol (3 mL) and 1N HCl (1.5 mL). Ethyl acetate (3x 30 mL) is used to extract and dried over magnesium sulfate. The residue after filtration and concentration is chromatographed on silica gel to provide (2*endo*,5*exo*)-5-acetoxybicyclo [2.2.1] heptan-2-ol.

To a solution of (2*endo,* 5*exo*)-5-acetoxybicyclo [2.2.1] heptan-2-ol (350 mg, 2.06 mmol) in THF (10 mL) is added phthalimide (454 mg, 3.09 mmol ), triphenylphosphine (810 mg, 3.09 mmol ) and diethyl azodicarboxylate (0.49 mL, 3.09 mmol ) at 0°C. The reaction is left to stir overnight and then is condensed on the rotovap and the residue is purified by column chromatography (20% ethyl acetate/hexane) to provide the desired product as a yellow solid.

A mixture of the above solid (300 mg, 1 mmol ) and hydrazine (0.126 mL, 2.2 mmol ) in 5 mL of methanol is heated to reflux for six hours. After removal of methanol, dichloromethane (3x 30 mL) is used to extract the residue. Concentration of the solvent affords (*exo,exo*)-5-aminobicyclo[2.2.1]heptan-2-acetate (127 mg, 75%) which is used in the coupling reaction without further purification.

Similarly, (2*endo*,5*exo*)-5-aminobicyclo[2.2.1]heptan-2-acetate, (2*endo,* 6*exo*)-6-aminobicyclo[2.2.1]heptan-2-acetate and (2*exo*,6*exo*)-6-aminobicyclo[2.2.1]heptan-2-acetate are prepared from proper starting material.

### INTERMEDIATE EXAMPLE 16 (2trans) -4-Amino-2-methylcyclohexanol

A mixture of 3-methyl-2-cyclohexenone (4 g, 36.36 mmol), toluenesulfonic acid (100 mg) and ethylene glycol (7 mL) in 100 mL of toluene is refluxed overnight and water formed is removed by Dean-Stark trap. The residue after concentration is chromatographed on silica gel (10% ethyl acetate/hexane) to give 3.36 g (62%) of 7-methyl-1,4-dioxa-spiro[4.5]dec-7-ene.

To a stirred solution of 7-methyl-1,4-dioxa-spiro[4.5]dec-7-ene (3.36 g, 22.47 mmol ) in tetrahydrofuran (THF) (125 mL) is added a 1M solution of borane in THF (22.47 mL, 22.47 mmol) at room temperature. The mixture stirred for one hour, and the reaction is quenched by adding H₂O (10 mL) at 0°C followed by sodium perborate tetrahydrate (10.0g, 66 mmol). The mixture is left to stir overnight. The two layers are separated, and the aqueous layer is washed several times with ethyl acetate (4 x 150 mL). The desired alcohol is obtained as a clear liquid after flash column chromatography.

The above alcohol (1.8 g, 10.5 mmol ) is dissolved in methanol (50 mL) and 1N HCl (16 mL). The reaction mixture is left to stir overnight. The acidic solution is neutralized with 1N sodium hydroxide (18 mL) and normal aqueous work-up followed. The crude mixture is purified by flash column (50% ethyl acetate) to give *trans* 4-hydroxy-3-methyl-cyclohexanone.

To a solution of *trans* 4-hydroxy-3-methyl-cyclohexanone (780 mg, 6.1 mmol) water (3 mL) is added hydroxylamine hydrochloride (550 mg, 7.92 mmol ), followed by the slow addition of a saturated solution of sodium carbonate (326 mg, 3.8 mmol) in water (1.02 mL). After stirring for thirty minutes, ether is added to the reaction mixture, and the two layers are separated. The organic layer is condensed and dissolved in ethanol (10 mL). To the refluxing ethanol solution is added sodium (1.8 g, 78.3 mmol ) over a period of one hour and the resulting mixture is heated for additional 2.5 hours. After removal of ethanol, n-propanol (10 mL), ether (25 mL), and water (3 mL) is added. The organic solution is dried over magnesium sulfate and filtered. Concentration of solvents affords a mixture of (2*trans*)-4-amino-2-methylcyclohexanol as a white solid.

### INTERMEDIATE EXAMPLE 17 2-methoxy-4,5-diaminophenol dihydro chloride

The title compound is prepared by hydrogenation of 2-methoxy-4,5-dinitrophenol according to the procedure of Ehrlich et al., J. Org.Chem., 1947, 12, 522.

### INTERMEDIATE EXAMPLE 18 7-hydroxy-6-methoxy-quinoxaline-2-ol and 6-hydroxy-7-methoxy-quinoxaline-2-ol.

The title compounds are prepared from 4-methoxy-5-hydroxybenzene-1,2-diamine dihydrochloride by reaction with NaOH and ethyl glyoxalate using the procedure of Intermediate Example 2.

### INTERMEDIATE EXAMPLE 19 7-hydroxy-6-methoxy-2-chloroquinoxaline and 6-hydroxy-7-methoxy-2-chloroquinoxaline.

The title compounds are prepared from 7-hydroxy-6-methoxy-quinoxaline-2-ol and 6-hydroxy-7-methoxy-quinoxaline-2-ol by reaction with POCl₃ using the procedure of Intermediate Example 3.

### EXAMPLE 30: PDGFR TYROSINE KINASE INHIBITORY ACTITY

To determine the effectiveness of compounds of this invention, the pharmacological tests described below, which are accepted in the art and recognized to correlate with pharmacological activity in mammals, are utilized. Compounds within the scope of this invention have been subjected to these various tests, and the results obtained are believed to correlate to useful cellular differentiation mediator activity. The results of these tests are believed to provide sufficient information to persons skilled in the pharmacological and medicinal chemistry arts to determine the parameters for using the studied compounds in one or more of the therapies described herein.

### EXAMPLE 30.1 PDGF-R Tyrosine Kinase Autophosphorylation ELISA assay

The titled assay is performed as described by Dolle et al. (J. Med Chem. 1994, 37, 2627), which is incorporated herein by reference, with the exception of using the cell lysates derived from Human aortic smooth muscle cells (HAMSC) as described below.

### EXAMPLE 30.2 Mitogenesis Assay General Procedure

### a. Cell Culture

Human aortic smooth muscle cells (passage 4-9) are plated in 96 well plates in a growth supporting medium at 6000 cells/well and allowed to grow 2-3 days.
At approximately 85% confluence, cells are growth arrested with serum free media (SFM).

### b. Mitogenesis Assay

After 24 hour serum deprivation, medium is removed and replaced with test compound/vehicle in SFM (200 µl/well). Compounds are solubilized in cell culture DMSO at a concentration of 10 mM and further dilutions are made in SFM.

After 30 min preincubation with compound, cells are stimulated with PDGF at 10 ng/mL. Determinations are performed in duplicate with stimulated and unstimulated wells at each compound concentration.

Four hours later, 1 µCi ³H thymidine/well is added.

Cultures are terminated 24 hours after addition of growth factor. Cells are lifted with trypsin and harvested onto a filter mat using an automated cell harvester (Wallac MachII96). The filter mat is counted in a scintillation counter (Wallac Betaplate) to determine DNA-incorporated label.

### EXAMPLE 30.3 Chemotaxis Assay

Human aortic smooth muscle cells (HASMC) at earlier passages are obtained from ATCC. Cells are grown in Clonetics SmGM 2 SingleQuots (media and cells at passages 4-10 are used. When cells are 80% confluent, a fluorescent probe, calcein AM (5 mM, Molecular Probe), is added to the media and cells are incubated for 30 minutes. After washing with HEPES buffered saline, cells are lifted with trypsin and neutralized with MCDB 131 buffer (Gibco) with 0.1% BSA, 10 mM glutamine and 10% fetal bovine serum. After centrifugation, cells are washed one more time and resuspended in the same buffer without fetal bovine serum at 30000 cells/50 mL. Cells are incubated with different concentrations of a compound of formula I (final DMSO concentration = 1 %) for 30 min at 37°C. For chemotaxis studies, 96 well modified Boyden chambers (Neuroprobe, Inc.) and a polycarbonate membrane with 8 mm pore size (Poretics, CA) are used. The membrane is coated with collagen (Sigma C3657, 0.1 mg/mL). PDGF-ββ (3 ng/mL) in buffer with and without a compound of formula I are placed in the lower chamber. Cells (30,000), with and without inhibitor, are placed in the upper chamber. Cells are incubated for 4 hours. The filter membrane is removed and cells on the upper membrane side are removed. After drying, fluoresce on the membrane is determined using Cytofluor II (Millipore) at excitation/emission wavelengths of 485/530 nm. In each experiment, an average cell migration is obtained from six replicates. Percent inhibition is determined from DMSO treated control values. From five points concentration-dependent inhibitions, IC₅₀ value is calculated. Results are presented as a mean±SEM from five such experiments.

### EXAMPLE 30.4 EGF-Receptor Purification

EGF-receptor purification is based on the procedure of Yarden and Schlessinger. A431 cells are grown in 80 cm² bottles to confluency (2 x 10⁷ cells per bottle). The cells are washed twice with PBS and harvested with PBS containing 11.0 mmol EDTA (1 hour at 37°C, and centrifuged at 600g for 10 minutes. The cells are solubilized in 1 mL per 2 x 10⁷ cells of cold solubilization buffer (50 mmol Hepes buffer, pH 7.6, 1% Triton X-100, 150 mmol NaCl, 5 mmol EGTA, 1 mmol PMSF, 50 mg/mL aprotinin, 25 mmol benzamidine, 5 mg/mL leupeptic, and 10 mg/mL soybean trypsin inhibitor) for 20 minutes at 4°C. After centrifugation at 100,000g for 30 minutes, the supernatant is loaded onto a WGA-agarose column (100 mL of packed resin per 2 x 10⁷ cells) and shaken for 2 hours at 4°C. The unabsorbed material is removed and the resin washed twice with HTN buffer (50 mmol Hepes, pH 7.6, 0.1% Triton X-100, 150 mmol NaCl), twice with HTN buffer containing 1 M NaCl, and twice with HTNG buffer (50 mmol Hepes, pH 7.6, 0.1% Triton X-100, 150 mmol NaCl, and 10% glycerol). The EGF receptor is eluted batchwise with HTNG buffer containing 0.5 M N-acetyl-D-glucosamine (200 mL per 2 x 10⁷ cells.). The eluted material is stored in aliquots at -70°C and diluted before use with TMTNG buffer (50 mmol Tris-Mes buffer, pH 7.6, 0.1% Triton X-100, 150 mmol NaCl, 10% glycerol).

### EXAMPLE 30.5 Inhibition of EGF-R Autophosphorylation

A431 cells are grown to confluence on human fibronectin coated tissue culture dishes. After washing 2 times with ice-cold PBS, cells are lysed by the addition of 500 mL/ dish of lysis buffer (50 mmol Hepes, pH 7.5, 150 mmol NaCl, 1.5 mmol MgCl₂, 1 mmol EGTA, 10% glycerol, 1% triton X-100, 1 mmol PMSF, 1 mg/mL aprotinin, 1 mg/mL leupeptin) and incubating 5 minutes at 4°C. After EGF stimulation (500 mg/mL 10 minutes at 37°C) immunoprecipitation is performed with anti EGF-R (Ab 108) and the autophosphorylation reaction (50 mL aliquots, 3 mCi [g⁻³²P]ATP) sample is carried out in the presence of 2 or 10 mM of compound of the present invention, for 2 minutes at 4°C. The reaction is stopped by adding hot electrophoresis sample buffer. SDA-PAGE analysis (7.5% els) is followed by autoradiography and the reaction is quantitated by densitometry scanning of the x-ray films.

### a. Cell Culture

Cells termed HER 14 and K721A are prepared by transfecting NIH3T3 cells (clone 2.2) (From C. Fryling, NCI, NIH), which lack *endo*genous EGF-receptors, with cDNA constructs of wild-type EGF-receptor or mutant EGF-receptor lacking tyrosine kinase activity (in which Lys 721 at the ATP-binding site is replace by an Ala residue, respectively). All cells are grown in DMEM with 10% calf serum (Hyclone, Logan, Utah).

### EXAMPLE 30.6 Selectivity vs. PKA and PKC is determined using commercial kits

### a. Pierce Colorimetric PKA Assay Kit, Spinzyme Format

Brief Protocol:
PKA enzyme (bovine heart) 1U/assay tube
Kemptide peptide (dye labeled) substrate
45 minutes @ 30°C
Absorbance at 570 nm

### b. Pierce Colorimetric PKC Assay kit, Spinzyme Format

Brief Protocol:
PKC enzyme (rat brain) 0.025U/assay tube
Neurogranin peptide (dye labeled) substrate
30 minutes @ 30°C
Absorbance at 570 nm

### EXAMPLE 30.7 p56^{LCK} Tyrosine Kinase Inhibition Activity Measurements

p56*^{LCK}* Tyrosine kinase inhibition activity is determined according to a procedure disclosed in United States Patent No. 5,714,493,

In the alternative, the tyrosine kinase inhibition activity is determined according to the following method. A substrate (tyrosine-containing substrate, Biot-(β Ala)₃-Lys-Val-Glu-Lys-Ile-Gly-Glu-Gly-Thr-Tyr-Glu-Val-Val-Tyr-Lys-(NH₂) recognized by P56^{LCK} , 1 µM) is first phosphorylated in presence or absence of a given concentration of the test compound, by a given amount of enzyme (enzyme is produced by expression of P56^{LCK} gene in a yeast construct) purified from a cloned yeast (purification of the enzyme is done by following classical methods) in the presence of ATP (10µM) MgCl2(2.5mM), MnCl2 (2.5mM), NaCl (25mM), DTT (0.4mM) in Hepes 50mM, pH 7.5, over 10 min at ambient temperature. The total reaction volume is 50µl, and the reactions are performed in a black 96-well fluoroplate. The reaction is stopped by addition of 150µl of stopping buffer (100mM Hepes pH7.5, KF 400mM, EDTA 133 mM, BSA 1g/l.) containing a selected anti tyrosine antibody labelled with the Europium cryptate (PY20-K) at 0.8µg/ml and allophycocyanine-labelled streptavidin (XL665) at 4µg/ml. The labelling of Streptavidin and anti-tyrosine antibodies were performed by Cis-Bio International (France). The mixture is counted using a Packard Discovery counter which is able to measure time-resolved homogeneous fluorescence transfer (excitation at 337 nm, readout at 620 nm and 665 nm). The ratio of the 665 nm signal / 620nm signal is a measure of the phosphorylated tyrosine concentration. The blank is obtained by replacing enzyme by buffer. The specific signal is the difference between the ratio obtained without inhibitor and the ratio with the blank. The percentage of specific signal is calculated. The IC₅₀ is calculated with 10 concentrations of inhibitor in duplicate using Xlfit soft. The reference compound is staurosporine (Sigma) and it exhibits an IC₅₀ of 30± 6 nM (n=20).

### EXAMPLE 30.8 Measurement of In Vitro Tumor Inhibition

The inhibition of tumor growth in vitro by the compounds of this disclosure is determined as follows:

C6 rat glioma cell line (provided by ATCC) is grown as monolayers in Dubelcco's Modified Eagle Medium containing 2 mM L-glutamine, 200 U/ml penicillin, 200 µg/ml streptomycin and supplemented with 10% (v/v) heat inactivated foetal calf serum. Cells in exponential phase of growth are trypsinized, washed with PBS and diluted to a final concentration of 6500 cells/ml in complete medium. Drug to be tested or control solvent are added to the cell suspension (2.5 ml) under a volume of 50 µl and 0.4 ml of 2.4% Noble Difco agar maintained at 45°C are added and mixed. The mixture is immediately poured into Petri dishes and left standing for 5 minutes at 4 °C. The number of cellular clones (>60 cells) are measured after 12 days of incubation at 37 °C under 5% CO₂ atmosphere. Each drug is tested at 10, 1, 0.1, and 0.01 µg/ml (final concentration in the agar) in duplicate. Results are expressed in percent inhibition of clonogenicity relatively to untreated controls. IC_{50'}s are determined graphically from semi-logarithmic plots of the mean value determined for each drug concentration.

### EXAMPLE 30.9 Measurement of Tumor Inhibition In Vivo

The inhibition of tumor growth in vivo by the compounds of this disclosure is determined using a subucatenous xenograft model as described in U.S. Pat. Nos. 5,700,823 and 5,760,066, in which mice are implanted with C6 glioma cells and tumor growth is measured using venier calipers.

The results obtained by the above experimental methods evidence that the compounds within the scope of the present disclosure possess useful PDGF receptor protein tyrosine kinase inhibition properties or p56*^{LCK}* tyrosine kinase inhibition properties, and thus possess therapeutic value. The above pharmacological test results may be used to determine the dosage and mode of administration for the particular therapy sought.

### EXAMPLE 31: Therapeutic Effects of GN963, GN804, and GN271 on Vascularization and Growth of Human Pancreatic Cancer in Orthotopic Organs of Nude Mice

### Example 31.1: In vitro studies

The expression of PDGF-Rα/β by human pancreatic cancer cells (L3.6pL), growing *in vitro* is first determined. The human tumor cells are cultured with different concentrations of PDGF AA, BB, or A/B, and examined for their expression of phosphorylated PDGF-R by immunohistochemistry and Western blot analysis.

The inhibition of phosphorylation *(in vitro)* of PDGF-R (α,β) by trans-4-(6,7-dimethoxy-quinoxalin-2-ylamino)-cyclohexanol sulfate, GN963, on human pancreatic cancer cells is assessed. Inhibition of phosphorylation is also assessed using GN804 and GN271. Cells are treated with PDGF AA or BB in the presence of different concentrations of GN963, GN804 and GN271 and the expression of the receptor and its level of phosphorylation are analyzed in presence or absence of a chemotherapeutic drug, such as Taxotere.

The expression of phosphorylated PDGF-R by organ-specific endothelial cells and effect of GN963 is further confirmed by culturing endothelial cells of the pancreas in presence of PDGF AA, BB, and A/B and in the presence or absence of GN963, GN804 or GN271 and by examining the expression level of the PDGF-R and phosphorylated PDGF-R. Lung endothelial cells serve as a negative (no expression of PDGF-R) control.

It is showed that blockade of the PDGF-Rβ increases sensitivity of endothelial cells to Taxotere. Mouse endothelial cells from pancreas are maintained in culture at a temperature 33°C where they are actively dividing. IC₅₀ studies with Taxotere are then carried out, and it is shown that GN963, GN804 or GN271 increases sensitivity of endothelial cells to cytotoxicity mediated by Taxotere. The levels of BCl₂ and PI3K/Akt in EC cells exposed to GN963 to demonstrate a decreased resistance to apoptosis.

Effect of pancreatic tumor-conditioned medium on an increased resistance of organ-specific endothelial cells to Taxotere and the reversal of this increased resistance are demonstrated in the presence of GN963, GN804 or GN271.

### Example 31.2: In vivo studies

A pancreatic tumor which is shown to upregulate expression of PDGF AA, BB, A/B at metastatic sites is used for the in vivo studies. Other tumor systems that are known to upregulate expression of PDGF may also be used such as ovarian cancer, prostate cancer, bone metastasis, or breast cancer bone metastasis.

Tumor cells L3.6pL derived from pancreatic cancer are injected into the pancreas of nude mice. The tumors are grown into detectable lesions.

A single oral dose of GN963 at 0 mg/kg, 25 mg/kg, 50 mg/kg, 100 mg/kg, 200, mg/kg, and 400 mg/kg is administered. Two days later, the mice are killed and the organs with and without tumors harvested, fixed, and immunostained to evaluate expression of PDGF AA, BB, A/B, PDGF-R (α,β) and status of phosphorylation of PDGF-R (α,β) on tumor cells and organ-specific endothelial cells. It is showed that tumor cells express the ligand and that both tumor cells and adjacent (but not distant) endothelial cells express the receptor (phosphorylated), and that expression of phosphorylated PDGF-R on endothelial cells is inhibited by oral administration of GN963.

Also, therapeutic effect of treatment with GN963, GN804 or GN271 alone, Taxotere alone, or GN963, GN804 or GN271 plus Taxotere is assessed in mice treated at a selected or optimal dose and schedule for 4-6 weeks or until control mice become moribund. The surviving mice are then killed and the size of tumor and metastasis is measured. Fixation of the tissues and staining of the tumor cells are performed. Endothelial cells are examined for division (PCNA), apoptosis (TUNEL), CD31 (endothelial cells). Expression of PDGF AA, BB, A/B, and phosphorylation of PDGF-Rα/β are further studied in these endothelial cells. A surprisingly higher survival rate is observed in mice treated with the synergistic combination of GN963, GN804 or GN271 and Taxotere.

The synergistic effect of the GN963, GN804 or GN271 and Taxotere combination is also showed in cancer refractory to chemotherapy. The primary antivascular response of the GN963 plus chemotherapy and hence, antitumor response, is confirmed by repeating this experiment of using human tumor cells selected for resistance to Taxotere (MDR cells).

### EXAMPLE 32: Inhibition of Angiogenesis by GN963

### Example 32.1: In Vitro Studies

Human umbilical vein cells (Huvec)s are grown with 2% FCS, 10 ng/ml rhFGF2 or 50 ng/ml VEGF and 10 µg/ml heparin, in the presence of different concentration of GN963, GN804 or GN271 prepared in house in comparison with DMSO diluent. IC50s is then determined. It is showed that SRC like kinases, and particularly cFYN, are involved.

### Example 32.2: In vivo Studies - Anti-angiogenic effect of GN963, GN804 or GN²⁷¹ and Treatment of rheumatoid arthritis.

Collagen-induced arthritis (CIA) is an experimental autoimmune disease is elicited in susceptible strains of rodents (rat and mouse) and nonhuman primates by immunization with type II collagen (CII), the major constituent protein of articular cartilage. Following immunization, these animals develop an autoimmune-mediated polyarthritis that shares several clinical, histological, and immunological features with the human autoimmune disease RA. As with rheumatoid arthritis, susceptibility to CIA in rodents is linked to the class II molecules of the major histocompatibility complex (MHC). The immune response to CII is characterized by both the stimulation of collagen-specific T cells and the production of high titers of antibody specific for both the immunogen (heterologous CII) and the autoantigen (mouse or rat CII). Histologically, mouse and rat CIA models are characterized by an intense synovitis that corresponds precisely with the clinical onset of arthritis. Within a few days of onset, erosion of cartilage and subchondral bone by pannus-like tissue is evident, and healing by fibrosis and ankylosis of involved joints follows slowly. Because of the important similarities between CIA and rheumatoid arthritis, this experimental model of autoimmune arthritis has been the subject of extensive investigation and can be considered the "golden standard" model. For this reason, this model can be used to determine efective treatments in the susceptible strain of mouse DBA/1N (haplotype H2^{q}).

### Collagen-Induced Arthritis in DBA/1N mice: pilot study (CAX 712/07)

### Experimental design

### Animals

Thirty male DBA/1N mice of 8-10 weeks (Charles River, Belgium) are used in this study. Mice are housed in individual cages and have free access to water and standard chow.

### Preparation of the collagen emulsion

One day before injection of the emulsion to the animals, bovine CII (MD Biosciences, Switzerland; Lot 112002) is dissolved in 0.05 M acetic acid to obtain a 2 mg.ml⁻¹ solution. The solution is kept at +4°C overnight under stirring. This solution can be kept frozen for I month.

The emulsion is prepared extemporaneously by mixing an equal volume of CII with complete Freund's adjuvant (CFA) or incomplete Freund's adjuvant (IFA) as follows. Using a high-speed homogenizer (Heidolph DIAX 600, Germany) with an ultra-fine shaft (type 6G, Heidolph, Germany), the CII solution is added drop by drop to CFA or IFA in a glass tube kept on ice to prevent denaturation of collagen at 8000 rpm for 2 min. To complete the emulsification and achieve a stable emulsion, the homogenizer sped-up to 20,500 rpm for 2 min. The emulsion is kept on ice until injection to the animals.

### Induction of collagen-induced arthritis

Two kinds of immunization have been assessed in this study, as described in the literature:
- Single immunization: a first group of 10 mice receive a s.c. injection at the base of the tail of 100 µg (100 µl) of CII emulsified in CFA (MD Biosciences, Switzerland; Lot 112102) on day 0.
- Double immunization: a second group of 10 mice receive a s.c. injection at the base of the tail of 100 µg (100 µl) of CII emulsified in CFA (MD Biosciences, Switzerland; Lot 112102) on day 0, followed by a s.c. injection at the base of the tail of 100 µg (100 µl) of CII emulsified in IFA (MD Biosciences, Switzerland; Lot 062102) on day 21 (boost injection).
- Sham group: a third group of 10 mice receive a s.c. injection at the base of the tail of 100 µl of saline on day 0.

All injections are performed under general gaseous anesthesia (1-2% isoflurane under a flux of O₂), using 27G needles.

### Follow-up of animals

Daily examination of mice is performed to ensure that no ethical problems occurred following immunization with CII. Body weight is measured twice a week.

Since this study is the first ever and to limit pain elicited by CIA, the protocol can end 10 days after occurrence of the first signs of the pathology for each individual (erythema or edema of any joint).

### Clinical evaluation of collagen-induced arthritis

CIA is evaluated on various clinical criteria as classically described in literature.

### Incidence of CIA

Incidence is calculated as the number of mice affected by CIA divided by the number of animals immunized with CII and is expressed as a percentage.

### Onset of CIA

Date of occurrence of the first signs of the pathology (redness and edema of any joint) is noted for all individuals.

### Arthritic score

Arthritic score is evaluated for each paw according to the following gradation:
- 0: normal;
- 1: swelling and redness of ankle or wrist or swelling and redness of one toe or finger joint;
- 2: moderate to severe swelling and redness of ankle or wrist;
- 3: swelling and redness of the entire hand or foot;
- 4: whole inflammation of any paw with several joints involved.

Arthritic score was assessed before injection of CII (baseline), 14 and 21 days after injection of CII, the day of onset and 10 days after onset.

### Paw thickness

Quantification of paw swelling is assessed for each paw by measurement of paw thickness with digital square calipers. Paw thickness is measured before (baseline) and 21 days after injection of CII, the day of onset and 10 days after onset.

### Histopathological evaluation of collagen-induced arthritis

At the end of the study, mice are sacrificed by cervical dislocation under general gaseous anesthesia and the 4 paws are harvested to be prepared for histological analysis as described in the report BS-03-009. Several histopathological criteria have been used to grade the severity of CIA: synovial hyperplasia (synovial membrane thickness of more than two cell layers), general inflammation, cartilage loss, bone destruction, pannus formation.

The severity of CIA is classified as normal, minimal, mild, moderate or severe based on the following criteria:
- 0: Normal;
- 1: Minimal: minimal to mild synovial inflammation, no cartilage or bone loss;
- 2: Mild: minimal to mild synovial inflammation, cartilage loss, and bone erosion limited to discrete foci;
- 3: Moderate: synovitis and erosion present but joint architecture intact;
- 4: Severe: synovitis, extensive erosions and joint architecture disrupted.
   Treatment of CIA mice is performed with the standard dexamethasone, as positive control, and various concentration of GN963, GN804 or GN271.

### EXAMPLE 33: Therapeutic effects of GN963, GN804 or GN271 on Transplants/metastases of 4T1 and 3LL tumor cells.

4T1 cells (0.5 x 10⁵) are injected into number 4 mammary glands of BALB/c virgin female mice. Mice are then treated with GN963, GN804 or GN271 in separate experiments before and after resection of the primary tumor 10 days after establishment. After 10 days, primary tumors are resected. Lungs were harvested 8 weeks later, and surface metastases were counted. For 3LL cells (0.5 x 10⁵) will be injected sub-cutaneously into Blk/6 mice. After 10 days, primary tumors are resected. Mice are then treated with GN963, GN804 or GN271 in separate experiments before and after resection of the primary tumor 10 days after establishment. Lungs are harvested 3-4 weeks later, and surface metastases are counted.

Tumor growths on the 4T1 and 3LL models that dosed therapeutically with GN963, GN804 or GN271 without the resection of the primary tumor are then assessed.

### EXAMPLE 34: Inhibition of SRC-like kinase for treatment and prevention of metastasis invasion

The role of SRC-like kinase in the metastatic cascade by using syngeneic murine models of metastasis is showed. The tumor lines are 3LL and 4T1 (Lewis lung carcinoma and breast adenocarcinoma cell lines). 3LL is syngeneic on Blk/6 mice and 4T1 on BALB/c mice. 4T1 or 3LL cell grown in culture in the presence of different concentration of GN963, GN804 or GN271 prepared in house in comparison with DMSO as a diluent will be used to generate IC50s. The role of SRC kinase and other SRC like kinases is then assessed in the survival of these cell types, utilizing RNA interference and dominant negative SRC constructs to ascertain the role of SCR in proliferation.

### EXAMPLE 35: Profiling of the kinase specificity of GN963 and its cis-isomer in comparison with Gleevec

The IC50 for recombinant kinase transphophorylation was determined for trans-4-(6,7-dimethoxy-quinoxalin-2-ylamino)-cyclohexanol sulfate (GN963), its cis-isomer, *e.g*., cis-4-(6,7-dimethoxy-quinoxalin-2-ylamino)-cyclohexanol sulfate and Gleevec. IC50 values are measured by testing 10 concentrations of each compound in singlicate (n = 1). The ability of a set of protein kinases - tyrosine and serine/threonine kinases expressed in a baculovirus system - to incorporate ³³P (from ³³P -ATP) in relevant substrates, in the presence of decreasing concentrations of the inhibitor (10 concentrations from 10⁻⁵ to 3.10⁻¹⁰, n=1) was determined. Table I summarizes the IC50 values obtained for a set of protein tyrosine kinases. Importantly, GN963 did not inhibit phosphorylation mediated by protein kinases belonging to families different from the tyrosine kinase family.

**Table 1**

| **IC50 values in nM** | | | |
|---|---|---|---|
| Protein Kinases | GN963 | Cis-isomer | Gleevec |
| PDGFRa | 730 | 1400 | 120 |
| PDGFRb | 8000 | 2200 | 1700 |
| Kit | 7200 | 6100 | 400 |
| Abl-1 | 13000 | 4400 | 6400 |
| Src | 3600 | 3200 | >10000 |
| Brk | 10000 | 5700 | >10000 |
| Lck | 7700 | 7500 | >10000 |
| Flt-3 | 1400 | >10000 | >10000 |
| FGR | 17000 | 4600 | >10000 |
| Irak | 4100 | >10000 | >10000 |
| Eph-B4 | 7200 | >10000 | >10000 |
| Met | >10000 | 10000 | >10000 |
| VEGF-R1 | >10000 | >10000 | >10000 |
| VEGF-R2 | >10000 | >10000 | >10000 |
| EGF-R | >10000 | >10000 | >10000 |
| FGF-R1 | >10000 | >10000 | >10000 |
| FGF-R4 | >10000 | >10000 | >10000 |
| IGF1-R | >10000 | >10000 | >10000 |
| Ins-R | >10000 | >10000 | >10000 |

This profiling confirmed both PDGF receptors as targets for GN963. Moreover, it allowed the identification of the additional following targets:
- members of the class III receptor tyrosine kinase family: KIT and FLT-3 (in addition to PDGFR)
- members of the SRC family: SRC, LCK, FGR
- and ABL, IRAK, Eph-B4 and potentially Met.

The pattern obtained with Gleevec (Abl, Kit and PDGFRs) was as expected. Importantly, the specificity pattern of the cis-isomer was similar to GN963 pattern, as it did not show any specific effect.

### EXAMPLE 36: Inhibition of FLT-3 mutant and SRC like tyrosine kinase by GN963 and Treatment of AML

The kinase profiling identified FLT3 as a target of GN963. This kinase plays an important role in hematopoiesis as well as in leukemogenesis. FLT3 is activated following binding of FLT3 ligand (FL), which causes receptor dimerization leading to increased kinase activity and activation of downstream signaling pathways including Stat5, Ras, and PI3-kinase. FLT3 normally regulates survival and proliferation of hematopoietic progenitor cells, in particular by synergy with other RTKs and cytokine receptors. FLT3 is also expressed on acute myelogenous leukemia (AML) cells from the majority of patients and stimulates survival and proliferation of leukemic blasts. Two classes of activating FLT3 mutations have been identified in AML patients: internal tandem duplication (ITD) mutations in the juxtamembrane region expressed in 25% to 30% of AML patients, and point mutations in the activation loop of the kinase domain found in approximately 7% of patients. Both classes of mutation result in constitutive FLT3 tyrosine kinase activity and have been shown to transform hematopoietic cell lines in vitro and in vivo.

FLT3-ITD is the most frequently observed molecular defect in AML and has been found in pediatric, adult, and elderly AML patients at frequencies of 10% to 16%, 21% to 27%, and 24% to 34%, respectively. FLT3-ITD has been shown to be the single most significant poor prognosis factor in AML in several recent independent studies. Clinically, FLT3-ITD is associated with increased leukocytosis, increased blast count, increased relapse rate, decreased disease-free survival, and poor overall survival. A recent study has shown that an increased ratio of FLT3-ITD relative to wild-type FLT3 (FLT3-WT) confers a poorer prognosis and that the FLT3-WT allele is absent in a minority of patients. The catalytic Asp835 point mutation is also associated with leukocytosis and poor prognosis, though not as statistically significant as FLT3-ITD. FLT3 therefore appears to be necessary for disease progression and is an attractive target for consideration in AML therapies.

Cellular assays to detect changes in FLT3 phosphorylation are performed using leukemia cell lines RS;411 and AML193 (which express wild-type FLT3) and MV4-11 and MOLM-13 (which express FLT3-ITD mutant). Treatment with GN963 inhibited FLT3-ITD phosphorylation in a dose-dependent manner allowing determination of an IC50, as determined by immunoprecipitations/western blot experiments.

The effects of GN963 on AML cell line proliferation are shown in short duration proliferation assays (24h after seeding, cells were incubated with inhibitor for 72h and proliferation assessed with the Cell Titer-Glo Luminescent kit from Promega). For the FLT3-ITD cell lines MV4-11 and MOLM-13, GN963 inhibited cellular proliferation in a dose-dependent manner with an IC50 ranging between 0.7 and 2µM. In contrast to the results with FLT3-ITD cell lines, RS4;11 and AML193 were not inhibited by GN963.

Also, the ability of GN963 to induce apoptosis in these AML cell lines was shown. MV4-11 and MOLM-13 cells were incubated 48 to 72 hours with GN963. Apoptosis was evaluated by means of flow cytometry to measure cellular binding of an annexin V-FITC conjugate as well as uptake of the vital stain propidium iodide.

The efficacy of GN963 to inhibit phosphorylation of FLT3 downstream signaling via the MAPK, PI3K and STAT5 pathways is assessed in AML FLT3-ITD cell lines. Treatment with GN963 inhibits STAT5, MAPK and PI3K phosphorylation in a dose-dependent manner allowing determination of an IC50, as determined in immunoprecipitations/western blot experiments.

### Example 36.1: In Vitro Studies

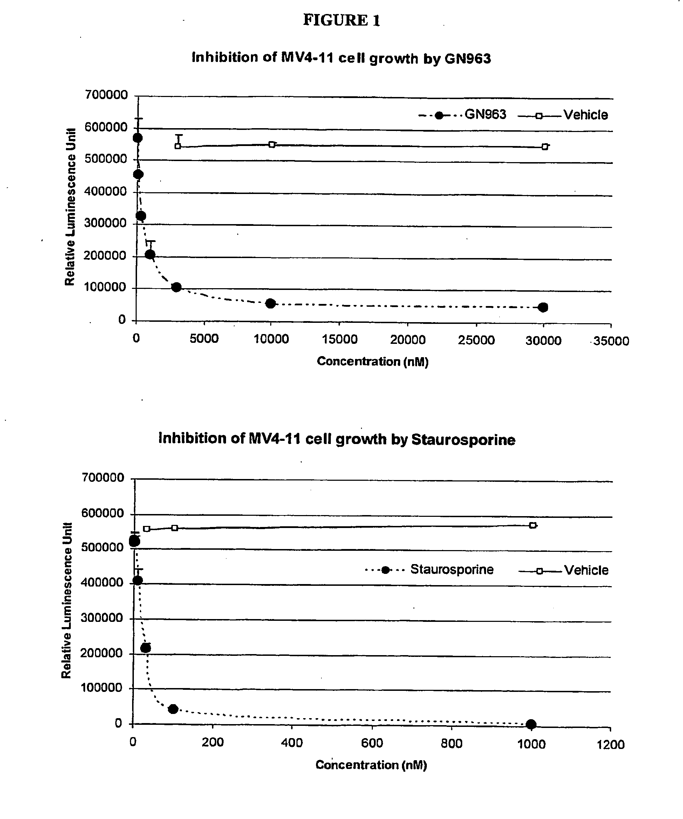

GN963 activity was first tested on MV4-11 (ATCC CRL-9591), a biphenotypic leukemia cell line with a FLT3 ITD mutation [Blood 2002;99:3885]. MV4-11 are added to 96-well plates at densities of 10 000 cells per well and incubated overnight in Iscove modified dulbecco's medium supplemented with 10% FCS (37°C. 5% CO₂). GN963 is added, and cell growth was measured at 72 hours using a luminescence assay (Promega). Staurosporine, a non specific kinase inhibitor, was used as positive control. Data show that GN963 inhibits MV4-11 cell growth in a dose dependent manner (Figure 1).

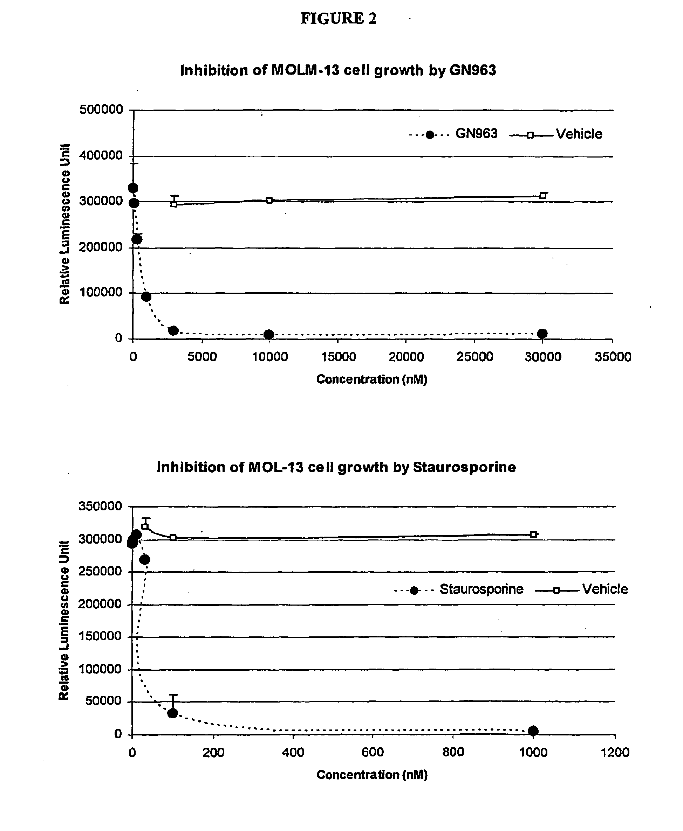

GN963 is also tested on MOLM13 (DSMZ ACC 554) known to express ITD-FLT3. MOLM-13 are used as described for MV4-11 in example 2. MOLM-13 are added to 96-well plates at densities of 10 000 cells per well and incubated overnight in RPMI 1640 supplemented with 10% FCS (37°C. 5% CO₂). GN963 was added, and cell growth was measured at 72 hours using a luminescence assay (Promega). Staurosporine, a non specific kinase inhibitor, is used as positive control. The results show a dose response effect of GN963 on MOLM-13 cell growth (Figure 2).

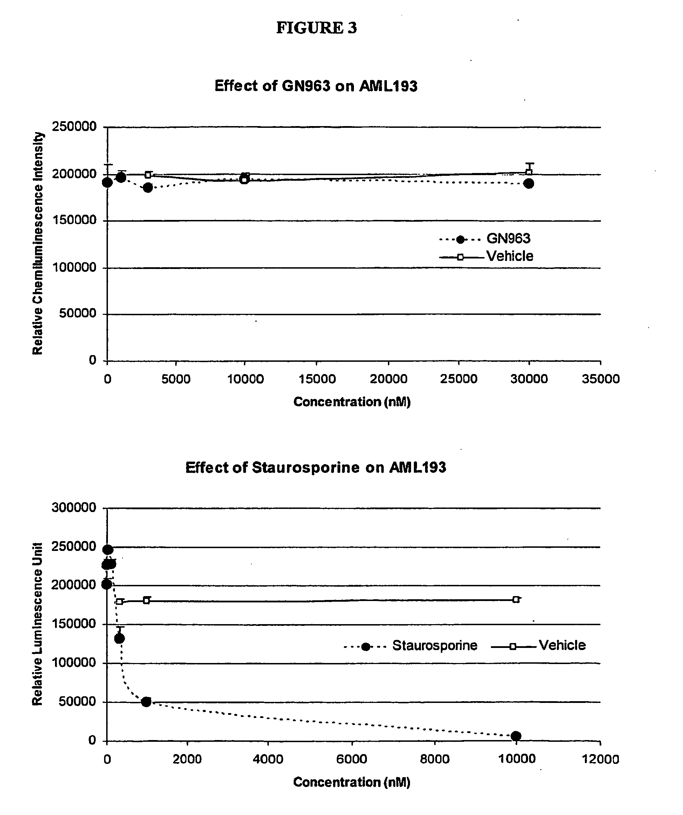

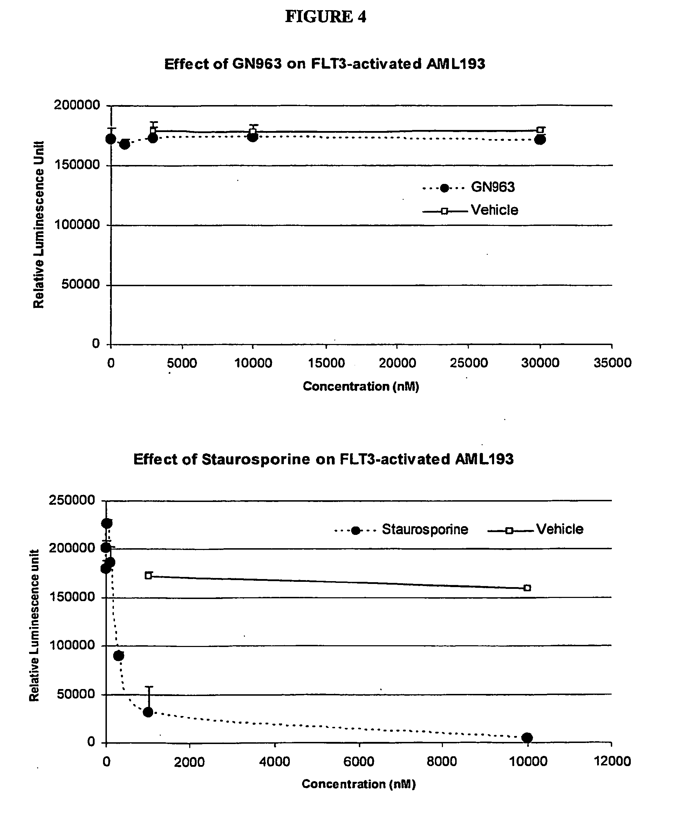

To determine whether GN963 has the same effect on FLT3 wild type, AML193 cell line (ATCC CRL-9589) expressing a wild type FLT3 was used. To remove growth factor, AML193 are rinsed once in medium without growth factors. AML193 are plated at densities 10 000 cells/well and incubated overnight with Iscove modified dulbecco's medium supplemented with 10% FCS. AML193 are cultured with or without 100 ng/mL of FLT3 ligand + GN963 or staurosporine for 72 hours. Cell growth is measured as described previously. GN963 has no effect on GN963 at concentrations used (30 µM) even when AML193 are cultured with FLT3 ligand. On the contrary, staurosporine inhibits AML193 cell growth even when FLT3 ligand was used (Figures 3 and 4).

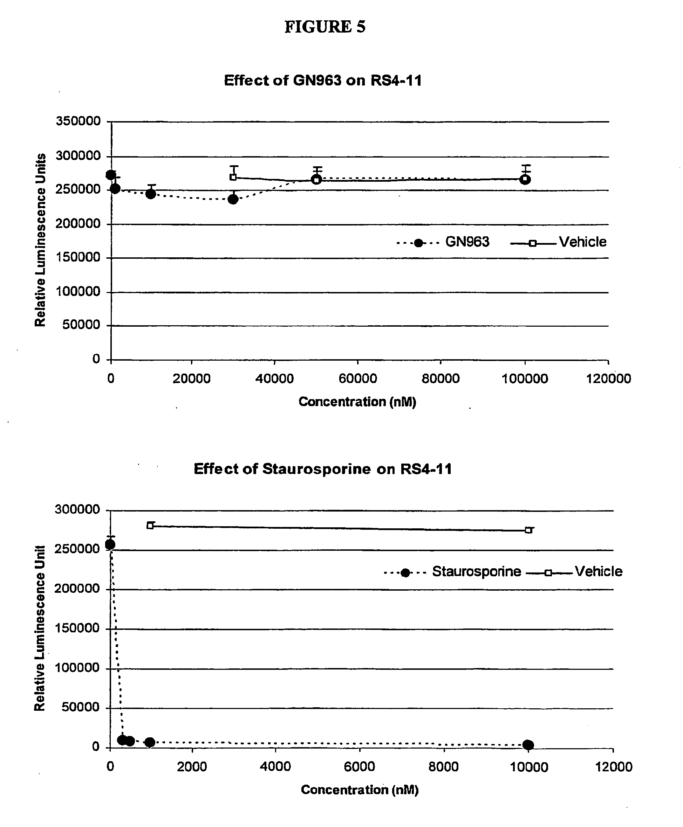

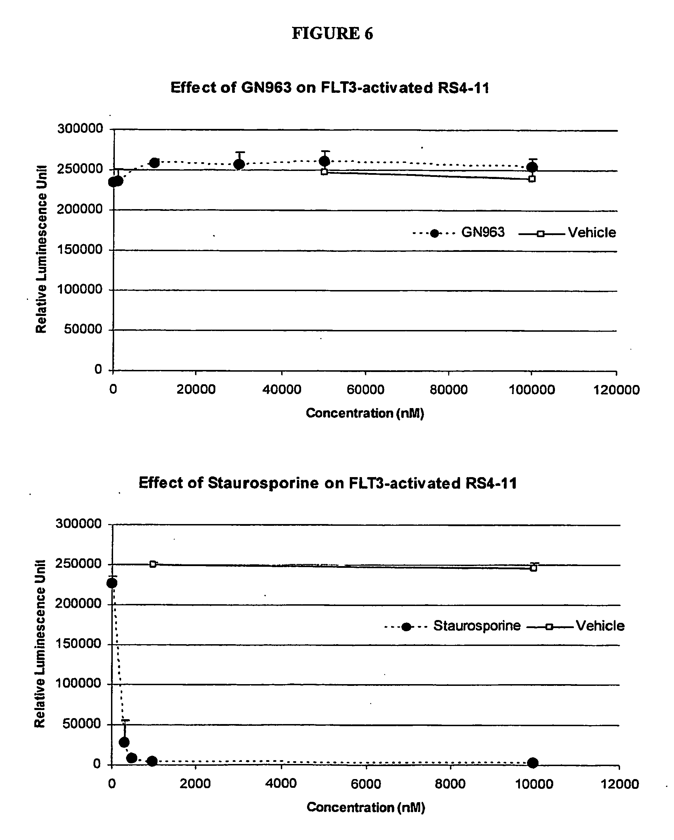

RS4-11, another cell line which express FLT3-wild type with a cytogenetic rearrangement similar to MV4-11 was obtained (ATCC CRL-1873). RS4-11 are cultured overnight at 10 000 cells /well in RPMI 1640 supplemented with 10%FCS without other growth factors. RS4-11 are then cultured for 72 hours with or without 100 ng/mL FLT3 ligand with GN963 or staurosporine. Cell growth is measured using a luminescence assay. GN963 has no effect on RS4-11 cell growth whereas staurosporine inhibits RS4-11 cell growth even when FLT3 ligand is added (Figures 5 and 6).

### EXAMPLE 36: Inhibition of FLT-3 mutant and SRC like tyrosine kinase by GN963 and Treatment of AML

### Example 36.2: In Vivo Studies

Athymic nu/nu mice receive subcutaneous injections into the hind flank on day 0 with 5.10⁶ MV4; 11or RS4;11 cells (human AML cells exhibiting FLT3-ITD mutation or FLT3-wild type, respectively). In vivo experiments evaluate the therapeutic effects of daily or 3 times per week oral administration of GN963 on pre-existing tumors (size 300-500 mm³). Animals are randomized into treatment groups of 15 mice each for efficacy studies. A range of doses (50-200 mg/kg) of GN963, GN804 or GN271 or its vehicle is administered. Tumor growth is measured twice weekly using Vernier calipers for the duration of the treatment. Tumor volumes are calculated as the product of length x width x height. On completion of the experiments, mice are sacrificed and tumors are harvested for determination of FLT3 and phosphorylated FLT3 in tumor lysates by immunoprecipitation and Western-blot. Our data provide evidence that compound has efficacy against tumor growth, consistent with inhibition of FLT-3 phosphorylation *in vivo.*

### Example 36.3: In Vivo Studies

NOD-SCID mice are pretreated with cyclophosphamide by intraperitoneal injection of 150 mg/kg/d for 2 days followed by 24 hours of rest prior to intravenous injection of 5.10⁶ MV4;11 cells (human AML cells exhibiting FLT3-ITD mutation) via the tail vein. A range of doses of GN963, GN804 or GN271 (50-200 mg/kg) or its vehicle is orally administered once daily or 3 times per week after 3 weeks of implantation of the cells and continued through the end of the experiment (15 mice per group). Kaplan-Meier survival plots of vehicle-treated and GN963-treated mice are established to demonstrate therapeutic efficacy of the compound. In a subset of animals, bone marrow cell suspensions are prepared by flushing mouse femurs with cold sterile PBS, at experimental timepoints (within 90 days of implantation). Morphologic examination of H&E-stained bone marrow sections from vehicle-treated or GN963, GN804 or GN271-treated animals is then performed to evaluate the extent of myeloproliferation of leukemia cells. Bone marrow is also used for flow cytometric analysis of human CD45 expression as a marker for MV4;11 cells from MV4;11-inoculated mice treated with vehicle or GN963, GN804 or GN271. Our data demonstrate evidence of prolonged survival in GN963, GN804 or GN271-treated groups, consistent with reduced numbers of large infiltrating cells with mitotic figures and reduced numbers of CD45 positive cells.

### EXAMPLE 37: Inhibition of ABLT315I tyrosine kinase mutant by GN963, GN804 or GN271 and Treatment of CML refractory to Gleevec and ALL

An important clinical concern pertaining to imatinib mesylate therapy is relapse after an initial response, particularly in patients with advanced phase CML. For example, among patients with accelerated phase enrolled in a phase 2 clinical trial, the incidence of disease progression at 24 months was 50%. Between 60% and 90% of patients who acquire imatinib mesylate resistance harbor one or more specific mutations in the kinase domain of BCR-ABL that impair the ability of imatinib mesylate to inhibit BCR-ABL kinase activity. These mutations presumably affect drug binding without eliminating adenosine 5'-triphosphate (ATP) binding or kinase activity.

Clinically observed mutations have been identified within several regions of the BCR-ABL kinase domain. 6 common kinase domain variants collectively account for at least 60% of reported BCR-ABL mutations in relapsed patients: Q252H, Y253F, E255K, **T315I.** M351T, and H396P. The panel encompasses several functionally distinct kinase domain regions, including the nucleotide binding P-loop (Q252H, Y253F, E255K), 2 imatinib mesylate contact residues (Y253F and **T315I**), the base supporting the activation loop (M351T), and the activation loop (H396P).

Currently, there is considerable interest in developing alternative ABL kinase inhibitors capable of inhibiting the BCR-ABL kinase domain mutants observed in relapsed patients. Up to now, no inhibitor able to inhibit the mutant T315I has been reported. Furthermore, homologous mutations have been found in other Gleevec-resistant pathologies such as GIST (Kit T670I) or HES (FIP 1L1-PDGFRa-T674I).

Interestingly, ABL, PDGFR and KIT display a threonine at this homologous position, while FLT3 harbors a phenylalanine. If the phenylalanine side chain does not interfere with GN963 binding to FLT-3, the hydrocarbon side chain of isoleucine may also be unable to prevent GN963 binding to ABL T315I. Moreover, mutating this phenylalanine to a threonine makes FLT-3 Gleevec-sensitive. Inversely, converting PDGFRβ threonine to phenylalanine makes PDGFRbeta Gleevec-resistant (JBC 278:5148). Altogether, this raises the possibility that GN963 is an inhibitor of the BCR-ABL T315I mutant.

As described in Example 35, the IC50 for the transphophorylation of a relevant peptide by recombinant human kinase ABL and its mutant T315I is determined for GN963, its cis-isomer and Gleevec. The ability of GN963 to inhibit AblT315I phosphorylation makes it a very attractive drug in relapsed patients displaying this mutation.

Also, the ability of GN963 to inhibit cellular BCR-ABL tyrosine phosphorylation and cellular proliferation, to induce apoptosis and to affect BCR-ABL downstream signaling is investigated in CML cell line models.

### Brief Description of the Figures

**Figure 1****:** displays inhibition of MV4-11 cell growth by increasing concentrations of GN963 and staurosporine versus the negative control (vehicle) measured at 72 hours using a luminescence assay.
**Figure 2****:** displays inhibition of MOLM-13 cell growth by increasing concentrations of GN963 Staurosporine versus the control (vehicle) measured at 72 hours using a luminescence assay.
**Figure 3****:** displays inhibition of AML193 cell growth by increasing concentrations of GN963 versus the control (vehicle) measured at 72 hours using a luminescence assay.
**Figure 4****:** displays inhibition of FLT-3 activated AML193 cell growth by increasing concentrations of Staurosporine versus the control (vehicle) measured at 72 hours using a luminescence assay.
**Figure 5****:** displays inhibition of RS4-11 cell growth by increasing concentrations of GN963 versus the control (vehicle) measured at 72 hours using a luminescence assay.
**Figure 6****:** displays inhibition of FLT-3 activated RS4-11 cell growth by increasing concentrations of GN963 versus the control (vehicle) measured at 72 hours using a luminescence assay.

## Claims

1. A method for screening for a combination of biological compounds capable of abrogating mature vasculature in a patient's tumor comprising:
• introducing tumor cells to a collection of microvascular cells including mural cells and pericytes;
• regularly administering to the tumor cell a PDGF-receptor beta inhibitor;
• administering to the tumor cells one or more anti-angiogenic or anti-cancer agents; and
• measuring the one or more of tumor volume, mean vessel density, endothelial cells division, or endothelial cells apoptosis in the cells compared to a control, whereby a difference between the control and the cells administered the PDGF-receptor beta inhibitor and the one or more anti-cancer agents can be detected.

2. A method for screening for a combination of biological compounds capable of inhibiting the activation loop between endothelial cell and smooth muscle cells within arterioles of perivasculature of a mammal's tumor comprising:
• introducing tumor cells to a collection of microvascular cells including mural cells and pericytes, with the exception that the collection of microvascular cells does not consist of bone cells or bone tissue;
• regularly administering to the tumor cells a PDGF-receptor beta inhibitor;
• administering to the tumor cells one or more anti-cancer agents; and
• measuring the one or more of tumor volume, mean vessel density, endothelial cells division, or endothelial cells apoptosis in the cells compared to a control, whereby a difference between the control and the cells administered the PDGF-receptor beta inhibitor and the one or more anti-cancer agents can be detected.

3. The method of any one of claims 1 and 2, wherein the PDGF-receptor beta inhibitor is the extracellular binding domain of the PDGF-Rβ fused to the constant region of an Ig (PDG F-Rβ-Fc).

4. The method of any one of claims 1 to 3, wherein the one or more anti-cancer agents is from the group of taxotere, paclitaxel, docetaxel, gemcitabine, flucrouracil, mitomycin, and epirubicin.

5. The method of any one of claims 1 to 4, wherein the at least one chemotherapeutic agent is selected from the group consisting of anti-microtubule agents, platinum coordination complexes, alkylating agents, antibiotic agents, topoisomerase II inhibitors, antimetaboltes, topoisomerase I inhibitors, hormones and hormone analogues, signal transduction pathway inhibitors, non-receptor tyrosine kinase angiogenesis inhibitors, immunotherapeutic agents, proapoptotic agents, and cell cycle signaling inhibitors.

6. The method of any one of claims 1 to 5, wherein the method of measuring endothelial cells division or endothelial cells apoptosis comprises TUNEL (in situ terminal dUTP nick-end labeling).

7. The method of any one of claims 1 to 6, wherein the collection of microvascular cells is one of the group consisting of brain tissue, lung tissue, pancreas tissue, ovarian tissue, liver tissue, lymph tissue, or skin tissue.

8. The method of any one of claims 1 to 7, wherein the treatment regimen is more than 10 days, and preferably more than 20 days.

## Patentansprüche

1. Verfahren zum Screening einer Kombination biologischer Verbindungen, die dazu in der Lage ist, das reife Gefäßsystem im Tumor eines Patienten abzutöten, bei dem man:
• Tumorzellen in eine Sammlung mikrovaskulärer Zellen einschließlich Wandzellen und Perizyten einführt;
• an den Tumor regelmäßig einen PDGF-Rezeptor-beta-Inhibitor verabreicht;
• an die Tumorzellen eine oder mehrere antiangiogene Mittel oder Antikrebsmittel verabreicht; und
• einen oder mehrere der Parameter Tumorvolumen, mittlere Gefäßdichte, Endothelzellteilung oder Endothelzellapoptose in den Zellen verglichen mit einer Kontrolle mißt, wobei ein Unterschied zwischen der Kontrolle und den Zellen, denen der PDGF-Rezeptor-beta-Inhibitor und das eine oder die mehreren Antikrebsmittel verabreicht wurden, nachgewiesen werden kann.

2. Verfahren zum Screening einer Kombination biologischer Verbindungen, die dazu in der Lage ist, die Aktivierungsschleife zwischen Endothelzellen und Glattmuskelzellen in den Arteriolen des Perivaskulärsystems eines Säugetiertumors zu hemmen, bei dem man
• Tumorzellen in eine Sammlung mikrovaskulärer Zellen einschließlich Wandzellen und Perizyten einführt, wobei allerdings die Sammlung mikrovaskulärer Zellen keine Knochenzellen bzw. kein Knochengewebe enthält;
• den Tumorzellen regelmäßig einen PDGF-Rezeptor-beta-Inhibitor verabreicht;
• den Tumorzellen eine oder mehrere Antikrebsmittel verabreicht; und
• einen oder mehrere der Parameter Tumorvolumen, mittlere Gefäßdichte, Endothelzellteilung oder Endothelzellapoptose in den Zellen verglichen mit einer Kontrolle mißt, wobei ein Unterschied zwischen der Kontrolle und den Zellen, denen der PDGF-Rezeptor-beta-Inhibitor und das eine oder die mehreren Antikrebsmittel verabreicht wurden, nachgewiesen werden kann.

3. Verfahren nach Anspruch 1 oder 2, wobei es sich bei dem PDGF-Rezeptor-beta-Inhibitor um die mit der konstanten Region eines Ig kondensierte extrazelluläre Bindungsdomäne des PDGF-Rβ (PDGF-F-Rβ-Fc) handelt.

4. Verfahren nach einem der Ansprüche 1 bis 3, wobei das eine oder die mehreren Antikrebsmittel aus der aus Taxoter, Paclitaxel, Docetaxel, Gemcitabin, Fluoruracil, Mitomycin und Epirubicin bestehenden Gruppe stammen.

5. Verfahren nach einem der Ansprüche 1 bis 4, wobei das wenigstens eine chemotherapeutische Mittel ausgewählt ist aus der Gruppe bestehend aus Antimikrotubulosmitteln, Platinkoordinationskomplexen, Alkylierungsmitteln, Antibiotika, Topoisomerase-II-Inhibitoren, Antimetaboliten, Topoisomerase-I-Inhibitoren, Hormonen und Hormonanaloga, Inhibitoren von Signalübertragungspfaden, Nicht-Rezeptortyrosinkinase-Angiogeneseinhibitoren, Immunotherapeutika, proapoptotischen Mitteln und Inhibitoren der Zellzyklussignalvermittlung.

6. Verfahren nach einem der Ansprüche 1 bis 5, wobei das Verfahren zum Messen der Endothelzellteilung oder der Endothelzellapoptose TUNEL (in situ terminal dUTP nick-end labeling) umfaßt.

7. Verfahren nach einem der Ansprüche 1 bis 6, wobei die Sammlung mikrovaskulärer Zellen aus der Gruppe bestehend aus Hirngewebe, Lungengewebe, Bauchspeicheldrüsengewebe, Eierstockgewebe, Lebergewebe, Lymphgewebe oder Hautgewebe stammt.

8. Verfahren nach einem der Ansprüche 1 bis 7, wobei das Behandlungsprotokoll mehr als 10 Tage und vorzugsweise mehr als 20 Tage umfaßt.

## Revendications

1. Méthode de criblage d'une combinaison de composés biologiques capables de détruire le système vasculaire mature dans une tumeur d'un patient comprenant :
• l'introduction de cellules tumorales à une collection de cellules microvasculaires y compris de cellules murales et de péricytes ;
• l'administration régulière à la cellule tumorale d'un inhibiteur du récepteur du PDGF bêta ;
• l'administration aux cellules tumorales d'un ou plusieurs agents anti-angiogéniques ou anti-cancéreux ; et
• la mesure d'un ou plusieurs parmi le volume de la tumeur, la densité moyenne des vaisseaux, la division des cellules endothéliales ou l'apoptose des cellules endothéliales dans les cellules par rapport à un témoin, une différence pouvant être détectée entre le témoin et les cellules auxquelles on a administré l'inhibiteur du récepteur du PDGF bêta et un ou plusieurs agents anti-cancéreux.

2. Méthode de criblage d'une combinaison de composés biologiques capables d'inhiber la boucle d'activation entre la cellule endothéliale et les cellules du muscle lisse dans les artérioles du système périvasculaire de la tumeur d'un mammifère comprenant :
• l'introduction de cellules tumorales à une collection de cellules microvasculaires y compris des cellules murales et des péricytes, sauf que la collection de cellules microvasculaires n'est pas constituée de cellules osseuses ou de tissus osseux ;
• l'administration régulière aux cellules tumorales d'un inhibiteur du récepteur du PDGF bêta ;
• l'administration aux cellules tumorales d'un ou plusieurs agents anti-cancéreux ; et
• la mesure d'un ou plusieurs parmi le volume de la tumeur, la densité moyenne des vaisseaux, la division des cellules endothéliales ou l'apoptose des cellules endothéliales dans les cellules par rapport à un témoin, une différence pouvant être détectée entre le témoin et les cellules auxquelles on a administré l'inhibiteur du récepteur du PDGF bêta et un ou plusieurs agents anti-cancéreux.

3. Méthode selon l'une ou l'autre des revendications 1 et 2, **caractérisée en ce que** l'inhibiteur du récepteur du PDGF bêta est le domaine de liaison extracellulaire du PDGF-Rβ fusionné à la région constante d'une Ig (PDGF-Rβ-Fc).

4. Méthode selon l'une quelconque des revendications 1 à 3, **caractérisée en ce qu'**un ou plusieurs des agents anti-cancéreux sont du groupe constitué de taxotère, de paclitaxel, de docétaxel, de gemcitabine, de fluorouracile, de mitomycine et d'épirubicine.

5. Méthode selon l'une quelconque des revendications 1 à 4, **caractérisée en ce qu'**au moins un agent chimiothérapeutique est choisi dans le groupe constitué d'agents antimicrotubules, de complexes de coordination du platine, d'agents d'alkylation, d'agents antibiotiques, d'inhibiteurs de la topoisomérase II, d'antimétabolites, d'inhibiteurs de la topoisomérase I, d'hormones et d'analogues d'hormones, d'inhibiteurs de la voie de transduction des signaux, d'inhibiteurs d'angiogenèse de tyrosine kinase non récepteur, d'agents immunothérapeutiques, d'agents proapoptotiques et d'inhibiteurs de la signalisation du cycle cellulaire.

6. Méthode selon l'une quelconque des revendications 1 à 5, **caractérisée en ce que** la méthode de mesure de la division de cellules endothéliales ou de l'apoptose de cellules endothéliales comprend la méthode TUNEL (marquage terminal dUTP in situ des coupures d'ADN).

7. Méthode selon l'une quelconque des revendications 1 à 6, **caractérisée en ce que** la collection de cellules microvasculaires est du groupe constitué de tissus du cerveau, de tissus du poumon, de tissus du pancréas, de tissus ovariens, de tissus du foie, de tissus de la lymphe ou de tissus de la peau.

8. Méthode selon l'une quelconque des revendications 1 à 7, **caractérisée en ce que** le régime de traitement est de plus de 10 jours, et de préférence de plus de 20 jours.
